# EUROPEAN PATENT SPECIFICATION

(11) **EP 2 363 410 B1**
(45) Date of publication and mention of the grant of the patent: **11.10.2017**
(21) Application number: 10184859.6
(22) Date of filing: 26.08.2004
(51) Int. Cl.: C07K 14/47, C12N 15/64, C12N 15/113

(54) **Isoforms of MUC1**
MUC1-Isoformen
Isoformes de MUC1

(30) Priority: 26.08.2003 US 498260 P
(43) Date of publication of application: 07.09.2011
(62) Divisional of application: 04782436.2
(73) Proprietor: Minerva Biotechnologies Corporation, Waltham, MA 02451 (US)
(72) Inventor: Bamdad, Cynthia, Waltham, 02451-1002 (US)
(74) Representative: McNamara, Kathryn

(56) References cited:
- WO-A-02/22685
- WO-A-02/056022
- WO-A2-96/03502
- WO-A2-03/054154
- LAN M S ET AL: "Cloning and sequencing of a human pancreatic tumor mucin cDNA", JOURNAL OF BIOLOGICAL CHEMISTRY, THE AMERICAN SOCIETY OF BIOLOGICAL CHEMISTS, INC., BALTIMORE, MD, US, vol. 265, no. 25, 5 September 1990 (1990-09-05), pages 15294-15299,ISS, XP002961404, ISSN: 0021-9258
- GENDLER S J ET AL: "MOLECULAR CLONING AND EXPRESSION OF HUMAN TUMOR-ASSOCIATED POLYMORPHIC EPITHELIAL MUCIN", JOURNAL OF BIOLOGICAL CHEMISTRY, THE AMERICAN SOCIETY OF BIOLOGICAL CHEMISTS, INC., BALTIMORE, MD, US, vol. 265, no. 25, 5 September 1990 (1990-09-05), pages 15286-15293, XP000764846, ISSN: 0021-9258

## Description

Described herein are drug screening assays, products for cancer diagnosis and for the evaluation of cancer treatment and using the portion of the receptor that remains on the cell as a molecular target for cancer therapeutics, to binding peptides, such as antibodies or antigen-binding fragments thereof to such receptor cleavage products, polypeptides comprising the receptor cleavage products, and nucleic acid molecules for encoding the same.

### Background

The molecular basis of cell growth and programmed cell death, termed apoptosis, is of great interest to pharmaceutical companies and cancer researchers, in general. It appears that in cancers one or both of these processes has gone awry. Drug discovery for cancers is increasingly focused on the development of therapeutics that interfere with critical steps in the processes of cell growth and programmed cell death. Of particular interest are agents that interfere with growth factor receptors. Typically, growth factor receptors have extracellular domains that interact in a highly specific way with cognate ligands to transmit a proliferation signal to the inside of the cell. Interactions and signaling pathways inside the cell tend to be conserved and are not cell-specific. Specificity is usually achieved via extracellular interactions. Agents that interfere with intracellular processes may be undesirable as therapeutics because they may have widespread effects in healthy as well as diseased cells. In contrast, therapeutics that target extracellular portions of growth factor receptors, especially if those portions are in some way altered in cancer cells, are highly desirable as they would specifically target cancer cells.

Accordingly, cell surface receptors, that have been linked to cancer, make up an important class of therapeutic targets. Many pharmaceutical companies are actively involved in screening drug libraries for compounds that bind to and block these cell surface receptors. For example, an important drug used to treat breast cancer is Herceptin (Pegram M, Lipton A, Hayes D, Webber B, Baselga J, Tripathy D, Baly D, Baughman S, Twaddell T, Glaspy J, Slamon D: Phase II study of receptor-enhanced chemosensitivity using recombinant humanized anti-p185 Her2/neu monoclonal antibody plus cisplatin, in patients with Her2/neu-overexpressing metastatic breast cancer refractory to chemotherapy treatment, J Clin Oncol, 1998,16(8): 2659-2671). This drug binds to and blocks HER2/neu (Ross J, Fletcher J: review, The Her2/neu oncogene in breast cancer: prognostic factor, predictive factor, and target for therapy. Stem Cells, 1998,16(6): 413-428) which is a cell surface receptor that is over-expressed on 30% of breast tumors.

Another cell surface receptor is called MUC1 (Treon S, Mollick J, Urashima M, Teoh G, Chauhan D, Ogata A, Raje N, Hilgers J, Nadler L, Belch A, Pilarski L and Anderson K: MUC1 core protein is expressed on multiple myeloma cells and is induced by dexamethasone. Blood, 1999, 93(4): 1287-1298). The MUC1 receptor is a Type I transmembrane glycoprotein from the mucin family that has been implicated in many human cancers. It is estimated that approximately 75% of all solid tumors *aberrantly* express the MUC1 receptor. The group of MUC1⁺ cancers includes more than 90% of breast carcinomas, 47% of prostate tumors and a high percentage of ovarian, colorectal, lung, and pancreatic cancers. MUC1 is normally expressed on glandular secretory epithelial cells as well as on epithelium that line the airways. There is some evidence that among the normal functions of the MUC1 receptor are roles in cell adhesion, fertility and immune response. The role of the MUC1 receptor in cancers has not yet been established in the literature. However, major differences in cell surface expression and receptor patterning in cancers have been well documented. The most striking difference between MUC1 expression on a healthy cell and expression in a cancer cell is that on a healthy cell, the receptor is clustered at the apical border, while on cancer cells the receptor is uniformly distributed over the entire surface of the cell. Additionally, there is some evidence that the receptor is overexpressed on tumor cells in addition to the aberrant patterning.

The normal function of MUC1 as well as its link to cancer has not yet been definitively determined. What is known is that a portion of the extracellular domain of MUC1 is shed or cleaved and can be detected in the serum of breast cancer patients. In breast cancer patients, levels of shed MUC1 in the serum are sometimes measured to monitor the patient's response to treatment. The cytoplasmic tail of MUC1 is rich in motifs for a variety of signal transduction proteins. It has been reported in the literature that Grb2 and SOS, which are common signaling proteins, associate with MUC1's cytoplasmic tail. It is noted in the scientific literature that in cancer cells, the extracellular domain is underglycosylated. Although the MUC1 receptor was cloned in 1990, its link to cancer has remained elusive.

The molecular cloning and expression of human tumor-associated polymorphic epithelial mucin (MUC1) was discussed in Gendler et al. (Journal of Biological Chemistry, The American Society of Biological Chemists, Inc., vol. 265, no. 25, 5 September 1990, pages 15286-15293). WO 02/22685A2 discloses the MUC1 extracellular domain and cancer treatment compositions and methods dervived therefrom.

The present disclosure describes discoveries that elucidate critical aspects of the mechanism by which MUC1 triggers cell proliferation and tumorigenesis. These discoveries provide novel molecular targets for drug screening assays which the inventors have used to identify compounds and binding peptides that inhibit the MUC1-dependent tumorigenesis. These discoveries also enable an early diagnostic assay and an accurate method for tracking the progress of cancer patients undergoing treatment.

### Summary of the Invention

The inventors present evidence herein supporting a mechanism whereby that a portion of the MUC1 receptor (proximal, i.e. external, to the cell surface), functions as a growth factor receptor. The addition of compounds that bind to the PSMGFR portion of the MUC1 receptor is shown herein to inhibit cell growth, presumably by preventing the dimerization of the MGFR portion of the receptor. The inventors also demonstrate herein that monovalent antibodies raised against the MGFR portion of the MUC1 receptor also inhibit cell growth by binding to and blocking the association of the MGFR portion of the receptor with cognate ligands.

The inventors present evidence herein, that describes that a shorter form of the MUC1 receptor, either a proteolyzed fragment that is comprised essentially of the natural sequence of the PSMGFRTC (i.e. nat-PSMGFRTC - SEQ ID NO: 37 in Table 1 below) or an alternative splice isoform such as the MUC1-Y (SEQ ID NO: 40 - Table 1), functions as a growth factor receptor. Herein, evidence is provided that supports the hypothesis that
dimerization of a shorter (i.e. truncated) form of the MUC1 receptor, comprised essentially of the nat-PSMGFRTC (SEQ ID NO: 37), transmits a signal to the inside of the cell, which then activates a cell growth signaling cascade.
The present disclosure also describes a monovalent fragment of an antibody and monovalent, single-chain antibodies that target a portion of the MUC1 receptor proximal to the cell surface (e.g. MGFR) that inhibits
receptor dimerization and thus can be used as a cancer therapeutic for MUC1⁺ cancers. A cell line that mimics MUC1⁺ cancer cells for use as a research tool for drug discovery is described. The present disclosure also provides experimental evidence that the dominant MUC1 species in breast tumors is a cleavage product that is comprised essentially of nat-PSMGFRTC (SEQ ID NO: 37).

In a first aspect of the invention, there is provided a method comprising: transfecting or transforming a host cell in vitro with an expression vector encoding an amino acid sequence of SEQ ID NO.37, or a variant of amino acid sequence of SEQ ID NO. 37 having amino acid substitutions up to 15 or any amino acid additions or deletions up to 15 at its N-terminus or C-terminus, being free of an interchain binding region of the cell surface receptor to the extent necessary to prevent spontaneous binding between them; and facilitating expression of the peptide by the cell so that the cell expresses the peptide as a transmembrane peptide on the cell surface.

In a second aspect of the invention, there is provided a transgenic non-human animal comprising an expression vector encoding an amino acid sequence of SEQ ID NO.37, or a variant of amino acid sequence of SEQ ID NO. 37 having amino acid substitutions up to 15 or any amino acid additions or deletions up to 15 at its N-terminus or C-terminus, being free of an interchain binding region of the cell surface receptor to the extent necessary to prevent spontaneous binding between them.
Preferred embodiments of the invention in any of its various aspects are as described below or as defined in the sub claims.

Another kit of the invention comprises a species able to bind to a portion of a cell surface receptor that includes the interchain binding region, and a signaling entity immobilized relative to or adapted to be immobilized relative to the species.

Another kit of the invention comprises an article (which can be a particle), and at least a fragment of the sequence that corresponds to that portion of a cell surface receptor that interacts with an activating ligand, such as a growth factor or modifying enzyme, to promote cell proliferation, the fragment being detached from any cell, fastened to or adapted to be fastened to the article.

In another aspect, the invention provides a series of methods.

One method comprises providing a peptide including a portion of a cell surface receptor that interacts with an activating ligand such as a growth factor to promote cell proliferation, the portion including enough of the cell surface receptor to interact with the activating ligand and the portion; and generating a antibody or antigen-binding fragment thereof that specifically binds to the peptide. An antibody or antigen binding fragment thereof produced by the above method is also disclosed..

In another embodiment, a method for treating a subject having a cancer characterized by the aberrant expression of MUC1, comprising administering to the subject an antibody or antigen-binding fragment thereof in an amount effective to ameliorate the cancer is disclosed.

In yet another embodiment, a method of treating a subject having cancer or at risk for developing cancer comprising administering to the subject an antibody or antigen-binding fragment thereof that specifically binds to a peptide including a portion of a cell surface receptor that interacts with an activating ligand such as a growth factor to promote cell proliferation, the portion including enough of the cell surface receptor to interact with the activating ligand is disclosed.

In another embodiment, a method of determining the aggressiveness and/or metastatic potential of a cancer comprising contacting a sample obtained from a subject having or suspected of having the cancer with an antibody, antigen-binding fragment thereof, or similar recognition entity that specifically binds to a peptide expressed on a cell surface; and determining an amount of the antibody, antigen-binding fragment thereof or cognate ligand that specifically binds to the sample is disclosed.

In yet another embodiment, a method is disclosed comprising transfecting or transforming a host cell with an expression vector encoding an amino acid sequence comprising a cell surface peptide including a portion of a cell surface receptor, the portion including enough of the cell surface receptor both to interact with an activating ligand, such as a growth factor or modifying enzyme, and to promote cell proliferation and being free of an interchain binding region of the cell surface receptor to the extent necessary to prevent spontaneous binding between portions; and facilitating expression of the peptide by the cell so that the cell presents the peptide on its surface.

In another embodiment, a method is disclosed comprising providing a peptide including a portion of a cell surface receptor, the portion including enough of the cell surface receptor both to interact with an activating ligand, such as a growth factor or modifying enzyme, and to promote cell proliferation and being free of an interchain binding region of the cell surface receptor to the extent necessary to prevent spontaneous binding between portions; and developing an expression vector comprising a nucleic acid molecule that encodes the peptide. An expression vector produced by the method described above is also disclosed.

In yet another embodiment, a method is disclosed comprising providing a cell expressing on its surface a peptide including a portion of a cell surface receptor, the portion including enough of the cell surface receptor both to interact with an activating ligand such as a growth factor and to promote cell proliferation and being free of an interchain binding region of the cell surface receptor to the extent necessary to prevent spontaneous binding between portions; contacting the cell with a candidate drug for affecting the ability of the activating ligand to interact with the peptide, and to the activating ligand; and
determining whether an intracellular protein that becomes phosphorylated upon interaction of the activating ligand with the peptide is phosphorylated.

In another embodiment, a method is disclosed comprising providing a cell expressing on its surface a peptide comprising MGFR; contacting the cell with a candidate drug for affecting the ability of an activating ligand to interact with MGFR, and to the activating ligand; and determining whether an ERK-2 protein within the cell is phosphorylated.

In yet another embodiment, a method is disclosed comprising simultaneously determining whether a drug candidate suspected of having the ability to interfere with the binding of an activating ligand to a cell surface receptor interferes with the binding of the activating ligand to the cell surface receptor and whether the drug candidate interacts with the cell surface receptor or the ligand.

In another embodiment, a method for determining the modification state of a biological molecule is disclosed, comprising providing a colloid particle, which is configured to become immobilized with respect to the biological molecule when the biological molecule is in a first modification state to a different extent than when the biological molecule is in a second modification state, in proximity with the biological molecule; and detecting immobilization of the colloid particle relative to the biological molecule.

Another method of the invention involves treating a subject having cancer or being at risk for developing cancer, the method comprises administering to the subject an agent that reduces cleavage of a cell surface receptor.

Another method of the invention for treating a subject having cancer or at risk for developing cancer comprises administering to the subject an agent that reduces cleavage of a cell surface receptor interchain binding region from the cell surface.

Another method of the invention comprises determining an amount of cleavage of a cell surface receptor interchain binding region from a cell surface, and evaluating indication of cancer or potential for cancer based upon the determining step.

Another method of the invention comprises determining a site of cleavage of a cell surface receptor in a sample from a subject, and evaluating an indication of cancer or potential for cancer based upon the determining step.

Another method of the invention involves determining a cleavage site of a cell surface. The method comprises contacting a cell with an agent that binds specifically to one potential cell surface receptor cleavage site and another agent that binds specifically to another potential cell surface receptor cleavage site. The ratio of binding of the two agents to the cell surface is compared in the method.

Another method of the invention comprises determining a first amount of cleavage of a cell surface receptor interchain binding region from a cell surface of a sample from a subject. A second amount of cleavage of cell surface receptor interchain binding region from a cell surface of a sample from the subject is also determined, and the first amount is compared to the second amount.

Another composition comprises an antibody or antigen-binding fragment thereof provided according to the invention.

Another composition comprises an antibody or antigen-binding fragment thereof that specifically binds to MGFR.

The invention also provides peptide species. One peptide species of the invention comprises at least a fragment of a sequence that corresponds to that portion of a cell surface receptor that interacts with an activating ligand such as a growth factor to promote cell proliferation, the portion being detached from any cell, and an affinity tag.

In another embodiment, an antibody or antigen-binding fragment thereof that specifically binds to MGFR is disclosed.

In yet another embodiment, an isolated protein or peptide comprising PSMGFR at its N-terminus, wherein the isolated protein or peptide does not comprise any of the amino acid sequences set forth in SEQ ID NOs: 1, 2,3, 6, or 7 is disclosed.

In another embodiment, an isolated protein or peptide comprising the amino acid sequences set forth in SEQ ID NO: 7 at its N-terminus is disclosed.

In another embodiment, an isolated protein or peptide comprising the amino acid sequences set forth in SEQ ID NO: 64 at its N-terminus is disclosed.

In another embodiment, an isolated protein or peptide comprising the amino acid sequences set forth in SEQ ID NO: 2 is disclosed.

In another embodiment, an isolated protein or peptide comprising the amino acid sequences set forth in SEQ ID NO: 60 is disclosed.

In another embodiment, an isolated protein or peptide comprising the amino acid sequences set forth in SEQ ID NO: 7 is disclosed.

In another embodiment, an isolated protein or peptide comprising the amino acid sequences set forth in SEQ ID NO: 64 is disclosed.

In another embodiment, an antibody or antigen binding fragment thereof that specifically binds to the amino acid sequence set forth in SEQ ID NO: 8 is disclosed.

In another embodiment, an antibody or antigen binding fragment thereof that specifically binds to the amino acid sequence set forth in SEQ ID NO: 65 is disclosed.

In another embodiment, an antibody or antigen binding fragment thereof that specifically binds to the unique region of the amino acid sequence set forth in SEQ ID NO: 39 is disclosed.

In another embodiment, an antibody or antigen binding fragment thereof that specifically binds to a region spanning the N-terminus and amino acid no. 104 of the amino acid sequence set forth in SEQ ID NO: 39 is disclosed.

In another series of embodiments, a method comprising acts of applying an antibody or antigen-binding fragment thereof as disclosed herein to a sample; observing an interaction of the antigen-binding fragment thereof with the sample; and making a diagnosis of the presence or absence of cancer or the agressiveness of a cancer based at least in part on information observed in the observing act.

In another embodiment, an isolated protein or peptide comprising His-PSMGFR, wherein the isolated protein or peptide does not comprise any of the amino acid sequences set forth in SEQ ID NOs: 1, 2, or 3 is disclosed.

In yet another embodiment, An isolated protein or peptide comprising the amino acid sequence set forth in SEQ ID NO: 7 is disclosed.

The invention also provides a series of isolated nucleic molecules, expression vectors comprising the nucleic acid molecules, and cells transfected with the expression vectors or the nucleic acid molecules. In one embodiment, an isolated nucleic acid molecule that encodes PSMGFRTC and functional variants and fragments thereof is disclosed.

In another embodiment, an isolated nucleic acid molecule that encodes the amino acid sequence set forth in SEQ ID NO: 37 and functional variants and fragments thereof is disclosed.

In yet another embodiment, an expression vector comprising either of the above-mentioned isolated nucleic acid molecules operably linked to a promoter is disclosed.

In another embodiment, a host cell transfected or transformed with an expression vector comprising either of the above-mentioned isolated nucleic acid molecules is disclosed.

In yet another embodiment, an isolated nucleic acid molecule that hybridizes to the nucleic acid sequence set forth in SEQ ID NO: 37 under high stringency conditions, and complements thereof is disclosed.

In another embodiment, an expression vector comprising the above-identified isolated nucleic acid molecule or complement thereof operably linked to a promoteris disclosed.

### Brief Description of the Drawings

**Fig. 1** is a schematic illustration of the MUC1 receptor (top) and the various truncated MUC 1 receptor isoforms produced according to the present disclosure;
**Fig. 4** is a graph of percent cell proliferation that shows that an antibody against an epitope of the MUC1 receptor which is proximal to the cell surface, i.e. extracellular, and that dimerizes the receptor, enhances cell proliferation in a manner typical of a growth factor/receptor - antibody interaction;
**Fig. 5** is a graph of percent cell proliferation that shows that an antibody against an epitope of the MUC1 receptor which is proximal to the cell surface, and that dimerizes the receptor, dramatically enhances cell proliferation;
**Fig. 9** is a silver-stained gel showing ligands that were fished out of cell lysates using a particular PSMGFR peptide, in the presence of the protease inhibitor PMSF;
**Fig. 10** is a silver-stained gel showing ligands that were fished out of cell lysates using the PSMGFR peptide of Fig. 9, in the absence of the protease inhibitor PMSF;
**Fig. 21** is a graph showing that bivalent anti-PSMGFR antibody stimulates cell growth in MUC1+ breast tumor cell line 1504;
**Fig. 22** is a graph showing that bivalent anti-PSMGFR antibody stimulates cell growth in MUC1+ breast tumor cell line 1500;
**Fig. 23** is another graph showing that bivalent anti-PSMGFR antibody stimulates cell growth in MUC1+ breast tumor cell line 1500;
**Fig. 24** is a graph showing that bivalent anti-PSMGFR antibody stimulates cell growth in MUC1+ breast tumor cell line T47D;
**Fig. 25** is a graph showing that bivalent anti-PSMGFR antibody stimulates cell growth in MUC1+ breast tumor cell line BT-474;
**Fig. 27** is a graph showing that monovalent anti-PSMGFR inhibits cell growth in MUC1+ breast tumor cell line 1504;
**Fig. 28** is a graph showing that monovalent anti-PSMGFR inhibits cell growth in MUC1+ breast tumor cell line 1500;
**Fig. 29** is a histogram showing that monovalent anti-PSMGFR competes with bivalent anti-PSMGFR and blocks color change in a nanoparticle assay;
**Fig. 30** are western blots showing that breast tumor cells produce MUC1 clevage products of apparent molecular weight 20-30 kDa;
**Fig. 31** is a western blot showing that bivalent anti-PSMGFR dimerizes MUC1 in T47D cells and activates intracellular MAP kinase cell proliferation pathway;
**Fig. 32** is a western blot showing that bivalent anti-PSMGFR activates intracellular MAP kinase cell proliferation pathway in 1504 breast tumor cells;
**Fig. 33** is a western blot showing that bivalent anti-PSMGFR activates intracellular MAP kinase cell proliferation pathway in 1500 breast tumor cells;
**Fig. 34** is a western blot showing that drug compounds compete with bivalent anti-PSMGFR and block activation of intracellular MAP kinase cell proliferation pathway;
**Fig. 35** is a western blot showing that monovalent anti-PSMGFR competes with bivalent anti-PSMGFR and block activation of intracellular MAP kinase cell proliferation pathway;
**Fig. 36** is a western blot showing that breast tumor cells present full-length as well as cleaved MUC1;
**Fig. 37** is a western blot showing that MUC1 cleavage products are N-glycosylated;
**Fig. 38** is a schematic illustration of the MUC1 receptor variants transfected into HEK cells; **Fig. 39** is a western blot showing a MUC1 tumor-specific cleavage product runs as an approximately 20 kDa band;
**Fig. 40** is a histogram showing that monovalent anti-PSMGFR inhibits cell growth in nat-PSMGFRTC transfectants;
**Fig. 41** is a western blot showing a bivalent anti-PSMGFR antibody induces ERK2 phosphorylation in HEK cells transfected with nat-PSMGFRTC isoform;
**Fig. 42** is a western blot showing a in nat-PSMGFRTC transfectants, bivalent anti-PSMGFR antibody induces ERK2 phosphorylation and monovalent anti-PSMGFR antibody inhibits ERK2 phosphorylation;
**Fig. 43** is a western blot showing receptor clevage products for MUC 1+ tumor cells and transfectants; and
**Fig. 44** is a western blot showing that breast tumor cells may produce two MUC1 clevage products.

### Detailed Description

### Definitions:

The term "MUC1 Growth Factor Receptor" (MGFR) is a functional definition meaning that portion of the MUC1 receptor that interacts with an activating ligand, such as a growth factor or a modifying enzyme such as a cleavage enzyme, to promote cell proliferation. The MGFR region of MUC1 is that extracellular portion that is closest to the cell surface and is defined by most or all of the PSMGFR, as defined below. The MGFR is inclusive of both unmodified peptides and peptides that have undergone enzyme modifications, such as, for example, phosphorylation, glycosylation, etc. Results of the present disclosure are consistent with a mechanism in which this portion is made accessible to the ligand upon MUC1 cleavage at a site associated with tumorigenesis that causes release of the some or all of the IBR from the cell.

The term "Interchain Binding Region" (IBR) is a functional definition meaning that portion of the MUC1 receptor that binds strongly to identical regions of other MUC1 molecules giving MUC1 the ability to aggregate (i.e. self-aggregate) with other MUC1 receptors via the IBRs of the respective receptors. This self-aggregation may contribute to MUC1 receptor clustering, observed in healthy cells.

In a preferred instance, the IBR may be approximately defined as a stretch of at least 12 to 18 amino acid sequence within the region of the full-length human MUC1 receptor defined as comprising amino acids 507 to 549 of the extracellular sequence of the MUC1 receptor (SEQ ID NO: 10), with amino acids 525 through 540 and 525 through 549 especially preferred (numbers refer to Andrew Spicer et al., J. Biol. Chem Vol 266 No. 23, 1991 pgs. 15099-15109; these amino acid numbers correspond to numbers 1067, 1109, 1085, 1100, 1085, 1109 of Genbank accession number P15941; PID G547937, SEQ ID NO: 10) or fragments, functional variants or conservative substitutions thereof, as defined in more detail below.

The term "cleaved IBR" means the IBR (or a portion thereof) that has been released from the receptor molecule segment which remains attached to the cell surface. The release may be due to enzymatic or other cleavage of the IBR. As used herein, when the IBR is "at the surface of a cell", it means the IBR is attached to the portion of the cell surface receptor that has not been shed, or cleaved. The cleaved IBR of interest is a "disease-associated cleavage", i.e. that type of cleavage that can result in cancer.
The term "Constant Region" (CR) is any non-repeating sequence of MUC1 that exists in a 1:1 ratio with the IBR and forms part of the portion of MUC1 that is shed upon cleavage in healthy and tumorigenesic cells.

The term "Repeats" is given its normal meaning in the art.

The term "Primary Sequence of the MUC1 Growth Factor Receptor" (PSMGFR) is a peptide sequence that defines most or all of the MGFR in some cases, and functional variants and fragments of the peptide sequence, as defined below. The PSMGFR is defined as SEQ NO: 36 listed below in Table 1, and all functional variants and fragments thereof having any integer value of amino acid substitutions up to 20 (i.e. 1, 2, 3, 4, 5, 6, 7, 8, 9, 10, 11, 12, 13, 14, 15, 16, 17, 18, 19, or 20) and/or any integer value of amino acid additions or deletions up to 20 at its N-terminus and/or C-terminus. A "functional variant or fragment" in the above context refers to such variant or fragment having the ability to specifically bind to, or otherways specifically interact with, ligands that specifically bind to, or otherwise specifically interact with, the peptide of SEQ NO: 36, while not binding strongly to identical regions of other peptide molecules identical to themselves, such that the peptide molecules would have the ability to aggregate (i.e. self-aggregate) with other identical peptide molecules. One example of a PSMGFR that is a functional variant of the PSMGFR peptide of SEQ NO: 36 (referred to as nat-PSMGFR - for "native") is SEQ NO: 7 (referred to as var-PSMGFR, which differs from nat-PSMGFR by including an -SPY- sequence instead of the native -SRY- (see bold text in sequence listings)). Var-PSMGFR may have enhanced conformational stability, when compared to the native form, which may be important for certain applications such as for antibody production. The PSMGFR is inclusive of both unmodified peptides and peptides that have undergone enzyme modifications, such as, for example, phosphorylation, glycosylation, etc. A histidine-tagged PSMGFR (e.g. See Table 1 - SEQ ID NO: 2) is abbreviated herein as His-PSMGFR. His-tagged peptide sequences are typically tagged at their C-terminus. In certain cases, there is provided an isolated protein or peptide comprising a PSMGFR, for example at the N-terminus of the protein or peptide, or consisting of a PSMGFR, wherein the isolated protein or peptide does not comprise any of the amino acid sequences set forth in SEQ IDs: 1, 2, 3, 6, or 7 listed below. In certain cases, there is provided an isolated protein or peptide comprising His- PSMGFR, for example at the N-terminus of the protein
or peptide, or consisting of His- PSMGFR, wherein the isolated protein or peptide does not comprise any of the amino acid sequences set forth in SEQ IDs: 1,2, or 3 listed below.

The term "Extended Sequence of the MUC1 Growth Factor Receptor" (ESMGFR) is a peptide sequence, defined below (See Table 1- SEQ ID NO: 3), that defines all of His-var-PSMGFR plus 9 amino acids of the proximal end of PSIBR.

The term "Tumor-Specific Extended Sequence of the MUC1 Growth Factor Receptor" (TSESMGFR) is a peptide sequence (See, as an example, Table 1- SEQ ID NO: 66) that defines a MUC1 cleavage product found in tumor cells that remains attached to the cell surface and is able to interact with activating ligands in a manner similar to the PSMGFR.

PSIBR is a peptide sequence, defined below (See Table 1- SEQ ID NO: 8), that defines most or all of the IBR.

"Truncated Interchain Binding Region" (TPSIBR) is a peptide sequence defined below (See Table 1- SEQ ID NO: 65), that defines a smaller portion of the IBR that is released from the cell surface after receptor cleavage in some tumor cells.

PSMGFRTC is a truncated MUC1 receptor isoform comprising PSMGFR and a at or within about up to 30 (i.e. within 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30) amino acids of its N-terminus and comprising the transmembrane and cytoplasmic sequences of full-length MUC1 receptor. As used herein, The phrase "at its N-terminus" referring to the location of a recited sequence within a larger molecule, such as a polypeptide or receptor, refers to such a sequence being no more than 30 amino acids from the N-terminal amino acid of the molecule. Optionally the PSMGFRTC, as well as the other truncated MUC1 receptor isoforms discussed below, can include a MUC1 N-terminal signaling sequence (Table 1-SEQ ID NO: 47, 58, or 59), typically between 20 and 30 amino acids in length, or a functional fragment or variant thereof. Such a sequence is typically encoded by the nucleic acid constructs encoding the truncated MUC1 receptor isoform and is translated but is typically cleaved prior to or upon insertion of the receptor in the membrane of the cell. Such a PSMGFRTC, i.e. including the optional signal sequence, would still be a peptide or protein "having a PSMGFR" sequence "at its N-terminus" by the above definition. An example is nat-PSMGFRTC (SEQ ID NO: 37, with or without the signal peptide of SEQ ID NO: 47,58, or 59 at the extreme N-terminus) having nat-PSMGFR (SEQ NO: 36) at its N-terminus (i.e. at the extreme N-terminal end or within 30 amino acids thereof).

The term "separation" means physical separation from a cell, i.e. a situation in which a portion of MUC 1 that was immobilized with respect to a cell is no longer immobilized with respect to that cell. E.g. in the case of cleavage of a portion of MUC 1, the portion that is cleaved is "separated" if it is free to migrate away from the cell and thereafter may be detected in a bodily fluid, or immobilized at a location remote from the cell from which it was cleaved such as another cell, a lymph node, etc.

The term "binding" refers to the interaction between a corresponding pair of molecules that exhibit mutual affinity or binding capacity, typically specific or non-specific binding or interaction, including biochemical, physiological, and/or pharmaceutical interactions. Biological binding defines a type of interaction that occurs between pairs of molecules including proteins, nucleic acids, glycoproteins, carbohydrates, hormones and the like. Specific examples include antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, complementary strands of nucleic acid, protein/nucleic acid repressor/inducer, ligand/cell surface receptor, virus/ligand, etc.

The term "binding partner" refers to a molecule that can undergo binding with a particular molecule. Biological binding partners are examples. For example, Protein A is a binding partner of the biological molecule IgG, and vice versa.

The term "aggregate" (noun) means a plurality of cell surface receptors or fragments thereof (e.g. MUC 1) immobilized with respect to each other with or without an intermediate auxiliary to the host system. This includes self aggregation of healthy receptors at a cell surface; self-aggregation of cleaved receptors or fragments bound to each other; cleaved receptors or fragments bound to receptors or fragments attached to a cell surface; receptors or fragments, whether attached to a cell or cleaved, immobilized with respect to each other via an intermediate auxiliary to the host. "Intermediate auxiliary to the host system" includes a synthetic species such as a polymer, dendrimer, etc., or a naturally-occurring species, for example an IgM antibody, which is not simply naturally present in the host system but is added to the host system from a source external to the host system. This excludes aggregation that is the result of an intermediate naturally present in the host system such as a growth factor that can cause disease-associated aggregation ("Inductive multimerization"). "Aggregate" (verb) or "aggregation" means the process of forming an aggregate (noun).

"Inductive multimerization" refers to aggregation wherein the aggregate formed can act to induce the cells to grow or proliferate. Inductive multimerization typically involves dimerization or tetramerization of cell surface receptors, for example by a growth factor or other activating ligand, but can also involve higher order multimerization, so long as the degree of multimerization is not so great as to mimic natural receptor clustering, in a particular cell type, which prevents receptors from signaling the cell to grow or proliferate.

"Preventative clustering" refers to multimerization of receptors to form an aggregate involving a sufficient number of receptors to mimic natural receptor clustering, in a particular cell type, which prevents receptors from signaling the cell to grow or proliferate, for example with an intermediate auxiliary to the host system.

A "ligand" to a cell surface receptor, refers to any substance that can interact with the receptor to temporarily or permanently alter its structure and/or function. Examples include, but are not limited to binding partners of the receptor, (e.g. antibodies or antigen-binding fragments thereof), and agents able to alter the chemical structure of the receptor (e.g. modifying enzymes).

An "activating ligand" refers to a ligand able interact with a receptor to transduce a signal to the cell. Activating ligands can include, but are not limited to, species that effect inductive multimerization of cell surface receptors such as a single molecular species with greater than one active site able to bind to a receptor; a dimer, a tetramer, a higher multimer, a bivalent antibody or bivalent antigen-binding fragment thereof, or a complex comprising a plurality of molecular species. Activating ligands can also include species that modify the receptor such that the receptor then transmits a signal. Enzymes can also be activating ligands when they modify a receptor to make it a new recognition site for other activating ligands, e.g. glycosylases are activating ligands when the addition of carbohydrates enhances the affinity of a ligand for the receptor. Cleavage enzymes are activating ligands when the cleavage product is the more active form of the receptor, e.g. by making a recognition site for a ligand more accessible. In the context of MUC1 tumor cells, an activating ligand can be a species that cleaves MUC1, chemically modifies the receptor, or species that interact with the MGFRs on the surface of the MUC1 tumor cells to transduce a signal to the cell that stimulates proliferation, e.g. a species that effects inductive multimerization.

A "growth factor" refers to a species that may or may not fall into a class of previously-identified growth factors, but which acts as a growth factor in that it acts as an activating ligand.

A "MUC1 presenting cell" refers to both non-cancerous and cancerous cells expressing MUC1 and/or MGFRs on the surface. A "MUC1 tumor cell" or "MUC1 cancer cell" or "cancerous MUC1 cell" refers to a cancerous tumor cell that aberrantly expresses MUC1 and/or MGFR on its surface.

"Colloids", as used herein, means nanoparticles, i.e. very small, self-suspendable or fluid-suspendable particles including those made of material that is, e.g., inorganic or organic, polymeric, ceramic, semiconductor, metallic (e.g. gold), non-metallic, crystalline, amorphous, or a combination. Typically, colloid particles used in accordance with the present disclosure are of less than 250 nm cross section in any dimension, more typically less than 100 nm cross section in any dimension, and in most cases are of about 2-30 nm cross section. One class of colloids suitable for use is 10-30 nm in cross section, and another about 2-10 nm in cross section. As used herein this term includes the definition commonly used in the field of biochemistry.

As used herein, a component that is "immobilized relative to" another component either is fastened to the other component or is indirectly fastened to the other component, e.g., by being fastened to a third component to which the other component also is fastened, or otherwise is transitionally associated with the other component. For example, a signaling entity is immobilized with respect to a binding species if the signaling entity is fastened to the binding species, is fastened to a colloid particle to which the binding species is fastened, is fastened to a dendrimer or polymer to which the binding species is fastened, etc. A colloid particle is immobilized relative to another colloid particle if a species fastened to the surface of the first colloid particle attaches to an entity, and a species on the surface of the second colloid particle attaches to the same entity, where the entity can be a single entity, a complex entity of multiple species, a cell, another particle, etc.

"Signaling entity" means an entity that is capable of indicating its existence in a particular sample or at a particular location. Signaling entities can be those that are identifiable by the unaided human eye, those that may be invisible in isolation but may be detectable by the unaided human eye if in sufficient quantity (e.g., colloid particles), entities that absorb or emit electromagnetic radiation at a level or within a wavelength range such that they can be readily detected visibly (unaided or with a microscope including an electron microscope or the like), or spectroscopically, entities that can be detected electronically or electrochemically, such as redox-active molecules exhibiting a characteristic oxidation/reduction pattern upon exposure to appropriate activation energy ("electronic signaling entities"), or the like. Examples include dyes, pigments, electroactive molecules such as redox-active molecules, fluorescent moieties (including, by definition, phosphorescent moieties), up-regulating phosphors, chemiluminescent entities, electrochemiluminescent entities, or enzyme-linked signaling moieties including horseradish peroxidase and alkaline phosphatase. "Precursors of signaling entities" are entities that by themselves may not have signaling capability but, upon chemical, electrochemical, electrical, magnetic, or physical interaction with another species, become signaling entities. An example includes a chromophore having the ability to emit radiation within a particular, detectable wavelength only upon chemical interaction with another molecule. Precursors of signaling entities are distinguishable from, but are included within the definition of, "signaling entities" as used herein.

As used herein, "fastened to or adapted to be fastened", in the context of a species relative to another species or to a surface of an article, means that the species is chemically or biochemically linked via covalent attachment, attachment via specific biological binding (e.g., biotin/streptavidin), coordinative bonding such as chelate/metal binding, or the like. For example, "fastened" in this context includes multiple chemical linkages, multiple chemical/biological linkages, etc., including, but not limited to, a binding species such as a peptide synthesized on a polystyrene bead, a binding species specifically biologically coupled to an antibody which is bound to a protein such as protein A, which is attached to a bead, a binding species that forms a part (via genetic engineering) of a molecule such as GST or Phage, which in turn is specifically biologically bound to a binding partner covalently fastened to a surface (e.g., glutathione in the case of GST), etc. As another example, a moiety covalently linked to a thiol is adapted to be fastened to a gold surface since thiols bind gold covalently. Similarly, a species carrying a metal binding tag is adapted to be fastened to a surface that carries a molecule covalently attached to the surface (such as thiol/gold binding) which molecule also presents a chelate coordinating a metal. A
species also is adapted to be fastened to a surface if a surface carries a particular nucleotide sequence, and the species includes a complementary nucleotide sequence.

"Covalently fastened" means fastened via nothing other than one or more covalent bonds. E.g. a species that is covalently coupled, via EDC/NHS chemistry, to a carboxylate-presenting alkyl thiol which is in turn fastened to a gold surface, is covalently fastened to that surface.

"Specifically fastened" or "adapted to be specifically fastened" means a species is chemically or biochemically linked to another specimen or to a surface as described above with respect to the definition of "fastened to or adapted to be fastened", but excluding all non-specific binding.

Certain cases make use of self-assembled monolayers (SAMs) on surfaces, such as surfaces of colloid particles, and articles such as colloid particles having surfaces coated with SAMs. In one set of preferred instances, SAMs formed completely of synthetic molecules completely cover a surface or a region of a surface, e.g. completely cover the surface of a colloid particle. "Synthetic molecule", in this context, means a molecule that is not naturally occurring, rather, one synthesized under the direction of human or human-created or human-directed control. "Completely cover" in this context, means that there is no portion of the surface or region that directly contacts a protein, antibody, or other species that prevents complete, direct coverage with the SAM. I.e. in preferred instances the surface or region includes, across its entirety, a SAM consisting completely of non-naturally-occurring molecules (i.e. synthetic molecules). The SAM can be made up completely of SAM-forming species that form close-packed SAMs at surfaces, or these species in combination with molecular wires or other species able to promote electronic communication through the SAM (including defect-promoting species able to participate in a SAM), or other species able to participate in a SAM, and any combination of these. Preferably, all of the species that participate in the SAM include a functionality that binds, optionally covalently, to the surface, such as a thiol which will bind to a gold surface covalently. A self-assembled monolayer on a surface, in accordance with the present disclosure, can be comprised of a mixture of species (e.g. thiol species when gold is the surface) that can present (expose) essentially any chemical or biological functionality. For example, they can include tri-ethylene glycol-terminated species (e.g. tri-ethylene glycol-terminated thiols) to resist non-specific adsorption, and other species (e.g. thiols) terminating in a binding partner of an affinity tag, e.g. terminating in a chelate that can coordinate a metal such as nitrilotriacetic acid which, when in complex with nickel atoms, captures a metal binding tagged-species such as a histidine-tagged binding species. There is provided a method for rigorously controlling the concentration of essentially any chemical or biological species presented on a colloid surface or any other surface. Without this rigorous control over peptide density on each colloid particle, co-immobilized peptides would readily aggregate with each other to form micro-hydrophobic-domains that would catalyze colloid-colloid aggregation in the absence of aggregate-forming species present in a sample. This is an advantage of the present disclosure, over existing colloid agglutination assays. In many cases the self-assembled monolayer is formed on gold colloid particles.

The kits described herein, contain one or more containers, which can contain compounds such as the species, signaling entities, biomolecules, and/or particles as described. The kits also may contain instructions for mixing, diluting, and/or administrating the compounds. The kits also can include other containers with one or more solvents, surfactants, preservative and/or diluents (e.g. normal saline (0.9% NaCl, or 5% dextrose) as well as containers for mixing, diluting or administering the components to the sample or to the patient in need of such treatment.

The compounds in the kit may be provided as liquid solutions or as dried powders. When the compound provided is a dry powder, the powder may be reconstituted by the addition of a suitable solvent, which also may be provided. Liquid forms of the compounds may be concentrated or ready to use. The solvent will depend on the compound and the mode of use or administration. Suitable solvents for are well known for drug compounds and are available in the literature.

The term "cancer", as used herein, may include but is not limited to: biliary tract cancer; bladder cancer; brain cancer including glioblastomas and medulloblastomas; breast cancer; cervical cancer; choriocarcinoma; colon cancer; endometrial cancer; esophageal cancer; gastric cancer; hematological neoplasms including acute lymphocytic and myelogenous leukemia; multiple myeloma; AIDS-associated leukemias and adult T-cell leukemia lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Kaposi's sarcoma, basocellular cancer, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor. Preferred cancers are; breast, prostate, lung, ovarian, colorectal, and brain cancer.

The term "cancer treatment" as described herein, may include but is not limited to: chemotherapy, radiotherapy, adjuvant therapy, or any combination of the aforementioned methods. Aspects of treatment that may vary include, but are not limited to: dosages, timing of administration, or duration or therapy; and may or may not be combined with other treatments, which may also vary in dosage, timing, or duration. Another treatment for cancer is surgery, which can be utilized either alone or in combination with any of the aforementioned treatment methods. One of ordinary skill in the medical arts may determine an appropriate treatment.

An "agent for prevention of cancer or tumorigenesis" means any agent that counteracts any process associated with cancer or tumorigenesis described herein. For example, an agent that interacts with (e.g. binds to) to MGFR thereby reducing or preventing interaction, with MGFR, of an agent that promotes tumorigenesis by its interaction with MGFR.

An "agent that reduces cleavage of a cell surface receptor interchain binding region" as used herein is any composition that prevents or reduces cleavage of the MUC1 receptor between the MGFR and the N-terminus of the IBR that would otherwise occur in the absence of the agent. Cleavage of the receptor between the MGFR and the N-terminus of the IBR can be caused by activity of enzymes that are membrane-associated or soluble, e.g. matrix metalloproteases (MMPs and MT-MMPs). Some of these enzymes are directly responsible for cleavage. Other enzymes can affect cleavage, (e.g. prevent cleavage at a particular location) by modifying MUC1 with sugar groups or phosphates that mask a recognition epitope associated with cleavage. Other enzymes can promote cleavage at a particular location by modifying MUC1 with sugar groups or phosphates that create a recognition motif for cleavage at that location. Other enzymes can promote cleavage of receptors by activating other cleavage enzymes. One way to select agents that reduce cleavage of a cell surface receptor IBR is to first identify enzymes that affect cleavage as described above, and screen agents, and their analogs, for their ability to alter the activity of those enzymes. Another way is to test agents that are known to affect the activity of similar enzymes (e.g. from the same family) for their ability to alter the site of cleavage of MUC1, and to similarly test analogs of these agents. Alternatively, agents are screened in a cell-free assay containing the enzyme and MUC1 receptors, and the rate or position of cleavage measured by antibody probing, Polymerase Chain Reaction (PCR), or the like. Alternatively, without first identifying enzymes that affect MUC1, agents are screened against cells that present MUC1 for the agents' ability to alter cleavage site or the rate of cleavage of MUC1. For example, agents can be screened in an assay containing whole cells that present MUC1 and aggregation potential of the cell supernatant can be measured, an indication of the amount of IBR that remains attached to the cleaved portion of MUC1, i.e. the degree of cleavage between MGFR and IBR. In another technique, agents can be screened in an assay containing whole cells that present MUC1, the supernatant removed, and the cell remain tested for accessibility of the MGFR portion, e.g. using a labeled antibody to the MGFR. Agents can be identified from commercially available sources such as molecular libraries, or rationally designed based on known agents having the same functional capacity and tested for activity using the screening assays.

An "agent that reduces cleavage of the MUC1 receptor," is any composition that prevents or reduces cleavage of the MUC1 receptor at any location. Such an agent can be used to treat a subject having cancer or at risk for developing cancer because if cleavage is prevented, then the accessibility of the MGFR, a functional receptor associated with cancer, is reduced or prevented. Such agents can be selected by exposing cells to a candidate agent and determine, in the supernatant, the amount of cleaved MUC1 receptor, relative to a control.

A subject, as used herein, refers to any mammal (preferably, a human), and preferably a mammal that may be susceptible to tumorigenesis or cancer associated with the abherrant expression of MUC1. Examples include a human, non-human primate, cow, horse, pig, sheep, goat, dog, or cat. Generally, the present disclosure is directed toward use with humans.

The samples used herein are any body tissue or body fluid sample obtained from a subject. Preferred are body fluids, for example lymph, saliva, blood, urine, milk and breast secretions, and the like. Blood is most preferred. Samples of tissue and/or cells for use in the various methods described herein can be obtained through standard methods including, but not limited to: tissue biopsy, including punch biopsy and cell scraping, needle biopsy, and collection of blood or other bodily fluids by aspiration or other methods.

The following patent applications and publications disclose or may disclose useful compositions, articles, and methods: U.S. Patent Application Publication No. 2003/0036199; International Publication No. 02/056022 A2; International patent application serial no. PCT/US00/01997, filed 01/25/00, entitled "Rapid and Sensitive Detection of Aberrant Protein Aggregation in Neurodegenerative Diseases", published as no. WO 00/43791, international patent application serial no. PCT/US00/01504, filed 01/21/00, entitled "Assays involving Colloids and Non-Colloidal Structures", published 07/27/00 as international patent publication no. WO 00/34783, U.S. patent application serial no. 09/631,818, filed 08/03/00, entitled "Rapid and Sensitive Detection of Protein Aggregation", a U.S. provisional patent application by Bamdad, et al., serial no. 60/248,865, filed 11/15/00, entitled "Endostatin-Like Angiogenesis Inhibition,"and a U.S. Utility Application Application of same title filed 11/15 2001.

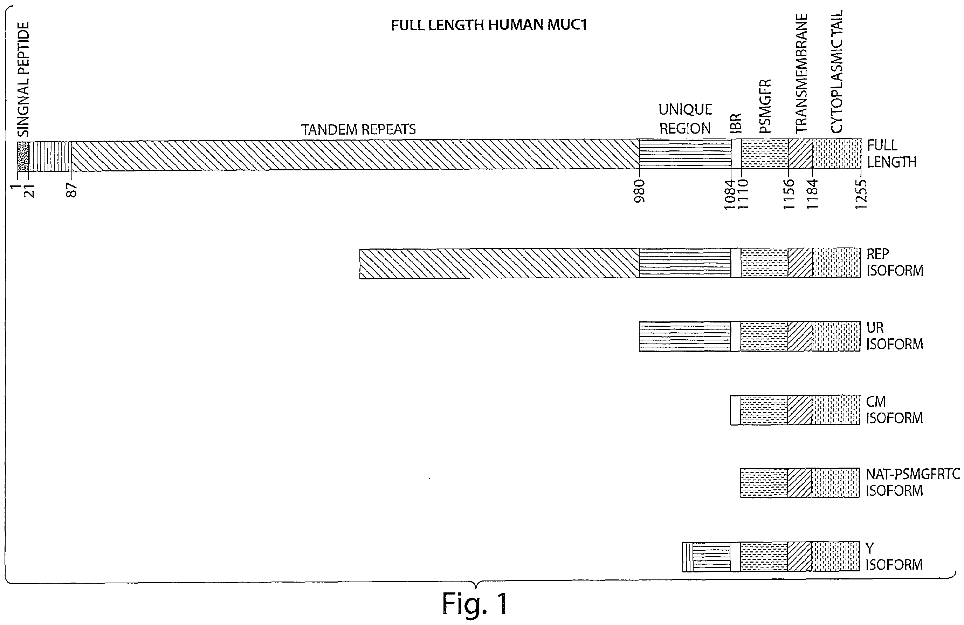

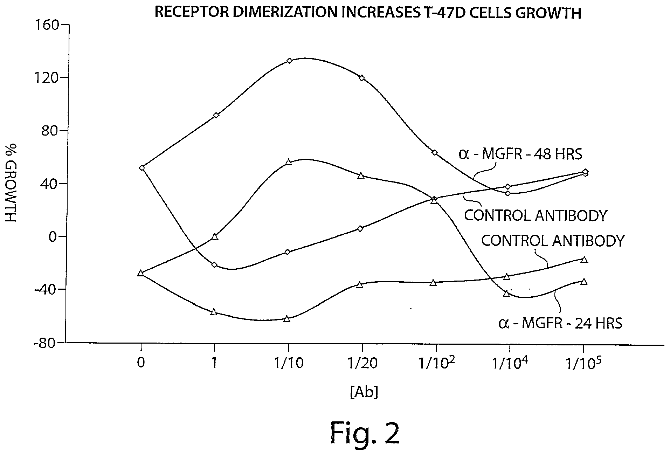

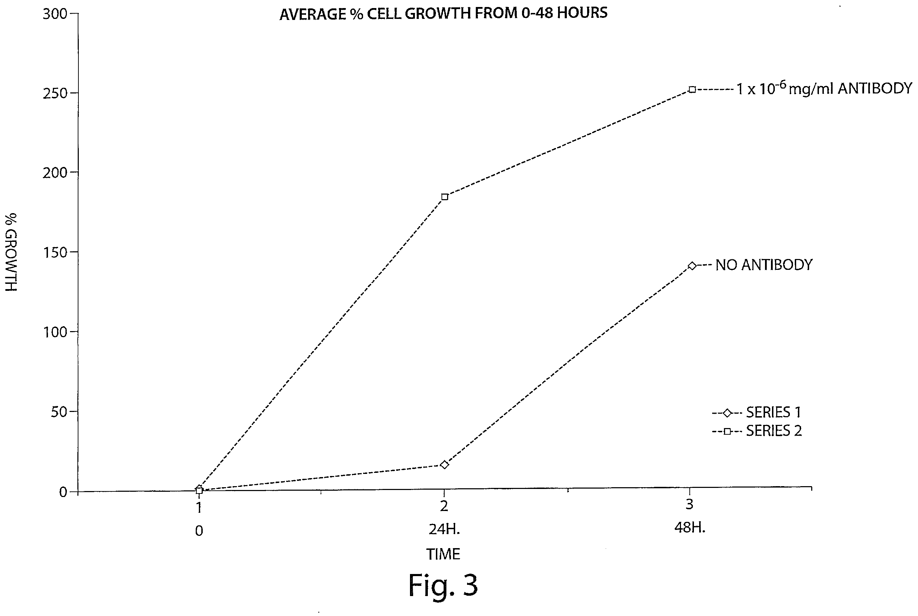

The present disclosure involves, in certain cases, novel molecular targets for drug screening, therapeutics and diagnostics related to cancers that are characterized by the aberrant expression of a class of cell surface receptors characterized by interchain binding regions. One such set of cancers are those characterized by the aberrant expression of MUC1. Much of the description herein involves cells that aberrantly express MUC1. It is to be understood that in these instances the description is to be considered exemplary, and that the principles apply to other cell surface receptors that function by a similar mechanism. With the disclosure herein, those of ordinary skill in the art will readily be able to identify other cell surface receptors that function by this or a similar mechanism, and to apply the present disclosure to those cancers characterized by aberrant expression of receptors. The present description is based on a novel mechanism involving cell surface receptors that have regions that self-aggregate,
exemplified by MUC1, which was elucidated by the inventors. MUC1 comprises several regions termed herein as follows, recited in an order starting from the region closest to the cell surface and progressing away from the cell. In U.S. Patent Application Publication No. 2003/0036199; International Publication No. 02/056022 A2; ("earlier application(s)") filed by the same inventors certain region of MUC 1 was defined differently. It is to be understood that the present definition supercedes. In the earlier, above-identified applications, the term "PSMGFR" was with reference to the exempleary peptide sequence of SEQ ID NO: 7 (currently referred to as "var-PSMGFR"). The expanded definition of PSMGFR given above is intended to apply in the present specification. The basic structure of the MUC1 receptor is illustrated in FIG.1. The receptor, as illustrated comprises: 1) cytoplasmic tail; 2) transmembrane section; 3) MGFR; 4) IBR, 5) Unique Region, 6) repeats, and N-terminus region comprising a signal peptide.

In healthy cells, MUC1 receptors are clustered at one portion of the cell surface. In contrast, MUC1-positive tumor cells are characterized by a loss of this "healthy" clustering. The present disclosure anticipates uses for detecting and treating aberrant expression of the MUC1 receptor in conditions other than cancer. For example, the MUC1 receptor is a key element in immune response and fertility. In the case of fertility, it may be beneficial for portions of the extracellular domain to be cleaved to induce embryo implantation. Methods described herein may be used for non-cancerous conditions to promote or inhibit receptor cleavage. Additionally, methods described herein may be used to diagnose conditions of infertility. In tumor cells, the MUC1 receptors are no longer clustered but instead are typically distributed over the entire cell surface or in some cancer types, the receptors form a series of clustered islands that are expressed over a considerable portion of the cell surface. This loss of clustering of the MUC1 receptor has been correlated to tumor aggressiveness, metastaic potential and eventual outcome for the patient. The inventors have shown that a cleavage product of the MUC1 receptor, that remains attached to the cell surface, referred to herein as the MGFR, functions as a growth factor receptor. When this portion of the receptor is available to activating ligands, cell proliferation is stimulated. The MGFR portion of the receptor can become accessible to activating ligands by a variety of methods. For example, cleavage of the receptor that releases some or all of the IBR makes the MGFR more accessible to activating ligands. Agents that reduced the cell surface expression of the entire MUC1 receptor keeps the receptors too far apart to cluster and thus increases
availability of the PSMGFR and ESMGFR to activating ligands that transduce the signal to the cell to proliferate. Agents that essentially completely inhibit the expression of the MUC1 receptor are good therapeutic candidates are provided. Examples of such inhibitory agents include but are not limited to anti-sense oligos and RNAis, or inhibitory RNAs.

In some cases, the MUC1 receptor may be cleaved to release the IBR or the TPSIBR, from the cell surface. Alternatively, cleavage can result in a release of a sufficient portion of the IBR that causes the MUC1 receptor to lose the ability to self-aggregate. Loss of aggregation of MUC1 may have several ramifications. Release of the IBR or sufficient portion of the IBR from the cell surface allows the receptors to evenly distribute on the cell surface, leaving the cytoplasmic tails free to associate with intracellular signaling proteins. External agents, such as modifying enzymes and/or activating ligands, are then able to bind to the remaining extracellular portion of the receptor and induce disease-associated signals, either via a change in the multimerization state, i.e., inductive multimerization, or as an induced conformational change. As is appreciated by those of ordinary skill in the art, ligands such as growth factors and hormones often induce receptor dimerization which triggers, in turn, an intracellular signaling cascade. Additional support for this mechanism is presented below in data showing that in MUC1+ tumor cell lines and transfected cells expressing truncated isoforms of the MUC1 receptor lacking an IBR, bivalent ligands, such as a bivalent antibody, directed against MGFR trigger signaling, and resulting cell proliferation, through the well-known MAP (mitogen activated protein) kinase signaling cascade, as indicated by detection of phosphorylation of ERK2 kinase. Significantly, such phosphorylation and proliferation was absent or less evident in similar cells treated with monovalent ligands to MGFR, such as a single chain antibody or a monovalent antigen-binding fragment of antibody.

Cell proliferation may result from accessibility of the MGFR portion to an activating ligand which can interact with the MGFR portion. For example, the self-aggregating IBR of the MUC1 receptor may form a dense reticulum which sterically prevents a ligand such as a growth factor from interacting with the MGFR portion of the receptor, which is proximal to the cell relative to the IBR. In a cancerous or tumor cell, this reticulum may be lost, allowing ligand interaction with the MGFR.

The above mechanistic model is consistent with a mechanism whereby the portion of the MUC1 receptor, that remains attached to the cell surface after shedding of the IBR region or the TPSIBR, i.e. the MGFR, functions as a receptor for ligands that trigger cell proliferation. Evidence is also presented herein that demonstrates that: (a) an interaction between a ligand and a portion of the MUC1 receptor (MGFR), which dimerizes the receptor, triggers cell proliferation; and (b) blocking the interaction of this portion of the MUC1 receptor (MGFR) with its ligand(s), blocks cell proliferation. When tumor cell lines, in which the MUC1 receptor is homogeneously expressed across the entire cell surface, are treated with an IgG antibody raised against the MGFR portion of the MUC1 receptor (e.g. PSMGFR), the rate of cell proliferation is greatly enhanced. Since intact IgG antibodies are bivalent, i.e. one antibody simultaneously binds to two adjacent MGFR portions on the cell surface, these results demonstrate that the antibody acts as an activating ligand, mimicing the effect of a growth factor, which dimerizes MGFR portions, and thus triggers a cell proliferation signaling cascade which is consistent with signaling via the cytoplasmic tails of the receptors. This is further supported by the experiments discussed below showing that dimerization of two adjacent MGFR portions on the cell surface induces ERK-2 phosphorylation indicative of MAP kinase cell proliferation signaling (See e.g. Fig. 31). This finding leads to two conclusions. First, an activating ligand(s) that binds to the MGFR portion of the MUC1 receptor causes inductive multimerization of the receptor. Secondly, an effective therapeutic strategy is therefore to block the MGFR portion of the receptor with a monomeric composition, thus preventing inductive multimerization and subsequent signaling cascades. For example, a single chain, or monovalent, antibody, or a monovalent fragment of an intact, bivalent antibody, see discussion of antibodies and antigen-binding fragments thereof below, raised against the MGFR portion of the MUC1 receptor (e.g. raised against PSMGFR or against any peptide comprising a PSMGFR sequence at its N-terminus) would function as an effective anti-cancer therapeutic. Data and examples supporting this contention are presented below. Another therapeutic strategy is to block the activity of enzymes that modify the receptor, which may be required for some ligand binding

The inventors present evidence that dimerization of the MUC1 receptor triggered cell growth in T47D breast tumor cells. Dimerization was achieved in by raising an IgG antibody to MGFR, recall that IgG antibodies are bivalent and therefore can dimerize, a portion of the MUC1 receptor that is proximal to the cell surface. The portion of the MUC1 receptor that was used was the MGFR, and the sequence of the peptide used for generating the antibody was SEQ ID NO: 7 (Table 1- var-PSMGFR). Herein, additional experimental results are presented that support the premise that dimerization of the MUC1 receptor triggers cell proliferation in a number of MUC1⁺ tumor cell lines, while having virtually no effect on cells that do not express or minimally express the MUC1 receptor. MUC1⁺ breast tumor cells, T47D, 1500, 1504, and BT-474 were obtained from the ATCC, as described below in Example 5. As controls, MUC1⁻ breast tumor cells MD-MB 453, HEK (human embilical kidney) cell line K293, and HeLa were also obtained from the ATCC, as described below in Example 5. Western blot analysis, performed as described below in Example 5, confirmed that T47D, 1500, and 1504 cells all expressed high levels of cleaved as well as uncleaved MUC1 and that the control cells did not. Our analysis showed that cell line BT-474 expressed no detectable levels of uncleaved MUC1, but did express an intermediate amount of cleaved MUC1.

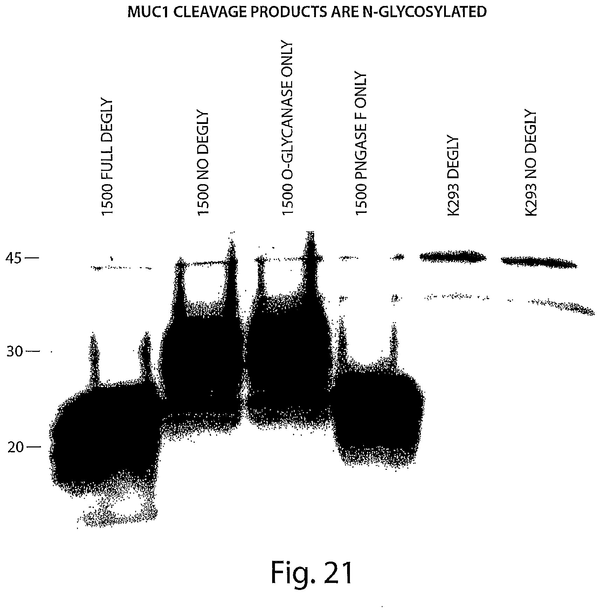

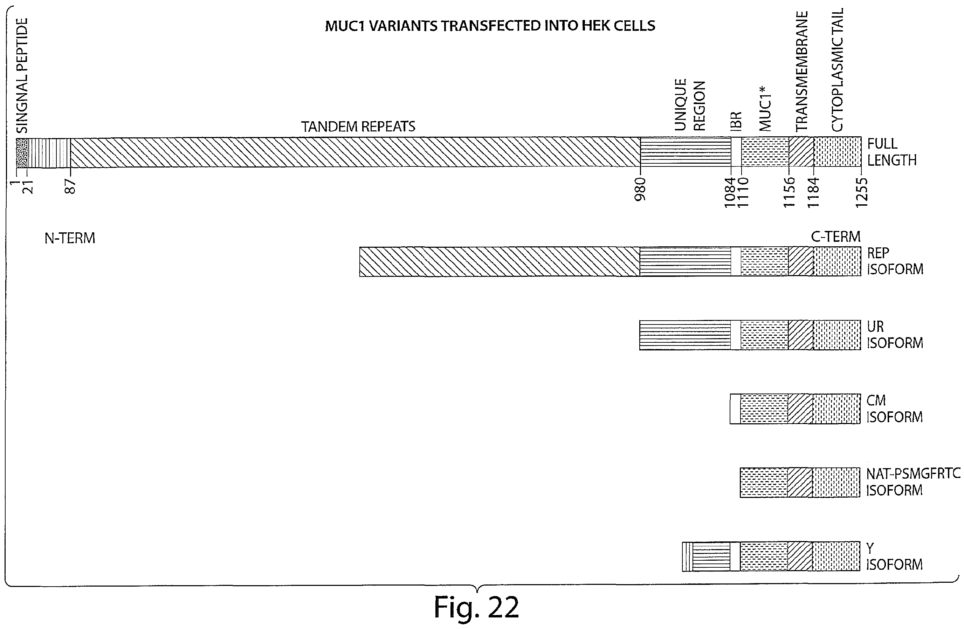

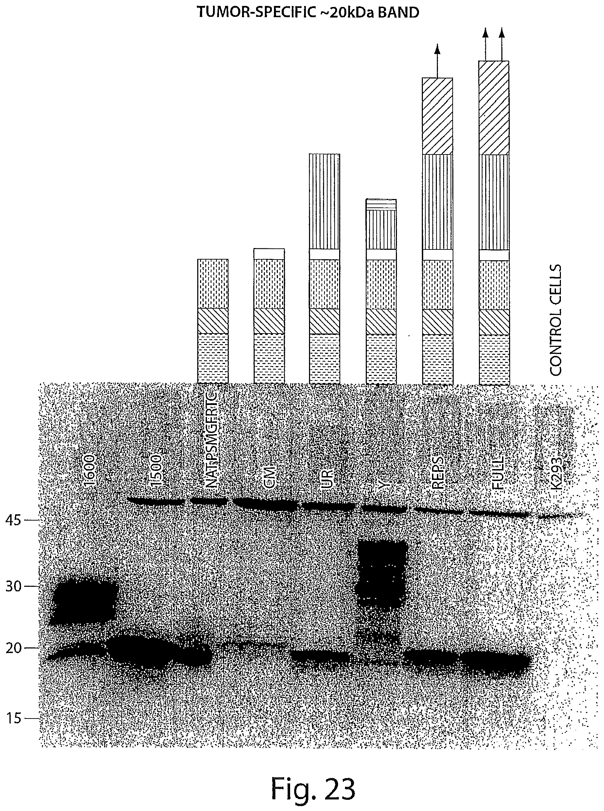

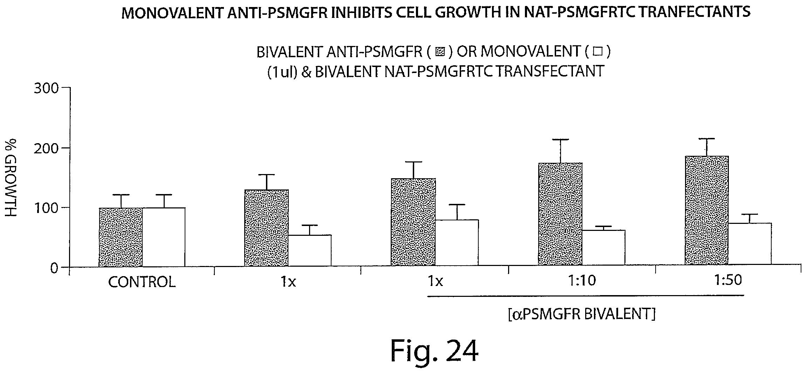

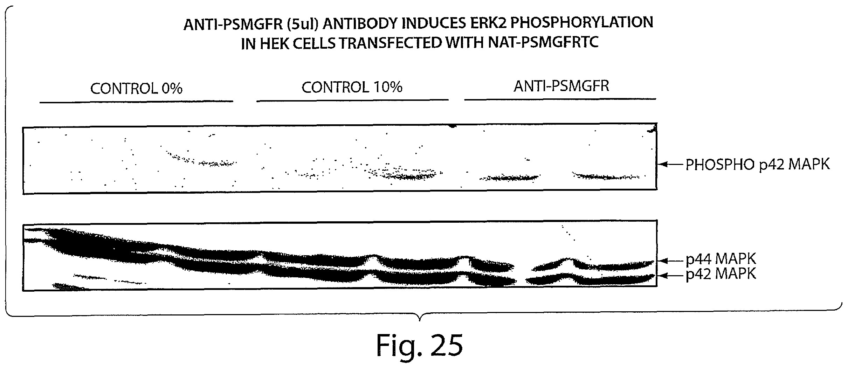

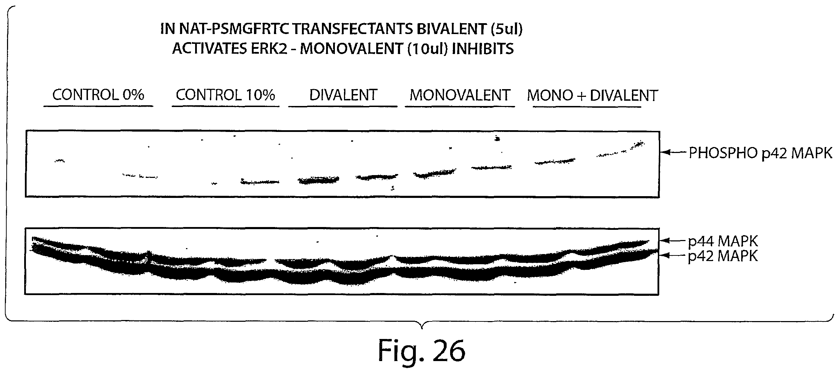

Rabbit polyclonal antibodies were raised against a synthetic peptide the sequence of which was derived from the PSMGFR (var-PSMGFR- SEQ ID NO: 7 of Table 1) by a commercial antibody service company (Zymed, CA), as described below in Example 8. The resultant antibody was purified by affinity chromatography over a column derivatized with the same peptide used to immunize the rabbits. To confirm that the resultant antibody recognized the MGFR of the MUC1 receptor, the antibody was used as the cognate probe in western blots, see Example 5, wherein samples of the immunizing peptide and protein preparations from the MUC1 positive as well as MUC1 negative cell lines were run on a 15% polyacrylimide gel. The bivalent (able to dimerize) antibody was added to the panel of breast tumor cell lines that express the MUC1 receptor along with control cell lines that did not. The addition of the antibody stimulated cell proliferation only in cells that expressed the MUC1 receptor. The 1504 breast tumor cells that were treated for either 5 or 6 days with the bivalent anti-PSMGFR antibody underwent 400% - 600% enhancement of cell growth, see Fig. 21 and Example 11. Still referring to Fig. 21, control cells K293 and Hela were unaffected by the same dosage of the same antibody, anti-PSMGFR. The shape of the proliferation enhancement curves argue that the antibodies dimerize the receptor; at very high antibody concentrations the rate of cell growth decreases, as each receptor is bound to a single antibody rather than one bivalent antibody bound to two receptors. Fig. 22 shows that cells from the breast tumor cell line 1500 underwent a 200% enhancement of cell proliferation after treatment with the bivalent anti-PSMGFR for 3 days. When bivalent anti-PSMGFR treatment was extended for a fourth day, the percentage enhancement of cell growth increased to 300%, see Fig. 23. Breast tumor cells from the T47D cell line were also tested for the ability of the bivalent anti-PSMGFR antibody to trigger cell proliferation. Fig. 24 shows that these cells also underwent an approximately 125% enhancement of cell growth, and as with 1500 and 1504 cell lines, the response was dependent on the concentration of the antibody. Breast tumor cell line BT-474 displayed similar stimulation of cell growth (150 - 200%) in response to bivalent anti-PSMGFR, see Fig. 25. MUC1⁻ cell line MDA-MB-453 was not affected by the addition of anti-PSMGFR at any concentration (data not shown).

Monovalent forms of anti-PSMGFR fragments block cell growth in MUC1 positive tumor cells. As previously described, MUC1⁺ tumor cells are induced to proliferate when the MUC1 receptor is dimerized. Specifically, the signal to proliferate is generated when a portion of the MUC1 receptor proximal to the cell surface is dimerized. As described above, one way in which the receptors can be dimerized is via a bivalent antibody directed against the MUC1 receptor. In a preferred instance, the antibody is directed against the MGFR and in yet a more preferred instance it is directed against at least a portion of the PSMGFR. As described above and further below, agents that bind to the MGFR portion of the MUC1 receptor in a monomeric rather than dimeric fashion can block the dimerization of the receptor and in so doing inhibit cell proliferation. The discussion below describes several chemical compounds that inhibit the growth of MUC1+ tumor cells by binding to the MGFR portion of the MUC1 receptor.

As mentioned above, yet another method of providing an agent that prevents dimerization of the MUC1 receptor is generating a monovalent antibody or a monovalent antigen binding fragment of an antibody directed against the MUC1 receptor. Monovalent antibodies/fragments raised against the MUC1 receptor would be excellent therapeutic agents for MUC1 positive cancers. Monovalent antibodies/fragments that target portions of the receptor proximal to the cell surface are preferred. Especially preferred are monovalent antibodies/fragments that bind to portions of the MUC1 receptor that are C- terminal to the beginning of the repeats section. Still more preferred are monovalent antibodies/fragments that target portions of the receptor C-terminal to the unique region of the MUC1 receptor and yet more preferred are monovalent antibodies/fragments that are directed against the PSMGFR sequence.

Peptides used for antibody production may or may not be glycosylated prior immunizing animals. The sequence of these peptides need not exactly reflect the sequence of MUC1 receptor as it exists in the general population. For example, the inventors observed that antibodies raised against the the PSMGFR peptide variant var-PSMGFR (SEQ ID NO: 7), having an "-SPY-" motif have a higher affinity and greater specificity for the MUC1 protein than antibodies raised against the actual native sequence (i.e. nat-PSMGFR, SEQ ID NO: 36), having an "-SRY-" motif. One may also, in certain cases, introduce mutations into the PSMGFR peptide sequence to produce a more rigid peptide that may enhance antibody production. For example the R to P mutation in the var-PFMGFR sequence of SEQ ID NO: 7 may actually have provided a more rigid peptide and was thus more immunogenic. Another method for producing antibodies against regions of peptides that are not particularly immunogenic, such as the IBR or TPSIBR is to tag the specific peptide sequence with an irrelevant sequence in which the amino acids are of the D-form and thus act to stimulate the immune response of the host animal. Peptide sequences that are used to immunize animals for antibody production may also be glycosylated. The MUC1 peptide sequences that were used herein for drug screening and to generate cognate antibodies were derived from the human species of MUC1. Since there is considerable conservation across species for the PSMGFR and IBR and some portions of the UR, it is anticipated that MUC1 peptides whose sequences are derived from other species can also be used in drug screens and to generate antibodies for these same purposes. Also disclosed is the generation of bi-specific antibodies and bi-specific antibodies formed thereby. Those skilled in the art are familiar with methods to generate antibodies wherein each recognition fragment of a bivalent antibody binds to different but essentially adjacent sites on the same antigen.

As described in greater detail below, MUC1 monovalent antibodies/fragments described above for use as cancer therapeutics may be polyclonal or monoclonal and may be obtained by immunizing a number of different animal species, i.e. rabbit, goat and the like. Additionally, techniques are known to those skilled in the art for generating hybridoma cells, which then are grown and harvested to yield a supply of antibody without the need for repeated animal immunization. Alternatively, humanized monovalent antibodies/fragments, also described in more detail below, that target these portions of the MUC1 receptor may be used as effective anti-cancer agents that are less likely to invoke immune responses in the patient. Methods described herein also encompass recombinant methods for antibody and Fab production that do not include animal immunization.

As explained below, methods of generating monovalent antibodies and monovalent antigen-binding fragments of antibodies are known to those skilled in the art. A standard method is the controlled proteolysis of a bivalent antibody. The inventors generated a monvalent PSMGFR-specific antibody fragment by proteolyzing their bivalent anti-PSMGFR. Monovalent anti-PSMGFR competes with the bivalent anti-PSMGFR antibody for the same binding site within the MGFR portion of the MUC1 receptor.

The present disclosure details how monovalent antibodies/fragments, which are directed against a portion of the MUC1 receptor that is proximal to the cell surface, inhibit the growth of MUC1+ tumor cells. Monovalent antibodies/fragments that targeted the PSMGFR portion of the MUC1 receptor were produced by proteolyzing the bivalent anti-PSMGFR antibody, which has been herein to induce cell growth, presumably by dimerizing the MUC1 receptors. Thus, it follows that the monovalent form of this very antibody would block cell proliferation by binding to the MGFR portion and thus prevent the binding of cognate ligands and/or dimerization.

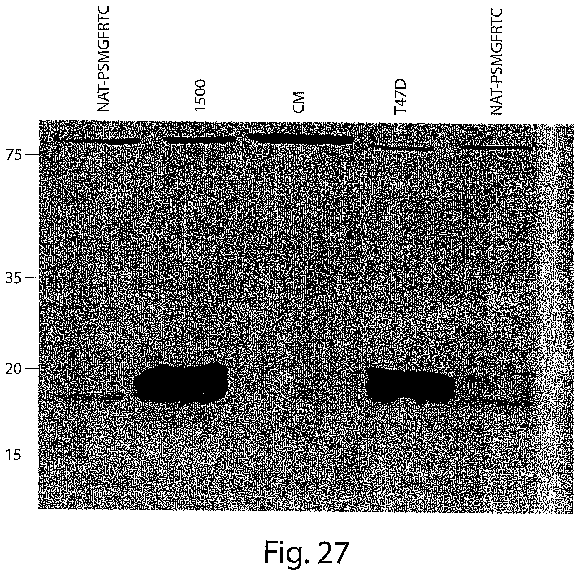

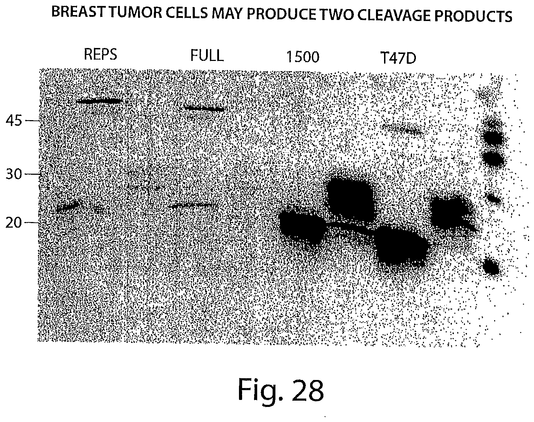

Herein we provide experimental results that demonstrate that monovalent antibody fragments that target the PSMGFR do in fact inhibit the growth of MUC1 positive tumor cells and have virtually no effect on control cell lines. Referring now to Fig. 21, recall that the addition of bivalent anti-PSMGFR induced a 600% enhancement of cell growth. Fig. 27, the method by which the results were generated being described in Ex. 11, shows that the addition of the monovalent form of the same anti-PSMGFR to a MUC1 positive breast tumor cell line, 1504, had the opposite effect in that cell growth inhibited by about 150%, which indicates induced cell death. The addition of the monovalent anti-PSMGFR had a similar effect on breast tumor cell line 1500, which is also MUC1⁺, see Fig. 28.

Monovalent anti-PSMGFR validates the in vitro drug screen; it inhibits the color change of the PSMGFR-immobilized nanoparticles caused by the addition of tumor cell lysates to the nanoparticles, as described in more detail below. It should be noted that the monvalent antibody/fragment also can inhibit the dimerization of the PSMGFR peptide in vitro. Nanoparticle-based drug screening assays to identify compounds that inhibit dimerization of the MGFR portion of the MUC1 receptor are described herein and in commonly-owned U.S. patent application publication no. 2003/0036199; and International Publication No. 02/056022 A2. In certain of these assays, histidine-tagged PSMGFR peptides, (e.g. SEQ ID NO: 2), were immobilized on NTA-Ni++-SAM-coated gold nanoparticles. Lysates and supernatants from MUC1 positive tumor cells, which presumably contain the cognate ligands of the MUC1 receptor, were added to the nanoparticles. Upon addition of the lysate/supernatant mixture, the color of the nanoparticle solution turns from its characteristic pink to blue, presumably when the cognate ligands dimerize MUC1 receptor peptides on two different nanoparticles. The addition of bivalent anti-PSMGFR antibody, in place of the lysate/supernatant solution, also causes the nanoparticle solution to turn from pink to blue, as the bivalent antibody also dimerizes two PSMGFR peptides on different nanoparticles. However, the addition of monovalent anti-PSMGFR to the drug screening assay, to which the lysate/supernatant has also been added, inhibits the color change, presumably by competing with natural, cognate ligands for binding to the PSMGFR peptide. Fig. 29 shows that the characteristic nanoparticle color change that occurs upon the addition of bivalent anti-PSMGFR was inhibited upon addition of the monovalent anti-PSMGFR

The present results also suggest that the MUC1 receptor is involved in apoptosis. The addition of the monvalent anti-PSMGFR not only inhibited cell growth, but also induced cell death. This indicates that the MUC1 receptor also mediates signaling pathways involved in the process of programmed cell death known as apoptosis.

Present cancer research literature presents a confusing picture as to whether or not the overall amount of MUC1 receptor produced by the cell can be correlated to metastatic potential or tumor aggressiveness. The results described herein support the idea that a key mechanism of cell growth in MUC1 positive cancers may depend more on the amount of MUC1 cleavage that occurs rather than the overall amount of MUC1 receptor that is expressed. Low molecular weight species that migrate on an acrylimide gel with an apparent molecular weight of around 20-30 kD (some glycosylated) exist in MUC1-positive tumor cells but do not exist in sufficient numbers to be detectable in non-tumor MUC1 cells. The inventors identified two cleavage sites of the MUC1 receptor in tumor cells. The first cleavage site occurs in the middle of the IBR and the second cleavage site, which our evidence indicates is the more tumorigenic form, occurs at the C-termial end of the IBR: the first cleavage site being located at the N-terminus of TPSIBR (SEQ ID NO: 8) and the second cleavage site being located at the N-terminus of the nat-PSMGFR having SEQ ID NO: 60. When cleavage occurs at the first site, the portion of the receptor that remains attached to the cell surface is the similar to TSESMGFR (See Table 1, SEQ ID NO. 66, but with the native SRY sequence). When cleaved at the second site, the portion that remaining portion is a PSMGFR as shown in Table 1, SEQ ID NO. 63. This low molecular weight species that is tumor specific consists essentially of the native PSMGFR sequence and in some cases the TSESMGFR sequence and is available to cognate ligands, i.e. not self-aggregated, than on the overall amount of MUC 1 receptor expressed by the cell. Supporting this conclusion, susceptibility of tumor cells to proliferate was found, within the context of the present disclosure, to be a function of the amount of the shorter form of the MUC1 receptor.

Comparison of the present results generated by western blot analyses, which quantitated the amount of low molecular weight MUC1 species produced by each cell type tested, with the above presented cell proliferation data shows that the susceptibility of the breast tumor cells to antibody-induced cell growth is proportional to the amount of the low molecular weight MUC1 species (25-30 Kd glycosylated; 19-20Kd unglycosylated) that the cell produces. Referring now to Fig 30, breast tumor cell lines 1500 and 1504 produce a considerably greater amount of the MUC1 cleavage product that runs at 19-20 Kd than the BT-474 BT cells or the control K293 and HeLa cells. Correspondingly, the anti-PSMGFR-induced increase in the proliferation of cell lines 1500 and 1504 was up to about 400% (Fig. 23) and up to about 600% (Fig. 21) respectively, while there was no detectable increase in the rate of cell growth for control cells (Fig. 21) and the growth of BT-474 cells increased by only up to about 200%, see Fig. 25.

In further support of the conclusion that cleavage products of the MUC1 receptor function as growth factor receptors in tumor cells, HEK cells were transfected with MUC1 variants that were either terminated after the PSMGFR (see Table 1, SEQ ID NO: 37) or after the entire interchain binding region (PSIBR) (SEQ ID NO:38). Cells transfected with the receptor that included the PSIBR grew at a rate 4-6 times slower than cells transfected with the MUC1 variants that were terminated after the PSMGFR (e.g. SEQ ID NO: 37). These results support the conclusion that the portion of the MUC1 receptor that acts as a growth factor receptor is a cleavage product in which much or all of the IBR is released from the cell surface. Further, these results support the conclusion that tumors in which a good percentage of the MUC1 receptors have been cleaved to release the TPSIBR (SEQ ID NO: 65) are especially aggressive cancers and those that are cleaved to release the entire IBR, leaving PSMGFR (SEQ ID NO: 63) attached to the cell surface are even more aggressive. Therefore, antibodies that are raised against the TPSIBR (SEQ ID NO: 65) portion of the MUC1 receptor can be used to assess the aggressiveness of cancers that are MUC1-positive.

Consistent with these findings, the amount of MGFR that is accessible on cells (tissues) predicts tumor aggressiveness and metastatic potential. Therefore, antibodies that recognize the MGFR portion of the receptor can be used to diagnose cancer or the propensity to develop cancer, to predict cancer aggressiveness and metastatic potential, to suggest therapeutic protocols and to track the progress of the therapeutic protocols.

Consequently, the aggressiveness or metastatic potential of tumor cells can be assessed by determining the amount of lower molecular weight MUC1 species that the cells produce. This can be determined, for example by SDS-PAGE analysis or western blot analysis using antibodies or antigen-binding fragments thereof raised against the MGFR portion of the receptor. In certain cases, the colloid assay techniques described herein could be utilized in which the antibodies/fragments are attached to a carrier that can be a nanoparticle or colloid. In a preferred instance, a patient's cells are probed with antibodies or antigen-binding fragments thereof directed toward the MGFR as this method reveals the amount of MGFR-containing MUC1 that remains attached to the cell surface and, importantly, whether or not it is accessible to cognate ligands. In a yet more preferred instance, a patient's cells are probed with antibodies or antigen-binding fragments thereof directed toward the PSMGFR. In practice, cells that display a high degree of MUC1 receptor that reacts with antibodies or antigen-binding fragments thereof that recognize the PSMGFR, or a portion thereof, is an indication that the MUC1 receptors present on these cells have undergone of a greater degree of cleavage, leaving the PSMGFR portion accessible to cognate ligands that activate a cell growth pathway. Tumors comprised of cells thusly characterized are of higher metastatic potential and/or are more aggressive. Therefore, using antibodies or antigen-binding fragments thereof that recognize the PSMGFR, or portions thereof, can be used to diagnose the metastatic potential or aggressiveness of a patient's tumor.
In certain cases, provided are antibodies or antigen-binding fragments thereof. In one case, provided is an antibody or antigen-binding fragment that specifically binds to MGFR. In certain cases, such an antibody or antigen-binding fragment thereof is bivalent, while in other cases it is monovalent. In certain cases, the above-mentioned antibodies or antigen-binding fragments thereof specifically bind to PSMGFR. In certain such cases, the antibodies or antigen-binding fragments thereof can specifically bind to the amino acid sequence set forth in SEQ. ID. NO.: 36 or a functional variant or fragment thereof comprising up to 15 amino acid additions or deletions at its N-terminus or comprising up to 20 amino acid substitutions; in other cases, it specifically binds to the amino acids set forth in SEQ. ID. NO.: 36 or a functional variant or fragment thereof comprising up to 10 amino acid substitutions; in other cases, the antibodies or antigen-binding fragments thereof specifically bind to the amino acid set forth in SEQ. ID. NO.: 36 or a functional variant or fragment thereof comprising up to 5 amino acid substitutions; and in yet another case the antibodies or antigen-binding fragments thereof specifically bind to the amino acid sequence set forth in SEQ. ID. NO.: 36. In certain cases, the antibody or antigen-binding fragment described herein is a human, humanized, xenogenic or a chimeric human-non-human antibody or antigen-binding fragment thereof. In certain cases, the antibodies or antigen-binding fragments thereof comprise an intact antibody or an intact single-chain antibody. For antibodies or antigen-binding fragments that are monovalent, in certain cases, they may comprise a single-chain Fv fragment, a Fab' fragment, a Fab fragment, or a Fd fragment. For antibodies or antigen-binding fragments described herein that are bivalent, certain instances comprise an antigen-binding fragment that is a F(ab')₂.

Also provided are, in certain cases, compositions comprising the antibody or antigen-binding fragments as described herein as an ingredient. In certain cases, such compositions comprise pharmaceutical compositions and further comprise a pharmaceutically-acceptable carrier. In certain such compositions, the antibody or antigen-binding fragment thereof can be polyclonal, while in other casesit can be monoclonal.

Also provided are, in certain cases, a variety of kits, in certain casesincluding any of the above-mentioned antibodies or antigen-binding
fragments thereof. In certain cases, such kit may also provide an article having a surface. In certain such cases, the antibody or antigen-binding fragment thereof can be fastened or adapted to be fastened to the surface of the article. In certain cases, the article comprises a particle. In such case, the kit further includes a second particle and a peptide sequence comprising a portion of a cell surface receptor that remains attached to the cell surface after shedding of the cell surface receptor interchain binding region, the peptide sequence being detached from any cell, and fastened to or adapted to be fastened to the second particle. In some instances, the kit may further include a candidate drug for affecting the ability of the peptide sequence to bind to other identical peptide sequences, and/or to the antibody or antigen-binding fragment thereof, in the presence of the antibody or antigen-binding fragment thereof. The peptide sequence provided can comprise, in certain cases, MGFR. In some of the above-described kits including a particle, the kit may further comprise a peptide sequence comprising a portion of a cell surface receptor that remains attached to the cell surface after shedding of the cell surface receptor interchain binding region, such peptide sequence being detached from any cell, and fastened to or adapted to be fastened to the particle. The above kit may, in certain instances, further comprise a second particle and have the peptide sequence mentioned above fastened to or adapted to be fastened to the second particle. The above-mentioned kits may be useful for performing various diagnostic, drug screening and other assays, which can involve colloid-colloid interactions and/or aggregation, as described in detail herein.

Also provided are, in certain cases, methods for producing or generating antibodies or antigen-binding fragments thereof that specifically bind to certain peptides, for example certain peptides disclosed herein. One particular instance, an antibody or antigen-binding fragment is raised against a peptide including a portion of a cell surface receptor that interacts with an activating ligand such as a growth factor to promote cell proliferation, such portion including enough of the cell surface receptor to interact with the activating ligand and being free of an interchain binding region to the extent necessary to prevent spontaneous binding between such portions. In certain such methods, the cell surface receptor comprises MUC1, in other instances MGFR, and in yet other such methods a peptide comprising PSMGFR at it N-terminus; in yet other cases, the peptide comprises at its N-terminus the amino acid set forth in SEQ. ID.
NO.: 36 or a functional variant or fragment thereof comprising up to 15 amino acid additions or deletions at its N-terminus and comprising up to 20 amino acid substitutions. In certain instances of the methods for producing antibodies or antigen-binding fragments thereof, an antibody or antigen-binding fragment is raised against PSMGFR. In certain such instances, such peptides used to generate the antibody or antigen-binding fragment thereof can consists of the amino acid sequence set forth in SEQ. ID. NOS.: 36 or 37 or a functional variant or fragment thereof that comprises up to 15 amino acid additions or deletions at its N-terminus and up to 20 amino acid substitutions.

In yet other instances, provided are methods for treating a subject having a cancer or other condition requiring treatment with one or more of the antibodies or antigen-binding fragments thereof. In one such case, provided is a method for treating a subject having a cancer characterized by the aberrant expression of MUC1. The method involves administering to the subject an antibody or antigen-binding fragment thereof in an amount effective to ameliorate the cancer. In certain such cases, the antibody or antigen-binding fragment thereof is administered in an amount effective to reduce tumor growth. In certain cases, any of the above-mentioned antibodies or antigen-binding fragments thereof, especially those which specifically bind to MGFR, PSMGFR, etc. can be used. In certain preferred cases, the antibody or antigen-binding fragment thereof is administered in an amount effective to block the interaction of a natural ligand with a portion of a MUC1 receptor, for example, MGFR, that remains attached to a cell after shedding of a interchain binding region of the MUC1 receptor. In other cases, the method involves administering an antibody or antigen-binding fragment thereof that is effective to reduce shedding of an interchain binding region of a MUC1 receptor. In many such instancesof the method, particularly those in which the antibody or antigen-binding fragment thereof specifically binds to MGFR, such a treatment method can involve administering to the subject the antibody or antigen-binding fragment thereof in an amount effective to prevent inductive dimerization of a cancer-associated growth factor receptor, such as aberrantly cleaved MUC1.

In yet another method provided, the above-described antibodies or antigen-binding fragments thereof can be utilized in a method for determining the aggressiveness and/or metastatic potential of a cancer. In one such method, a sample obtained from a subject having or suspected of having a cancer is contacted with an
antibody or antigen-binding fragment thereof as described herein that specifically binds to a peptide associated with the cancer that is expressed on the cell surface. The method involves determining an amount of the antibody or antigen-binding fragment that specifically binds to the sample, such amount being indicative of the aggressiveness and/or metastatic potential of the cancer. Certain such cases, the sample utilized comprises cells of the subject and/or a solublized lysate thereof. In certain such cases, the peptide expressed on the cell surface can include a portion of a cell surface receptor that interacts with an activating ligand such as a growth factor to promote cell proliferation, wherein the portion includes enough of the cell surface receptor to interact with the activating ligand while being free of any interchain binding region to the extent necessary to prevent spontaneous binding between portions. In certain such cases, the cell surface receptor is MUC1 and the peptide comprises MGFR or a peptide comprising PSMGFR at its N-terminus. In certain preferred instances of such methods, the antibody or antigen-binding fragment thereof can be immobilized relative to or adapted to be mobilized relative to a signaling entity, such as any of the signaling entities described previously. In certain such instances, the signaling entity can comprise one or more particles, such as colloid particles.

Provided are peptide binding agents which, for example, can be antibodies or fragments of antibodies having the ability to selectively bind to PSMGFR and/or MGFR. Antibodies include polyclonal and monoclonal antibodies, prepared according to conventional methodology.

Significantly, as is well-known in the art, only a small portion of an antibody molecule, the paratope, is involved in the binding of the antibody to its epitope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule and comprises one type of monovalent antibody fragment as provided. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation. Accordingly, a monovalent antibody fragment according to certain instancesmay be an Fd fragment.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity.

As is now well known in the art, the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody. See, e.g., U.S. patents 4,816,567, 5,225,539, 5,585,089, 5,693,762 and 5,859,205. Such antibodies, or fragments thereof are within the scope of the present disclosure.

In certain instances, fully human monoclonal antibodies also can be prepared by immunizing mice transgenic for large portions of human immunoglobulin heavy and light chain loci. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (HAMA) responses when administered to humans.

Certain cases comprise methods for producing the provided antibodies, or antigen-binding fragments thereof, that include any one of the step(s) of producing a chimeric antibody, humanized antibody, single-chain antibody, Fab-fragment, F(ab')₂ fragment, bi-specific antibody, fusion antibody, labeled antibody or an analog of any one of those. Corresponding methods are known to the person skilled in the art and are described, e.g., in Harlow and Lane "Antibodies, A Laboratory Manual", CSH Press, Cold Spring Harbor, 1988. The production of chimeric antibodies is described, for example, in WO89/09622. Methods for the production of humanized antibodies are described in, e.g., EP-A1 0 239 400 and WO90/07861. A further source of antibodies to be utilized in accordance with the present disclosure are so-called xenogeneic antibodies. The general principle for the production of xenogeneic antibodies such as human antibodies in mice is described in, e.g., WO 91/10741, WO 94/02602, WO 96/34096 and WO 96/33735. As discussed below, the antibodies, described herein may exist in a variety of forms (besides intact antibodies; including, for example, antigen binding fragments thereof, such as Fv, Fab and F(ab')2, as well as in single chains (i.e. as single chain antibodies); see e.g., WO88/09344.

Thus, as will be apparent to one of ordinary skill in the art, the present disclosure also provides, in certain instances, for F(ab')₂, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')₂ fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. Also included are so-called single chain antibodies.

Moreover, certain cases relate to compositions comprising the aforementioned antibodies or antigen-binding fragments or chemical derivatives thereof. The composition may further comprise a pharmaceutically acceptable carrier. The term "chemical derivative" describes a molecule that contains additional chemical moieties that are not normally a part of the base molecule. Such moieties may improve the solubility, half-life, absorption, etc. of the base molecule. Alternatively the moieties may attenuate undesirable side effects of the base molecule or decrease the toxicity of the base molecule. Examples of such moieties are
described in a variety of texts, such as Remington's Pharmaceutical Sciences. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. Aerosol formulations such as nasal spray formulations include purified aqueous or other solutions of the active agent with preservative agents and isotonic agents. Such formulations are preferably adjusted to a pH and isotonic state compatible with the nasal mucous membranes, e.g., for intranasal administration. Formulations for rectal or vaginal administration may be presented as a suppository with a suitable carrier.

A therapeutically effective dose refers to that amount of antibodies and/or antigen-binding fragments described herein ameliorate the symptoms or conditions of the cancer or other disease being treated. Therapeutic efficacy and toxicity of such compositions can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The biological activity of the antibodies and/or antigen binding fragments thereof, as described herein, indicates that they may have sufficient affinity to make them candidates for drug localization to cells expressing the appropriate surface structures, e.g. MGFR. Thus, targeting and binding to cells of the antibodies and/or antigen binding fragments thereof, described herein could be useful for the delivery of therapeutically or diagnostically active agents (including targeting drugs, DNA sequences, RNA sequences, lipids, proteins and gene therapy/gene delivery. Thus, the antibody and/or antigen binding fragments thereof, described herein can be labeled (e.g., fluorescent, radioactive, enzyme, nuclear magnetic, colloid, other signaling entity, etc.) and used to detect specific targets in vivo or in vitro including "immunochemistry" like assays in vitro. In vivo they could be used in a manner similar to nuclear medicine imaging techniques to detect tissues, cells, or other material expressing MGFR. Another method involves delivering a therapeutically active agent to a
patient. The method includes administering at least one antibody or an antigen-binding fragment thereof and the therapeutically active agent to a patient. Preferably, the therapeutically active agent is selected from drugs, DNA sequences, RNA sequences, proteins, lipids, and combinations thereof.

In certain cases, also provided are drug screening assays, treatment protocol screening assays, diagnostic assays, etc. that involve determining whether or not an intracellular protein has become chemically modified in a manner indicative of its participating in an intracellular signaling pathway. One such method involves providing a cell expressing on its surface a peptide that can act as a growth factor receptor, such as MUC1. The assay involves contacting such a cell with a candidate drug for affecting the ability of an activating ligand of the cell surface peptide to interact with the peptide, in the presence of the activating ligand, and determining whether an intracellular protein that becomes phosphorylated if the activating ligand interacts with the cell surface peptide, in fact, becomes phosphorylated. Such method is especially useful for cells expressing MGFR, or a peptide comprising PSMGFR at its N-terminus, such as PSMGFRTC.

As described below, for instances involving MUC1-expressing cells, intracellular cell proliferation signaling occurs via the MAP kinase pathway and interaction of the cell surface receptor with its ligand, and associated inductive multimerization, involves phosphorylation of an intracellular protein comprising ERK-2. In certain such cases, the screening method utilizes a sample comprising a plurality of cells, which may, in certain instances, be lysed or permeablized, after having being exposed to the candidate drug and activating ligand. In certain instances, after exposure to the drug candidate and activating ligand and following cell lysis or permeabilization, the method involves separating proteins contained in intracellular contents of the cells on a gel, for example using Western blot techniques, and visualizing or otherwise detecting the separated proteins. Such detection can be effected, as well understood by those of ordinary skill in the art utilizing antibodies or other molecules that specifically bind to the intracellular proteins to be detected. In certain cases, such molecules can be antibodies or antigen-binding fragments thereof, preferably including an auxiliary signaling entity permitting detection or visualization, such as one of those described previously. In certain instances, detecting phosphorylation of the intracellular protein after gel
separation involves contacting the gel-separated proteins with a biological molecule, such as the above-mentioned antibodies, etc. that specifically bind to a phosphorylated form of the intracellular protein (e.g., phos-ERK-2, but not to the intracellular protein when it is not phosphorylated). Western blot techniques useful for performing the above-described method are well known to those skilled in the art and are described in more detail below in Example 5.

In certain instances, instead of using the above-described gel-based method for determining phosphorylation of the intracellular protein within the context of the present assays, a colloid-based aggregation assay, similar to those described elsewhere and herein, can be utilized for detecting the presence of the phosphorylated form of the intracellular protein. In such instances, after the above-described step of lysing or permeabilizing the cells exposed to the activating ligand and candidate drug, the intracellular contents are contacted with a plurality of colloid particles. The plurality of colloid particles preferably includes a first subset thereof that are immobilized relative to a first biological molecule, such as an antibody or antigen-binding fragment thereof, that specifically binds to a phosphorylated form of the intracellular protein but not to the intracellular protein when it is not phosphorylated, and to a second subset of colloid particles that are immobilized relative to another biological molecule, e.g., antibody or antigen-binding fragment thereof, that specifically binds to the intracellular protein at an epitope that is different from that to which the first biological molecule specifically binds. If the sample includes a phosphorylated form of the intracellular protein, the colloids will aggregate and a color change will be observed. However, if the intracellular protein is not phosphorylated, no aggregation indicative of cross-linking of the colloid particles to each other will be observed.

Such methods as described above can enable these methods to facilitate simultaneously determining whether a drug candidate suspected of having the ability to interfere with the binding of an activating ligand to a cell surface receptor interferes with the binding of the activating ligand to the cell surface receptor, and whether the drug candidate acts by interacting with the cell surface receptor or with the ligand. In short, most advantageously when the present method is performed under conditions of excess ligand concentration (as compared to drug candidate concentration), the above-described methods for drug screening based on detection of phosphorylation, or other modification, of intracellular proteins will tend to show a positive test result only when the candidate drug acts by interacting directly with, e.g. by becoming immobilized relative to, the cell surface receptor, as opposed its acting via a mode of action wherein the candidate drug binds to or otherwise interacts with the ligand. This one-step behavior will be best observed whenever the screening assay is performed utilizing the cell surface receptor ligand in sufficient excess such that any binding to the ligand by the candidate drug, which may prevent binding of the ligand to the receptor, would not reduce the ligand available for receptor binding and inductive multimerization within the assay system.

Moreover, such a colloid-based assay as described immediately above is not limited to assays and systems for detecting intracellular signaling via phosphorylation of intracellular proteins. The above-described colloid-based assays are more generally applicable. For example, such an assay method can involve a screening test for determining the modification state of essentially any biological molecule. Such a screening method can involve, in certain cases, assays involving detection of immobilization of a colloid particle relative to a biological molecule, wherein the colloid particle is configured such that it becomes immobilized with respect to the biological molecule when the biological molecule is in a first modification state to a different extent than when the biological molecule is in a different modification state.

Modification states that may be determined using such methods include whether or not a particular biological molecule is phosphorylated, glycosylated, acetylated, etc. Moreover, while, in preferred instances, such colloid-based assays involve colloid-colloid aggregation for detection, in other instances, the colloid-utilized may simply be used as a signaling entity for detecting whether or not the biological molecule under evaluation is modified or not by using Western blotting or another gel-based assay, etc. For example, in one such assay, biological molecules whose modification state is to be tested can be contacted with an agent, such an antibody, that specifically binds to the biological molecule when it is in a first state of modification but not when it is in a second state of modification. Such an agent could, in certain instances, be immobilized relative to a colloid particle, which could provide a signaling entity able to be detected, for example, in a gel; or, alternatively, the colloid could be immobilized with respect to another binding entity, such as a secondary antibody, having specificity for the agent binding to the biological molecule whose modification state is being determined.

In certain instances of such a method - utilizing a colloid-aggregation detection assay for determining the modification state of the biological molecule - a sample containing the biological molecule is contacted with a plurality of colloid particles of at least a first and a second type. A first subset (type) of the colloid particles is immobilized relative to a first agent that specifically binds to the biological molecule when it is in a first state of modification but not to the biological molecule when it is in the second state of modification, and a second set (type) of the colloid particles is immobilized relative to a second agent that specifically binds to the biological molecule at an epitope that is different from the epitope at which the first agent specifically binds. As described above, in such a test, if the biological molecules, or a subset thereof, are in an first state of modification, the plurality of colloid particles, as described above, will tend to aggregate, in proportion of the concentration of the biological molecule in the first state of modification, thereby causing a color change in the assay sample. However, if the biological molecule is present only in the second state of activation, the first type of colloid will not become bound to it and no cross-linking or aggregation of the colloids, or color change resulting therefrom, will occur. In certain instances of such an assay as described above, the first agent on the first subset of colloid particles would be an antibody or other binding entity that specifically binds to the biological molecule only when it is in the first state of modification, and the second agent on the second subset of colloid particles could be an agent that binds to the biological molecule in either the first state of modification or the second state of modification.

Such a colloid aggregation assay as described immediately above can be advantageously employed for determining, for example, which of a plurality of intracellular signaling pathways is activated upon binding of an activating ligand to a cell surface receptor. In such assays, a plurality of different types of colloid particles could be employed including a plurality of different first and second colloid types having agents immobilized thereon able to detect a plurality of different modification states of a plurality of different intracellular signaling proteins enabling determination of the activity of various cell signaling pathways within a cell to be determined, in certain cases in a single assay.

As discussed above, one aspect of the present disclosure involves the discovery that dimerization of the extracellular portion of the MUC1 receptor activates the MAP kinase pathway within the cell, which is a known signaling cascade that induces cell proliferation. The present disclosure also, in certain cases, involves various diagnostic assays, drug and treatment screening protocols, etc., related to the discovery by the inventors that dimerization of the MUC1 receptor triggers cell proliferation via the MAP (mitogen activated protein) kinase cell proliferation signaling pathway. More specifically, dimerization of the MGFR portion of the receptor is necessary and sufficient to activate the MAP kinase pathway and induce cell proliferation. The MAP kinase signaling cascade is one of the intracellular signaling pathways that is fairly well understood. To summarize, a mitogen binds to the extracellular portion of a transmembrane receptor and alters its conformation in such a manner that a signal is then transduced to the cell interior. As described above, a downstream step in this cascade is the phosphorylation of ERK2. It is known in the art that once ERK2 has been phosphorylated, cell proliferation proceeds. The inventors demonstrate that the addition of a bivalent antibody, which recognizes the MGFR, dimerizes the MUC1 receptor and in some way generates or reveals binding sites for signaling proteins that bind to the cytoplasmic tails of the MUC1 receptor. Figs. 31-33, respectively, show that in T47D, 1504, and 1500 breast tumor cells, dimerization of the MUC1 receptor via bivalent anti-PSMGFR results in ERK2 phosphorylation, see Ex. 12. The effect is dose-dependent and time-dependent. Further, synthetic compounds, which the inventors previously showed bind to the MGFR portion of the MUC1 receptor, compete with the bivalent anti-PSMGFR for binding to this region of the MUC1 receptor. In a competitive inhibition assay, the compounds effectively prevent (also in a dose-dependent way) the binding of the bivalent antibody to the MGFR, resulting in a loss of dimerization of the receptor and a loss of ERK2 phosphorylation, see Fig. 34 and Ex. 12.

Additionally, the monovalent form of the anti-PSMGFR competed with the bivalent antibody and effectively inhibited ERK2 phosphorylation. When excess monovalent anti-PSMGFR was added to breast tumor cells along with the amount of bivalent anti-PSMGFR that was shown to be sufficient to stimulate ERK2 phosphorylation, that phosphorylation was blocked, presumably because the monovalent antibody blocked the dimerization of the MUC1 receptor. Fig. 35 shows that monovalent anti-PSMGFR competes with the bivalent antibody and blocks the phosphorylation of ERK2 in cell line 1500.

Accordingly, in certain cases, the phosphorylation state of ERK2 can be monitored as a method for identifying therapeutics for MUC1 positive cancers. It was described above that monovalent compounds and monovalent anti-PSMGFR
that bound to the MGFR competed with the bivalent antibody for binding to the site and in so doing inhibited the activation of the MAP kinase signaling pathway; ERK2 phosphorylation did not occur. This suggests a drug screen that will identify agents that affect signaling through the MUC1 receptor. In this drug screen, bivalent anti-PSMGFR is added to MUC1 positive tumor cells. Drug candidates are also added and the phosphorylation state of ERK2 is measured, as described previously. Cells in which ERK2 phosphorylation is inhibited indicates that that drug candidate successfully competed with the bivalent antibody for binding to the MGFR and in so doing inhibited its dimerization and subsequent activation of the MAP kinase signaling pathway. This drug screen also can identify compounds that act on intracellular proteins that affect the ERK2 arm of the MAP kinase signaling pathway.

Monitoring levels of ERK2 phosphorylation, induced by adding the bivalent anti-PSMGFR, also provides a method for determining which compounds identified as being able to inhibit the MUC1 cell proliferation pathway do so by directly binding to the MGFR rather than to an associated factor such as the ligand.

In addition, a more efficacious MUC1+ cancer treatment protocol can result from simultaneously treating the patient with drugs that target: the MUC1 receptor, signaling elements within the MAPkinase/ERK2 pathway, and/or drugs that target the ligands to MGFR

Also provided is an agent that binds together MGFR portions of MUC1 following disease-associated cleavage to effect preventative clustering of the receptors. The agent can be any species that includes multiple sites each able to bind to a MFGR portion, and immobilized with respect to each other. E.g. a polymer or dendrimer or other continuous entity can include multiple sites each able to bind to a MGFR portion, causing clustering of these portions or other structural constraint that inhibits their association with factors that promote cell proliferation. Alternatively, IgM-type monoclonal or polyclonal antibodies raised against the MGFR or PSMGFR could be utilizied. Each anti-MGFR IgM antibody could be able to aggregate ten MGFRs on the cell surface to form preventative clusters.

In addition, some or all of the above-identified antibodies, or antigen-binding fragments thereof directed that were specifically bind to the MGFR portion of the MUC1 receptor can be modified to allow the antibodies, or antigen-binding fragments to act as a targeted delivery agent by attaching a cytotoxic drug or other agent (e.g. a radioactive substance) able to selectively kill cells to which the ligands become immobilized. In this way, such a therapeutic can be directed to the tumor cells. For example, an agent that binds to the MGFR region of the MUC1 receptor can be modified with a radioactive substance to destroy tumor cells that aberrantly express the MUC1 receptor. Other toxic substances, such as ricin, as well as other therapeutics, can be attached to agents that bind the MGFR. Alternatively, antibodies, or antigen-binding fragments that bind to the MGFR could be modified to present a imaging agent for use in diagnostic imaging of MUC 1⁺ tumors and metastases. Such antibodies, or antigen-binding fragments can also, alternatively, be modified to act as drugs that can be useful for prevention and/or treatment of cancer. In one instance, an antibodies, or antigen-binding fragments, which in its unmodified form binds to multiple MGFRs causing inductive multimerization, is modified to remove or de-activate all but one of its active binding sites for MGFR, such that each modified antibody or antigen-binding fragment is able to bind to only a single receptor. A specific example of this would be the production of monovalent fragments of an anti-MGFR IgG via, for example, enzymatic or other cleavage methods. In another instance, individual antibody or antigen-binding fragment are modified such that they are immobilized with respect to additional ligand molecules/peptides also able to bind MGFR, e.g. through covalent coupling, noncovalent coupling, co-immobilization with respect to a substrate, etc., such that the modified, multi-unit ligand is able th effect preventative clustering of the receptors to which it binds.

Identification of ligand(s) for the portion of MUC1 that remains bound to the cell after cleavage can allow for development of powerful assays to screen for drugs that disrupt this interaction. Interaction of potential binding partners with the extracellular portion of MUC1 that remains after cleavage can be studied both by conventional techniques (western blotting, ELISA, MALDI, etc.) and using our colloid-colloid color change assay or colloid-bead coloration assay. The peptide sequence of the remaining extracellular portion of MUC1 can be attached to beads or colloids via a histidine tag. Potential binding partners can be histidine-tagged and attached to a second set of colloids (or beads) and assayed for binding to the colloid-immobilized portion of MUC1. Alternatively, potential binding partners can be attached to beads or colloids by EDC/NHS coupling or can be nonspecifically adsorbed to beads for the assay. An interaction between the MUC1 peptide and the potential binding partner can be detected by either a change in solution color (for the colloid-colloid assay) or by agglomeration of the colloids onto the bead, causing the bead to appear red (for the colloid-bead assay). An entire cDNA library can be screened using this technique in a short period of time to identify the natural ligand of the remaining extracellular MUC1. (see PCT/US00/01997, WO 00/34783, 09/631,818, and "Detection of Binding Species with Colloidal and Non-Colloidal Structures", filed 11/15/00).

In certain cases, biopsy specemins can be studied, or tissue can be studied interoperatively (e.g. tissue at a surgical site can be studied without removal of the tissue from the subject) to determine tumorigenesis or potential for tumorigenesis. In either of these studies, a primary indicator of tumorigenesis or potential for tumorigenesis is the amount of MGFR at a cell surface accessible to interaction with external agents such as growth factors, etc. This determination can be made, for example, by determining the amount of an antibody to the MGFR region that binds to the sample, either using standard antibody binding study techniques, or by exposing the sample to colloids to which antibodies specific to the MGFR region have been immobilized and determining binding of the colloids to the samples using techniques described in International patent publication numbers WO 00/34783 and WO 00/43791, referenced above. In another technique (perhaps more suited for an excised sample), antibodies to the MGFR region and to the IBR can be exposed to the sample and a determination made of the ratio of binding of each to the sample. A healthy sample will exhibit little or no antibody binding to the MGFR region. A sample indicating tumerigenesis or potential for tumorigenesis will show a non-zero ratio of MGFR antibody binding to IBR antibody binding.

One aspect of the disclosure is the identification of antibodies or antigen-binding fragments thereof that directly bind to the MGFR portion of the MUC1 receptor. Therefore, a sensitive method for diagnosing early tumors is to administer to the patient, antibodies or antigen-binding fragments thereof that bind to a PSMGFR that have also been derivatized with contrast or imaging agents. These antibodies or antigen-binding fragments thereof will agglomerate onto tumors wherein this portion of the MUC1 receptor is accessible. Antibodies or antigen-binding fragments thereof described herein that bind to the MGFR region as well as other compounds that can be identified using methods disclosed herein can be readily modified to carry imaging agents. Such imaging agents may include but are not limited to, technetium, rhenium, ¹²³I, and other contrast agents or radioactive entities commonly used in imaging techniques. Imaging techniques include but are not limited to single photon computed tomography (SPECT), MRI, microscopy and the like. In some applications, an attached colloid can act as an imaging agent. Since the carrier for the imaging agent can also be a therapeutic, this technique can combine an early diagnostic with a directed therapeutic.

A series of isolated proteins or peptides is provided. Provided peptides may include, but are not limited to, those defined above as PSMGFR and PSMGFRTC, and those listed as SEQ ID NOs: 1, 2, 3, 4, 5, 6, 36, 7, 8, 9, 37, 38, 39, 40, 41, 47, 60-66 and 14-35. Additionally, any protein or peptide, not specifically mentioned above that is encoded by any of the isolated nucleic acid molecules discussed below, is encompassed by the present discussion. Also encompassed by the present discussion are unique fragments of the above-mentioned proteins or peptides.

Proteins can be isolated from biological samples including tissue or cell homogenates, and can also be expressed recombinantly in a variety of prokaryotic and eukaryotic expression systems by constructing an expression vector appropriate to the expression system, introducing the expression vector into the expression system, and isolating the recombinantly expressed protein. Short polypeptides, including antigenic peptides (such as are presented by MHC molecules on the surface of a cell for immune recognition) also can be synthesized chemically using well-established methods of peptide synthesis.

Thus, as used herein with respect to proteins, "isolated" means separated from its native environment and present in sufficient quantity to permit its identification or use. Isolated, when referring to a protein or polypeptide, means, for example: (i) selectively produced by expression of a recombinant nucleic acid or (ii) purified as by chromatography or electrophoresis. Isolated proteins or polypeptides may, but need not be, substantially pure. The term "substantially pure" means that the proteins or polypeptides are essentially free of other substances with which they may be found in nature or *in vivo* systems to an extent practical and appropriate for their intended use. Substantially pure proteins may be produced by techniques well known in the art. Because an isolated protein may be admixed with a pharmaceutically acceptable carrier in a pharmaceutical preparation, the protein may comprise only a small percentage by weight of the preparation. The protein is nonetheless isolated in that it has been separated from the substances with which it may be associated in living systems, e.g. isolated from other proteins.

The disclosure also encompasses unique fragments of the proteins or peptides described herein. A fragment of any one of the proteins or peptides described herein, for example, generally has the features and characteristics of fragments including unique fragments as discussed herein in connection with nucleic acid molecules. As will be recognized by those skilled in the art, the size of a fragment which is unique will depend upon factors such as whether the fragment constitutes a portion of a conserved protein domain. Thus, some regions of the proteins or peptides will require longer segments to be unique while others will require only short segments, typically between 5 and 12 amino acids (e.g. 5, 6, 7, 8, 9, 10, 11, and 12 amino acids long).

Unique fragments of a protein preferably are those fragments which retain a distinct functional capability of the protein. Functional capabilities which can be retained in a fragment of a protein include interaction with antibodies, interaction with other proteins or fragments thereof, selective binding of nucleic acid molecules, and enzymatic activity. One important activity is the ability to act as a signature for identifying the polypeptide.

Those skilled in the art are well versed in methods for selecting unique amino acid sequences, typically on the basis of the ability of the fragment to selectively distinguish the sequence of interest from non-family members. A comparison of the sequence of the fragment to those on known data bases typically is all that is necessary.

This disclosure embraces variants of the proteins or peptides described herein. As used herein, a "variant" of a protein is a protein which contains one or more modifications to the primary amino acid sequence of such protein. Modifications which create a protein variant can be made to such protein 1) to produce, increase, reduce, or eliminate-an activity of the protein; 2) to enhance a property of the protein, such as protein stability in an expression system or the stability of protein-protein binding; 3) to provide a novel activity or property to a protein, such as addition of an antigenic epitope or addition of a detectable moiety; and/or 4) to provide equivalent or better binding to a ligand molecule. Modifications to a protein can be made via modifications to the nucleic acid molecule which encodes the protein, and can include deletions, point mutations, truncations, amino acid substitutions and additions of amino acids or non-amino acid moieties. Alternatively, modifications can be made directly to the protein, such as by cleavage, substitution of one or more amino acids during chemical systhesis, addition of a linker molecule, addition of a detectable moiety, such as biotin, addition of a fatty acid, etc. Modifications also embrace fusion proteins comprising all or part of an amino acid sequence as described herein. One of skill in the art will be familiar with methods for predicting the effect on protein conformation of a change in amino acid sequence, and can thus "design" a variant polypeptide according to known methods. One example of such a method is described by Dahiyat and Mayo in Science 278:82-87, 1997, whereby proteins can be designed *de novo.* The method can be applied to a known protein to vary only a portion of the protein sequence. By applying the computational methods of Dahiyat and Mayo, specific variants of a DOS protein can be proposed and tested to determine whether the variant retains a desired conformation.

In certain cases, variants include proteins which are modified specifically to alter a feature of the protein unrelated to its desired physiological activity. For example, cysteine residues can be substituted or deleted to prevent unwanted disulfide linkages. Similarly, certain amino acids can be changed to enhance expression of a protein by eliminating proteolysis by proteases in an expression system (e.g., dibasic amino acid residues in yeast expression systems in which KEX2 protease activity is present).

Mutations of a nucleic acid molecule which encode a protein or peptide, as described herein, preferably preserve the amino acid reading frame of the coding sequence, and preferably do not create regions in the nucleic acid which are likely to hybridize to form secondary structures, such a hairpins or loops, which can be deleterious to expression of the variant protein.

Mutations can be made by selecting an amino acid substitution, or by random mutagenesis of a selected site in a nucleic acid which encodes the protein. Variant proteins are then expressed and tested for one or more activities to determine which mutation provides a variant protein with the desired properties. Further mutations can be made to variants (or to the non-variant proteins) which are silent as to the amino acid sequence of the protein, but which provide preferred codons for translation in a particular host, as well known to those of ordinary skill in the art. Still other mutations can be made to the noncoding sequences of a gene expressing the protein or cDNA clone to enhance expression of the protein. The activity of variants of particular proteins can be tested by cloning the gene encoding the variant protein into a bacterial or mammalian expression vector, introducing the vector into an appropriate host cell, expressing the variant protein, and testing for a functional capability of the protein, for example its ability to bind to or interact with a particular ligand or its ability to act as ligand to a particular biomolecule, such as a receptor. Preparation of other variant proteins may favor testing of other activities, as will be known to one of ordinary skill in the art.

The skilled artisan will also realize that certain amino acid substitutions, such as for example conservative amino acid substitutions, may be made in the provided proteins or peptides to provide "functional variants" of the foregoing proteins or peptides, i.e, variants which possess functional capabilities of the corresponding provided proteins or peptides. As used herein, a "conservative amino acid substitution" refers to an amino acid substitution which does not alter the relative charge or size characteristics of the protein in which the amino acid substitution is made. Conservative substitutions of amino acids include substitutions made amongst amino acids within the following groups: (a) M, I, L, V; (b) F, Y, W; (c) K, R, H; (d) A, G; (e) S, T; (f) Q, N; and (g) E, D.

For example, in one case, one can make amino acid substitutions, e.g. conservative amino acid substitutions, to the amino acid sequence of a protein or peptide as described herein. The substituted peptides can then be tested for one or more of the desired functions of the non-substituted peptide, *in vivo* and/or *in vitro.* These variants can be tested for, for example, improved stability or other desirable properties and, which could, for example, render them more useful, *inter alia*, in pharmaceutical compositions.

Functional variants of the provided proteins or peptides, i.e., variants of proteins or peptides which retain functionality of the original proteins or peptides, can be prepared according to methods for altering polypeptide sequence known to one of ordinary skill in the art such as are found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Conservative amino-acid substitutions typically are made by alteration of the nucleic acid molecule encoding a protein or peptide. Such substitutions can be made by a variety of methods known to one of ordinary skill in the art. For example, amino acid substitutions may be made by PCR-directed mutation, site-directed mutagenesis according to the method of Kunkel (Kunkel, Proc. Nat. Acad. Sci. U.S.A. 82: 488-492, 1985), or by chemical synthesis of a gene encoding a protein. Where amino acid substitutions are made to a small unique fragment of a protein or peptide as described herein, the substitutions can be made by directly synthesizing the peptide. The activity of functional variants or fragments of the provided protein or peptides can be tested by cloning the gene encoding the altered protein into a bacterial or mammalian expression vector, introducing the vector into an appropriate host cell, expressing the altered protein, and testing for a functional capability of the proteins as disclosed herein.
The foregoing methods can be performed, e.g. by sequential repetition, to yield functional variants having up to 1, 2, 3, 4, 5, 6, 7, 8, 9, 10, 11, 12, 13, 14, 15, 16, 17, 18, 19, 20, or more amino acid substitutions. Similarly, the above or other functional variants can be prepared having, or also having, up to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more amino acid additions or deletions at their C- and/or N-terminus. Variants of the proteins or peptides prepared by the foregoing methods can be sequenced, if desired, to determine the amino acid sequence and thus deduce the nucleotide sequence which encodes such variants. The present disclosure in another aspect provides nucleic acid sequences encoding a variety of truncated MUC1 receptor proteins, or functional variants or fragments thereof, and other nucleic acid sequences that hybridize to the above nucleic acid sequences under high stringency conditions. The sequence of certain of the nucleic acid molecules are presented in Table 2 below as SEQ ID NOs : 42-46, and the predicted amino acid sequences of these genes' protein products, each comprising an isoform of a truncated MUC1 receptor protein, are presented in Table 1 below. Thus peptide sequences are provided representing truncated isoforms of the MUC1 receptor, genes encoding those peptide sequences and functional modifications and variants of the foregoing, useful fragments of the foregoing, as well as therapeutic and diagnostic products and methods relating thereto. The peptides referred to herein as truncated MUC1 receptor proteins include fragments of the full length MUC1 receptor but do not include the full length MUC1 receptor protein (i.e. SEQ ID NO: 10). Likewise, nucleic acid molecules that encode the various truncated isoforms of the MUC1 receptor described herein can include fragments of the MUC1 gene coding region, but do not include the full length MUC1 coding region.

According to one instance, an isolated nucleic acid molecule is provided. The isolated nucleic acid molecule is selected from the group consisting of:
(a) nucleic acid molecules which encode the MUC1 truncated receptor isoform peptides listed as SEQ ID NOs. 37, 38, 39, 40, and 41 in Table 1), or functional variants or fragments thereof, including, for example, the nucleotide sequences: SEQ ID NOs: 42, 43, 44, 45, and 46, respectively, and
(b) nucleic acid molecules which hybridize under highly stringent conditions to the nucleic acid molecules of (a),
(c) deletions, additions and substitutions of the nucleic acid molecules of (a) or (b),
(d) nucleic acid molecules that differ from the nucleic acid molecules of (a), (b) or (c) in codon sequence due to the degeneracy of the genetic code, and
(e) complements of (a), (b), (c), or (d).

Certain isolated nucleic acids described herein are nucleic acid molecules which encode a truncated isoform of the MUC1 receptor, or a functional fragment or varient thereof, or a functional equivalent thereof (e.g., a nucleic acid sequence encoding the same protein as encoded by one of the nucleic acid sequences, e.g. SEQ ID NO. 42, listed below in Table 2), provided that the functional fragment or equivalent encodes a protein which exhibits the functional activity of a truncated isoform of the MUC1 receptor encoded by such a listed sequence. As used herein, the functional activity of the truncated isoforms of the MUC1 receptor refers to the ability of the truncated isoforms of the MUC1 receptor peptide sequence to specifically interact with ligands for MGFR and to modulate cell growth or cell proliferation in response to such interaction. In certain cases, the isolated nucleic acid molecule is SEQ ID NO: 42.

Provided herein are nucleic acid molecules which hybridize under high stringency conditions to a nucleic acid molecule consisting of the nucleotide sequences set forth in SEQ ID NOs: 42-46. Such nucleic acids may be DNA, RNA, composed of mixed deoxyribonucleotides and ribonucleotides, or may also incorporate synthetic non-natural nucleotides. Various methods for determining the expression of a nucleic acid and/or a polypeptide in normal and tumor cells are known to those of skill in the art.

The term "highly stringent conditions" or "high stringency conditions"as used herein refers to parameters with which those skilled in the art are familiar. Nucleic acid hybridization parameters may be found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. More specifically, stringent conditions, as used herein, refers, for example, to hybridization at 65°C in hybridization buffer (3.5 x SSC, 0.02% Ficoll, 0.02% polyvinyl pyrrolidone, 0.02% Bovine Serum Albumin, 2.5mM NaH2PO4 (pH 7), 0.5% SDS, 2mM EDTA). SSC is 0.15M sodium chloride/0.15M sodium citrate, pH 7; SDS is sodium dodecyl sulphate; and EDTA is ethylenediaminetetracetic acid. After hybridization, the membrane upon which the DNA is transferred is washed at 2 x SSC at room temperature and then at 0.1 x SSC/0.1 x SDS at temperatures up to 68°C.

The foregoing set of hybridization conditions is but one example of highly stringent hybridization conditions known to one of ordinary skill in the art. There are other conditions, reagents, and so forth which can be used, which result in a highly stringent hybridization. The skilled artisan will be familiar with such conditions, and thus they are not given here. It will be understood, however, that the skilled artisan will be able to manipulate the conditions in a manner to permit the clear identification of homologs and alleles of the nucleic acid molecules described herein. The skilled artisan also is familiar with the methodology for screening cells and libraries for expression of such molecules which then are routinely isolated, followed by isolation of the pertinent nucleic acid molecule and sequencing.

In general homologs and alleles of a specific SEQ ID NO. enumerated herein (see Table 2) typically will share at least 40% nucleotide identity and/or at least 50% amino acid identity to such a nucleotide sequence or amino acid sequence, respectively, in some instances will share at least 50% nucleotide identity and/or at least 65% amino acid identity and in still other instances will share at least 60% nucleotide identity and/or at least 75% amino acid identity. Preferred homologs and alleles share nucleotide and amino acid identities with SEQ ID NO: 42 and SEQ ID NO: 37, respectively; or SEQ ID NO: 43 and SEQ ID NO: 38, respectively; or SEQ ID NO: 44 and SEQ ID NO: 39, respectively; or SEQ ID NO: 45 and SEQ ID NO: 40, respectively; or SEQ ID NO: 46 and SEQ ID NO: 41, respectively; and encode polypeptides of greater than 80%, more preferably greater than 90%, still more preferably greater than 95% and most preferably greater than 99% identity. The percent identity can be calculated using various, publicly available software tools developed by NCBI (Bethesda, Maryland) that can be obtained through the internet (ftp:/ncbi.nlm.nih.gov/pub/). Exemplary tools include the BLAST system available at http://www.ncbi.nlm.nih.gov, which uses algorithms developed by Altschul et al. (Nucleic Acids Res. 25:3389-3402, 1997). Pairwise and ClustalW alignments (BLOSUM30 matrix setting) as well as Kyte-Doolittle hydropathic analysis can be obtained using the MacVector sequence analysis software (Oxford Molecular Group). Watson-Crick complements of the foregoing nucleic acid molecules also are embraced by the present disclosure.

Also provided herein are degenerate nucleic acid molecules which include alternative codons to those present in the native materials. For example, serine residues are encoded by the codons TCA, AGT, TCC, TCG, TCT and AGC. Each of the six codons is equivalent for the purposes of encoding a serine residue. Thus, it will be apparent to one of ordinary skill in the art that any of the serine-encoding nucleotide triplets may be employed to direct the protein synthesis apparatus, *in vitro* or *in vivo*, to incorporate a serine residue into an elongating peptide sequence. Similarly, nucleotide sequence triplets which encode other amino acid residues include, but are not limited to: CCA, CCC, CCG and CCT (proline codons); CGA, CGC, CGG, CGT, AGA and AGG (arginine codons); ACA, ACC, ACG and ACT (threonine codons); AAC and AAT (asparagine codons); and ATA, ATC and ATT (isoleucine codons). Other amino acid residues may be encoded similarly by multiple nucleotide sequences. Thus, the present disclosure embraces degenerate nucleic acids that differ from the biologically isolated nucleic acids in codon sequence due to the degeneracy of the genetic code.

Also provided herein are isolated unique fragments of SEQ ID NOs: 42-46 and/or complements of SEQ ID NOs: 42-46. A unique fragment is one that is a 'signature' for the larger nucleic acid. It, for example, is long enough to assure that its precise sequence is not found in molecules outside of the nucleic acid molecules defined above. Those of ordinary skill in the art may apply no more than routine procedures to determine if a fragment is unique within the human or mouse genome.

Unique fragments can be used as probes in Southern blot assays to identify such nucleic acid molecules, or can be used in amplification assays such as those employing PCR. Unique fragments also can be used to produce fusion proteins for generating antibodies or determining binding of the polypeptide fragments, or for generating immunoassay components. Likewise, unique fragments can be employed to produce nonfused fragments of certain polypeptides as described herein useful, for example, in the preparation of antibodies, in immunoassays. Unique fragments further can be used as antisense oligonucleotides to inhibit the expression of nucleic acids and polypeptides, particularly for therapeutic purposes. Also encompassed by the present disclosure are antisense oligonucleotides of the above-described nucleic acid molecules.

Generally, as used herein, the term "antisense oligonucleotide" or "antisense" describes an oligonucleotide that is an oligoribonucleotide, oligodeoxyribonucleotide, modified oligoribonucleotide, or modified oligodeoxyribonucleotide which hybridizes under physiological conditions to DNA comprising a particular gene or to an mRNA transcript of that gene and, thereby, inhibits the transcription of that gene and/or the translation of that mRNA. Those skilled in the art will recognize that the exact length of the antisense oligonucleotide and its degree of complementarity with its target will depend upon the specific target selected, including the sequence of the target and the particular bases which comprise that sequence. It is preferred that the antisense oligonucleotide be constructed and arranged so as to bind selectively with the target under physiological conditions, i.e., to hybridize substantially more to the target sequence than to any other sequence in the target cell under physiological conditions. One of skill in the art can easily choose and synthesize any of a number of appropriate antisense molecules for use in accordance with the present teachings. In order to be sufficiently selective and potent for inhibition, such antisense oligonucleotides should comprise at least 10 and, more preferably, at least 15 consecutive bases which are complementary to the target, although in certain cases modified oligonucleotides as short as 7 bases in length have been used successfully as antisense oligonucleotides (Wagner et al., Nature Biotechnology 14: 840-844, 1996). In certain cases, the antisense oligonucleotides as described hereinmay include "modified" oligonucleotides. That is, the oligonucleotides may be modified in a number of ways known in the art, which do not prevent them from hybridizing to their target but which enhance their stability or targeting, or which otherwise enhance their therapeutic effectiveness. Antisense oligonucleotides may be administered as part of a pharmaceutical composition. Such a pharmaceutical composition may include the antisense oligonucleotides in combination with any standard physiologically and/or pharmaceutically acceptable carriers which are known in the art. The compositions should be sterile and contain a therapeutically effective amount of the antisense oligonucleotides in a unit of weight or volume suitable for administration to a patient.

As will be recognized by those skilled in the art, the size of the above-mentioned unique fragment will depend upon its conservancy in the genetic code. Thus, some regions of SEQ ID NOs : 42-46 and their complements will require longer segments to be unique while others will require only short segments, typically between 12 and 32 nucleotides or more in length (e.g. 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63 ,64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115 or more), up to the entire length of the disclosed sequence. Many segments of the polynucleotide coding region or complements thereof that are 18 or more nucleotides in length will be unique. Those skilled in the art are well versed in methods for selecting such sequences, typically on the basis of the ability of the unique fragment to selectively distinguish the sequence of interest from other, unrelated nucleic acid molecules. A comparison of the sequence of the fragment to those on known data bases typically is all that is necessary, although *in vitro* confirmatory hybridization and sequencing analysis may be performed.

A unique fragment can be a functional fragment. A functional fragment of a nucleic acid molecule as decribed hereinis a fragment which retains some functional property of the larger nucleic acid molecule, such as coding for a functional polypeptide, binding to proteins, regulating transcription of operably linked nucleic acid molecules, and the like. One of ordinary skill in the art can readily determine using the assays described herein and those well known in the art to determine whether a fragment is a functional fragment of a nucleic acid molecule using no more than routine experimentation.

As used herein with respect to nucleic acid molecules, the term "isolated" means: (i) amplified *in vitro* by, for example, polymerase chain reaction (PCR); (ii) recombinantly produced by cloning; (iii) purified, as by cleavage and gel separation; or (iv) synthesized by, for example, chemical synthesis. An isolated nucleic acid molecule is one which is readily manipulable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid molecule may be substantially purified, but need not be. For example, a nucleic acid molecule that is isolated within a cloning or expression vector is not pure in that it may comprise only a tiny percentage of the material in the cell in which it resides. Such a nucleic acid molecule is isolated, however, as the term is used herein because it is readily manipulable by standard techniques known to those of ordinary skill in the art. An isolated nucleic acid molecule as used herein is not a naturally occurring chromosome.

Yet another instance of the present description embraces the use of sequences, such as those discussed immediately above, that encode a peptide or fragment or variant thereof, in expression vectors, to transfect host cells and cell lines, be these prokaryotic (e.g., *E. coli*), or eukaryotic (e.g., CHO cells, COS cells, yeast expression systems and recombinant baculovirus expression in insect cells). Especially useful are mammalian cells such as human, mouse, hamster, pig, goat, primate, etc. They may be of a wide variety of tissue types, and they may be primary cells or cell lines. The expression vectors include the pertinent sequence, i.e., those provided nucleic acids encoding the peptide sequences , described above, operably linked to a promoter.

In certain cases, expression vectors comprising any of the isolated nucleic acid molecules provided herein, preferably operably linked to a promoter, are provided. In a related aspect, host cells transformed or transfected with such expression vectors also are provided. Expression vectors containing all the necessary elements for expression are commercially available and known to those skilled in the art. See, e.g., Sambrook et al., Molecular Cloning: A Laboratory Manual, Second Edition, Cold Spring Harbor Laboratory Press, 1989. Cells are genetically engineered by the introduction into the cells of heterologous DNA (RNA) encoding a protein of the present disclosure, fragment, or variant thereof. The heterologous DNA (RNA) is placed under operable control of transcriptional elements to permit the expression of the heterologous DNA in the host cell.

As used herein, a "vector" may be any of a number of nucleic acid molecules into which a desired sequence may be inserted by restriction and ligation for transport between different genetic environments or for expression in a host cell. Vectors are typically composed of DNA although RNA vectors are also available. Vectors include, but are not limited to, plasmids, phagemids and virus genomes. A cloning vector is one which is able to replicate in a host cell, and which is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium or just a single time per host before the host reproduces by mitosis. In the case of phage, replication may occur actively during a lytic phase or passively during a lysogenic phase.

An "expression vector" is one into which a desired DNA sequence may be inserted by restriction and ligation such that it is operably joined to regulatory sequences and may be expressed as an RNA transcript. Vectors may further contain one or more marker sequences suitable for use in the identification of cells that have or have not been transformed or transfected with the vector. Markers include, for example, genes encoding proteins that increase or decrease either resistance or sensitivity to antibiotics or other compounds, genes that encode enzymes whose activities are detectable by standard assays known in the art (e.g., β-galactosidase or alkaline phosphatase), and genes that visibly affect the phenotype of transformed or transfected cells, hosts, colonies or plaques (e.g., green fluorescent protein). Preferred vectors are those capable of autonomous replication and expression of the structural gene products present in the DNA segments to which they are operably joined.

As used herein, a coding sequence and regulatory sequences are said to be "operably" joined when they are covalently linked in such a way as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript might be translated into the desired protein or polypeptide.

The precise nature of the regulatory sequences needed for gene expression may vary between species or cell types, but shall in general include, when necessary, 5' non-transcribed and 5' non-translated sequences involved with the initiation of transcription and translation respectively, such as a TATA box, capping sequence, CAAT sequence, and the like. Especially, such 5' non-transcribed regulatory sequences will include a promoter region that includes a promoter sequence for transcriptional control of the operably joined gene. Regulatory sequences may also include enhancer sequences or upstream activator sequences as desired. The vectors of the present disclosure may optionally include 5' leader or signal sequences. The choice and design of an appropriate vector is within the ability and discretion of one of ordinary skill in the art.

Possible regulatory elements permitting expression in prokaryotic host cells comprise, e.g., the PL, lac, trp or tac promoter in E. coli, and examples of regulatory elements permitting expression in eukaryotic host cells are the AOX1 or GAL1 promoter in yeast or the CMV-promoter, SV40-promoter, RSV-promoter (Rous sarcoma virus), CMV-enhancer, SV40-enhancer or a globin intron in mammalian and other animal cells.

Beside elements which are responsible for the initiation of transcription such regulatory elements may also comprise transcription termination signals, such as the SV40-poly-A site or the tk-poly-A site, downstream of the polynucleotide. Furthermore, depending on the expression system used, leader sequences capable of directing the polypeptide to a cellular compartment, e.g. the cell cytoplasmic membrane, or secreting it into the medium may be added to the coding sequence of the polynucleotides of the present disclosure and are well known in the art. The leader sequence(s) is (are) assembled in appropriate phase with translation, initiation and termination sequences, and in certain cases, a leader sequence capable of directing the polypeptide to a cellular compartment or directing secretion of translated protein, or a portion thereof, into the periplasmic space or extracellular medium. In this context, suitable expression vectors are known in the art such as Okayama-Berg cDNA expression vector pcDV1 (Pharmacia), pCDM8, pRc/CMV, pcDNA1 pcDNA3 (Invitrogen), or pSPORT1 (GIBCO BRL).

Also provided are host cells transformed with a polynucleotide or vector as described herein. Such host cell may be a prokaryotic or eukaryotic cell. The polynucleotide or vector which is present in the host cell may either be integrated into the genome of the host cell or it may be maintained extrachromosomally. The host cell can be any prokaryotic or eukaryotic cell, such as a bacterial, insect, fungal, plant, animal or human cell. Certain fungal cells are, for example, those of the genus Saccharomyces, in particular those of the species S. cerevisiae. The term "prokaryotic" is meant to include all bacteria which can be transformed or transfected with DNA or RNA molecules for the expression of a peptide sequence as described herein. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells, for example NSO and CHO cells. Depending upon the host employed in a recombinant production procedure, the peptide sequences encoded by the polynucleotides of the present disclosure may be glycosylated or may be non-glycosylated. A polynucleotide of the present disclosure can be used to transform or transfect the host using any of the techniques commonly known to those of ordinary skill in the art. Furthermore, methods for preparing fused, operably linked genes and expressing them in, e.g., mammalian cells and bacteria are well-known in the art (Sambrook, Molecular Cloning: A Laboratory Manual, Cold Spring Harbor Laboratory, Cold Spring Harbor, NY, 1989). The genetic constructs and methods described therein, or straightforward modifications thereof, can be utilized for expression of the polypeptides described herein in eukaryotic or prokaryotic hosts. In certain cases, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. Suitable source cells for the DNA sequences and host cells for polypeptide expression can be obtained from a number of sources, such as the American Type Culture Collection ("Catalogue of Cell Lines and Hybridomas," Fifth edition (1985) Rockville, Maryland, U.S.A.).

In some instances, a virus vector for delivering a nucleic acid molecule encoding a peptide sequence as described herein is selected from the group consisting of adenoviruses, adeno-associated viruses, poxviruses including vaccinia viruses and attenuated poxviruses, Semliki Forest virus, Venezuelan equine encephalitis virus,
retroviruses, Sindbis virus, and Ty virus-like particle. Examples of viruses and virus-like particles which have been used to deliver exogenous nucleic acids include: replication-defective adenoviruses (e.g., Xiang et al., Virology 219:220-227, 1996; Eloit et al., J. Virol. 7:5375-5381, 1997; Chengalvala et al., Vaccine 15:335-339, 1997), a modified retrovirus (Townsend et al., J. Virol. 71:3365-3374, 1997), a nonreplicating retrovirus (Irwin et al., J. Virol. 68:5036-5044, 1994), a replication defective Semliki Forest virus (Zhao et al., Proc. Natl. Acad. Sci. USA 92:3009-3013, 1995), canarypox virus and highly attenuated vaccinia virus derivative (Paoletti, Proc. Natl. Acad. Sci. USA 93:11349-11353, 1996), non-replicative vaccinia virus (Moss, Proc. Natl. Acad. Sci. USA 93:11341-11348, 1996), replicative vaccinia virus (Moss, Dev. Biol. Stand. 82:55-63, 1994), Venzuelan equine encephalitis virus (Davis et al., J. Virol. 70:3781-3787, 1996), Sindbis virus (Pugachev et al., Virology 212:587-594, 1995), and Ty virus-like particle (Allsopp et al., Eur. J. Immunol 26:1951-1959, 1996). In certain instances, the virus vector is an adenovirus.

Another virus, which can potentially be used for certain applications, is the adeno-associated virus, a double-stranded DNA virus. The adeno-associated virus is capable of infecting a wide range of cell types and species and can be engineered to be replication-deficient. It further has advantages, such as heat and lipid solvent stability, high transduction frequencies in cells of diverse lineages, including hematopoietic cells, and lack of superinfection inhibition thus allowing multiple series of transductions. The adeno-associated virus can integrate into human cellular DNA in a site-specific manner, thereby minimizing the possibility of insertional mutagenesis and variability of inserted gene expression. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Adenoviruses and retroviruses have been approved for human gene therapy trials. In general, the retroviruses are replication-deficient (i.e., capable of directing synthesis of the desired proteins, but incapable of manufacturing an infectious particle). Such genetically altered retroviral expression vectors can have general utility for the high-efficiency transduction of genes *in vivo.* Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, M., Gene Transfer and Expression, A Laboratory Manual, W.H. Freeman Co., New York (1990) and Murry, E.J. Ed. "Methods in Molecular Biology," vol. 7, Humana Press, Inc., Cliffton, New Jersey (1991).

In certain instances, the foregoing nucleic acid delivery vectors: (1) contain exogenous genetic material that can be transcribed and translated in a mammalian cell and that can produce a peptide sequence as disclosed herein that is localized within, and oriented with respect to, the cytoplasmic membrane of the cell, such that an extracellular receptor portion of the peptide sequence (e.g. PSMGFR) is expressed on the external surface of the cell cytoplasmic membrane, and (2) contain on a surface a ligand that selectively binds to a receptor on the surface of a target cell, such as a mammalian cell, and thereby gains entry to the target cell.

Various techniques may be employed for introducing nucleic acid molecules as disclosed herein into cells, depending on whether the nucleic acid molecules are introduced *in vitro* or *in vivo* in a host. Such techniques include transfection of nucleic acid molecule-calcium phosphate precipitates, transfection of nucleic acid molecules associated with DEAE, transfection or infection with the foregoing viruses including the nucleic acid molecule of interest, liposome-mediated transfection, and the like.

For certain uses, it is preferred to target the nucleic acid molecule to particular cells. In such instances, a vehicle used for delivering a nucleic acid molecule as disclosed hereininto a cell (e.g., a retrovirus, or other virus; a liposome) can have a targeting molecule attached thereto. For example, a molecule such as an antibody specific for a surface membrane protein on the target cell or a ligand for a receptor on the target cell can be bound to or incorporated within the nucleic acid molecule delivery vehicle. Especially preferred are monoclonal antibodies. Where liposomes are employed to deliver the nucleic acid molecules , proteins that bind to a surface membrane protein associated with endocytosis may be incorporated into the liposome formulation for targeting and/or to facilitate uptake. Such proteins include capsid proteins or fragments thereof tropic for a
particular cell type, antibodies for proteins which undergo internalization in cycling, proteins that target intracellular localization and enhance intracellular half life, and the like. Polymeric delivery systems also have been used successfully to deliver nucleic acid molecules into cells, as is known by those skilled in the art. Such systems even permit oral delivery of nucleic acid molecules.

In addition to delivery through the use of vectors, nucleic acids as described hererin may be delivered to cells without vectors, e.g. as "naked" nucleic acid delivery using methods known to those of skill in the art.

A transgenic non-human animal comprising an expression vector as described herein is provided. As used herein, "transgenic non-human animals" includes non-human animals having one or more exogenous nucleic acid molecules incorporated in germ line cells and/or somatic cells. Thus the transgenic animal include animals having episomal or chromosomally incorporated expression vectors, etc. In general, such expression vectors can use a variety of promoters which confer the desired gene expression pattern (e.g., temporal or spatial). Conditional promoters also can be operably linked to nucleic acid molecules to increase or decrease expression of the encoded polypeptide molecule in a regulated or conditional manner. *Trans-*acting negative or positive regulators of polypeptide activity or expression also can be operably linked to a conditional promoter as described above. Such *trans-*acting regulators include antisense nucleic acid molecules, nucleic acid molecules that encode dominant negative molecules, transcription factors, ribozyme molecules specific for nucleic acid molecules, and the like. The transgenic non-human animals are useful in experiments directed toward testing biochemical or physiological effects of diagnostics or therapeutics, for example for cancers characterized by aberrant expression of MUC1. Other uses will be apparent to one of ordinary skill in the art.

Also provided are so-called expression kits, which allow the artisan to prepare a desired expression vector or vectors. Such expression kits include at least one of the previously discussed nucleotide sequences encoding a peptide sequence as previously discussed. Other components may be added, as desired, as long as the above-mentioned sequences are included.

The results presented herein, within the context of the present disclosure demonstrate that MUC1 transfectants (i.e. cells transfected with nucleic acid molecules encoding isoforms of the MUC1 receptor on the cell surface) behave similarly to native MUC1+ breast tumor cells.

The results presented within the context of the present description also suggest that the PSMGFR is the functionally necessary and sufficient portion of the MUC1 receptor that mediates cell growth. Evidence presented herein suggests that the MUC1 receptor, which is aberrantly expressed in about 75% of all human solid tumors, is a key receptor that mediates the growth of tumor cells. Results presented herein and discussed above and in the examples provide evidence that a portion of the MUC1 receptor, which remains attached to the cell surface after cleavage, is the part of the MUC1 extracellular domain that is sufficient and necessary for MUC1-dependent cell proliferation. As shown and discussed previously, dimerization of the MGFR portion of the MUC1 receptor induced cell proliferation. Although the exact site of receptor cleavage has not yet been determined, the present disclosure presents experimental results that suggest that the necessary and sufficient portion of the MUC1 receptor that is required to stimulate cell proliferation is the portion of the receptor that includes essentially all of the native PSMGFR (nat-PSMGFR SEQ ID NO: 36)

MUC1⁺ breast tumor cells, T47D, 1500, 1504, and BT-474 were obtained from the ATCC (See Example 5). As controls, MUC1⁻ breast tumor cells MDA-MB-453, HEK (human embilical kidney) cell line K293, and HeLa cells were also obtained from the ATCC (See Example 5). Western blot analysis was performed (See Example 5) to determine the expressed levels of MUC1 in each cell type. High percentage (15%) as well as low percentage (6%) polyacrylimide gels were run in order to visualize uncleaved as well as cleaved MUC1 receptor. Lower percentage gels, able to visualize proteins of molecular weights ranging from 50 kDa to 350 kDa, were probed with an antibody, VU4H5 (Santa Cruz Biotechnology, Santa Cruz, CA) that recognized the ADDTR sequence that is present in the terminal repeat portion of the extracellular domain. Higher percentage gels that were used to visualize proteins between 6.5 - 50 kDa were probed with a the rabbit polyclonal antibody that targets the PSMGFR, described above. Fig. 36 , the method for producing which is described in Ex. 5, is a western blot that shows that cell line 1504 expressed the highest levels of uncleaved MUC1, followed by T47D, then 1500 cells, with BT-474, K293, and HeLa cells showing no detectable amount of uncleaved MUC1. Fig. 30, the method for producing which is described in Ex. 5, is another western blot that shows that three breast tumor cell lines 1504, 1500 and T47D all expressed similar quantities of a proteolyzed MUC1 that ran with an apparent molecular weight of 20 - 30 kDa. It is noted that Wreschner et. al published data that showed that this MUC1 cleavage product is not expressed in normal, healthy breast tissue. BT-474 expressed a considerably lower level of cleaved MUC1. In addition, the protein bands of BT-474 are concentrated at 20 kDa with a low intensity presence at around 15 kDa (See Fig. 30). K293 cells showed no MUC1 expression as expected and HeLa cells showed minimal MUC1 between 20 - 30 kDa as previously reported in the literature. MDA-MB-453 also did not express detectable levels of cleaved or uncleaved MUC1(data not shown).

Cellular proteins often undergo post-translational modifications such as cleavage, glycosylation and phosphorylation. In particular, it is known that, under some circumstances, the extracellular domain of the MUC1 receptor is cleaved (at an unknown location) and shed into the bloodstream. The extracellular portion of the receptor can also be glycosylated, although it has been reported that in tumor cells, it is often under-glycosylated. The fact that the MUC 1 receptor undergoes these indeterminate modifications makes it difficult to characterize the portion of the receptor that remains on the cell surface after cleavage in terms of length. Note that the degree of glycosylation alters the molecular weight of the receptor when analyzed by western blot, for example. Therefore, in order to compare expression levels of MUC1 among various cells types and to get a better determination of their true molecular weights, it is advantageous to deglycosylate protein samples prior to western gel analysis. Fig. 37, the method for producing which is described in Ex. 5, shows that after deglycosylation, the MUC1 protein bands converged to form a prominent band with an apparent molecular weight of about 20 kDa. These results suggest that all the breast tumor cell lines tested produced MUC 1 cleavage products of approximately the same length but that had differential glycosylation. Note that non-deglycosylated 1504, 1500 and T47D samples showed three clear MUC1 protein bands. The PSMGFR sequence contains three Arginine residues that can be glycosylated. Further analysis, refer to see Fig. 37 (lanes 3 versus 4), suggested that the MUC1 proteolysis product was in fact N- and not O-glycosylated.

To determine which amino acids were being glycosylated, the 1500 cell line was deglycosylated using enzymes that specifically remove O- or N-lined glycol units. Fig. 37 shows that the shift in molecular weight only occurs after treatment with the N-specific deglycosylase. This suggests that the PSMGFR region of the MUC1 receptor is only N-glycosylated.

In another set of cases, MUC1 isoform constructs of varying length were transfected into HEK cells and analyzed by western blot using anti-PSMGFR to determine cleavage patterns of the various MUC1 constructs. To investigate which portion of the extracellular domain of the MUC1 receptor is necessary and sufficient for or involved in the growth factor-like function described herein and to investigate the sequence of the portion of the receptor that remains on the cell surface after cleavage in tumor cells, HEK (human embryonic kidney) cells were transfected with plasmids designed to generate MUC1 receptor variants of different lengths. Fig. 38 is a cartoon that depicts the MUC1 constructs that were generated, see Exs. 6-7. In summary, constructs were generated to produce the entire MUC1 receptor (SEQ ID NO: 10 - Table 1), a MUC1 receptor variant with only 1.3 kilobases of the repeats section ("Rep isoform" - SEQ ID NO: 41 - Table 1), a MUC1 receptor variant that terminates after the IBR (interchain binding domain)("CM isoform" - SEQ ID NO: 38 - Table 1), a MUC1 receptor variant that terminates after the PSMGFR ("nat-PSMGFRTC isoform" - SEQ ID NO: 37 - Table 1), and the entire MUC1-Y alternative splice variant ("Y isoform" - SEQ ID NO: 40 - Table 1). Fig. 39 shows that apparently all of the MUC1 variants undergo cleavage, except the nat-PSMGFRTC isoform and the CM isoform constructs. There is evidence in the literature that sequences C-terminal to the end of the IBR are required for enzyme cleavage of the receptor.

It should be noted that it was observed that the cells transfected with the nat-PSMGFRTC isoform grew faster than the parent cell line and considerably faster (approximately 2-times) than the full length or repeats (Rep isoform) constructs. Further, the inventors have observed that HEK cells transfected with the nat-PSMGFRTC isoform are capable of anchorage-independent cell growth. Note that anchorage-independent cell growth is a phenomenon that is not yet understood but is a hallmark characteristic of true tumor cells. It was also observed that attempts to transiently transfect cells with the full length or repeats (Rep isoform) constructs were difficult and frequently failed. In sharp contrast, the nat-PSMGFRTC isoform construct transfected easily each and every time it was tried. These results support the premise that it is the PSMGFR portion of the receptor that acts as a growth factor receptor.

To determine whether in HEK cells the MUC1 truncated receptors would behave as they do in tumor cells, a series of cell growth stimulation and inhibition were performed. In particular, bivalent antibodies and monovalent antibody binding fragments were added to these cells to determine their effect on cell growth and the phosphorylation state of ERK2. Fig. 40 shows that the addition of bivalent anti-PSMGFR (solid red bars) to the nat-PSMGFRTC isoform construct transfected cells caused an 80% enhancement of cell proliferation, while the addition of monovalent anti-PSMGFR inhibited native cell growth by 50%. The figure also shows that the monovalent antibody competed with the bivalent antibody to diminish the extent of the enhanced cell growth. Fig. 41 shows that the addition of the bivalent antibody triggers ERK2 phosphorylation while Fig. 42 shows that the monovalent antibody competes with the bivalent to block ERK2 phosphorylation. nat-PSMGFRTC isoform transfected cell lines therefore constitute an excellent research tool for drug discovery for MUC1⁺ cancers, since they behave similarly to tumor cells and this form of the receptor appears to be constitutively active.

To summarize, the extracellular domains that were represented in the transfected cells generated within the context of the present disclosure included: 1) the PSMGFR alone (nat-PSMGFRTC isoform - SEQ ID NO: 37 - Table 1); 2) the PSMGFR and the PSIBR (CM isoform - SEQ ID NO: 38 -Table 1); 3) the PSMGFR, PSIBR and a unique sequence that was ended just before the repeats region (UR isoform - SEQ ID NO: 39 - Table 1); 4) the PSMGFR, PSIBR, unique sequence and 1 kb of repeat sequences (Rep isoform - SEQ ID NO: 41 - Table 1); 5) the entire MUC1 extracellular domain (Full length MUC1 receptor - SEQ ID NO: 10 - Table 1); 6) and the entire extracellular domain of the Y isoform (SEQ ID NO: 40 - Table 1). Cells were grown and treated according to the standard protocols for analyzing the molecular weights of specific proteins by SDS-PAGE followed by western blot (see Example 5). The antibody used in the western blot stage of the analysis specifically recognizes the PSMGFR sequence of the MUC1 receptor. The various transfectants were then analyzed by western blot.

As previously described, the construct expressed constitutively in MUC1+ cancer cell lines, in which the MUC1 receptor is believed to be terminated at or near the N-terminal end of the PSMGFR, produced a series of protein bands between 20 and 30 Kd when glycosylated (See Fig. 37). After deglycosylation, the bands shifted to a molecular weight of about 20 Kd (Fig. 37). Significantly, the calculated molecular weight of the nat-PSMGFRTC isoform transfected construct is 19 Kd.

The MUC1 construct termed "Y isoform" produced a series of protein bands reacted with anti-PSMGFR in a western blot that moved through an SDS-PAGE gel with apparent molecular weights that ranged between 35 - 45 Kd (see Fig. 39). After deglycosylation, the motility of the proteins shifted to apparent molecular weights that ranged from 29 - 40 Kd. The calculated molecular weight of the transfected MUC1-Y isoform is 29 Kd. A faint protein band at 20 Kd appeared, which is consistent with the idea that some minimal cleavage of the Y isoform occurs to yield a proteolyzed fragment whose molecular weight is consistent with that of the nat-PSMGFRTC isoform. Comparison of the glycosylated protein lanes in Fig. 39 shows a clear difference between breast cancer patient-derived MUC1 proteins (1500 cell line) and the Y isoform transfected into HEK cells ("Y" lane). Referring still to the Figure 39, the lanes that were loaded with breast tumor cell samples do not show protein bands between 35 and 45 Kd; however, the glycosylated Y isoform protein bands are quite visible and intense between 35 and 45 Kd. These results may not rule out the possibility that patient-derived breast tumor cells may produce an alternative splice isoform, such as the Y isoform, in addition to MUC1, since it may be at low concentration and not visible by western blot analysis. However, these results clearly indicate that the dominant MUC1 species being produced by these breast tumor cell lines is not the Y isoform.

The CM isoform construct produced a doublet that ran at about 28 Kd. After deglycosylation, the bands shifted to a lower molecular weight of about 25 Kd. Comparison indicates that this construct is resistant to cleavage as the lower 20 Kd band apparent in the nat-PSMGFRTC isoform construct is not present.

Similar to the CM isoform construct, the UR isoform construct produced MUC1 protein bands that ran at 29 - 30 Kd. After deglycosylation, the bands shifted to molecular weights of about 24 Kd. A faint band at 20 Kd is visible, indicating that some cleavage of this construct takes place.

The "Full Length" construct and the Rep isoform construct appear to behave identically, producing PsMGFR specific bands at 25 - 30 Kd that shift to about 23 Kd after deglycosylation. By western blot it is impossible to distinguish this species from those produced by the patient derived breast tumor cells.

The (calculated) molecular weight of a portion of the MUC1 receptor that includes the cytoplasmic tail, the transmembrane domain and the PSMGFR (unglycosylated) is about 19Kd, (i.e. nat-PSMGFRTC isoform - see SEQ ID NO: 37 - Table 1). Also discovered within the context of the present description is that breast tumor cells express a shorter form of the MUC1 receptor that normal breast cells do not express and it runs on an SDS-PAGE gel at about 20Kd (deglycosylated), suggesting that the extracellular domain of this tumor specific form consists essentially of the PSMGFR.

To ascertain whether breast tumor cells, derived from actual breast cancer patients, produce a MUC1 cleavage product that is similar to the transfection constructs described above, we analyzed the MUC1 proteins from several breast tumor cell lines using the same western blot method described above and in Example 5. Breast tumor cell lines 1500, 1504, T47D, BT-474 and MDA-MB-453 were tested. The molecular weights of the MUC1 receptor fragments were then determined by performing standard western blot analysis, again using the antibody raised against the PSMGFR, as described above. Western blots showed that the breast tumor cell lines produced several MUC1 protein bands that run between 25 and 30 Kd. As with the nat-PSMGFRTC isoform construct, deglycosylation of the breast tumor derived samples caused the series of MUC1 protein bands (25 - 30 Kd) to shift to a band having an approximate molecular weight of 20 Kd, see Fig. 43. These western blots show that the lower molecular weight MUC1 species that the breast tumor cells produce are similar to the MUC1 construct discussed above in which the receptor is truncated after the PSMGFR. These results indicate that the portion of the MUC1 receptor that remains attached to the cell surface after cleavage consists primarily of the PSMGFR sequence. It is noted that it appears that the 1500 and T47D cells may produce two cleavage products, running as a doublet on the gel having a band at about 19-20 Kd and another at about 22 Kda. The 22 Kda band appears to correspond with the band produced by the CM isoform, which includes the PSIBR at its N-terminus. Significantly, (1) no band at 19-20 Kda is evident for the CM isoform construct, and (2) the 22 Kd band was also evident in the MUC1 cleavage products produced by transfected cells expressing "healthy" forms (i.e. Full Length receptor and Rep isoform - See Fig. 44). These results support the contention that the 22 Kd band product represents a product of normal, non-aberrant cleavage and that aberrant cleavage (i.e. producing the 19-20 Kd band may be prevented by the presence of the IBR. Since the resolution of molecular weights by SDS-PAGE analysis is only accurate to within about 1 Kd, which is about 9 amino acids, the actual portion of the MUC1 receptor that remains on the surface of tumor cells may be +/- 9 - 15 amino acids N-terminal to the end of the PSMGFR and could possibly be as much as +/-20 amino acids. However, the gels show that the proteins run at approximately the same molecular weight. It is believed that these bands are cleavage products because the same gels were also probed with antibodies that recognize the repeats region of the receptor. The protein bands that stained positive for the repeats ran at molecular weights between 250 and 300 Kd, indicating that a longer version of the receptor was expressed then cleaved. Portions of the receptor that stained positive for the PSMGFR ran at a molecular weight that is consistent with the calculated molecular weight for the cytoplasmic tail, transmembrane region and the PSMGFR. Moreover, the inventors data presented herein suggests that the unique protein bands that a Y isoform construct produces are not the same as the MUC1 fragments produced by the patient-derived, or naturally occurring, breast tumor cells.

As referred to previously, one aspect of the present discussion is directed to methods for treating a subject diagnosed with or at risk of developing a cancer or tumor characterized by the aberrant expression of MUC1. The treatments involve the use of drugs or "agents" as described herein. That is, one aspect involves a series of compositions useful for treatment of cancer or tumor characterized by the aberrant expression of MUC1, including these compositions packaged in kits including instructions for use of the composition for the treatment of such conditions. That is, the kit can include a description of use of the composition for participation in any biological or chemical mechanism disclosed herein that is associated with cancer or tumor. The kit also can include instructions for use of a combination of two or more compositions as described herein. Instructions also may be provided for administering the drug orally, intravenously, or via another known route of drug delivery. These and other cases can also involve promotion of the treatment of cancer or tumor according to any of the techniques and compositions and combinations of compositions described herein.

In one set of instances, patients can be treated with compositions as described herein, even though the patients exhibit indication for treatment of one of the compositions as described herein for a condition different from cancer or tumor, including conditions that can be unrelated to cell proliferation or conditions that can accompany cell proliferation, cancer, or tumor. That is, if a composition as described herein is known for treatment of a different condition, some instances also involve use of that composition for treatments that accompany cell proliferation, cancer, or tumor disease where indicated. These and other instances can include such treatment where the dosage, delivery technique or vehicle, combination with other pharmaceutical compositions or lack of combination with other pharmaceutical compositions, rate of administration, timing of administration, or other factor differs from the use of the composition for treatment of the condition different from cell proliferation, cancer, or tumor. In another set of cases, treatment of cell proliferation, cancer, or tumor with compositions as described herein may occur under conditions that are similar to or overlap the use of compositions as described herein for treatment of a different condition, but the compositions as described herein are promoted for treatments that accompany cell proliferation, cancer, or tumor or includes instructions for treatments that accompany cell proliferation, cancer, or tumor as mentioned above. As used herein, "promoted" includes all methods of doing business including methods of education, hospital and other clinical instruction, pharmaceutical industry activity including pharmaceutical sales, and any advertising or other promotional activity including written, oral, and electronic communication of any form, associated with compositions as described herein in connection with treatments that accompany cell proliferation, cancer, or tumor. "Instructions" can and often do define a component of promotion, and typically involve written instructions on or associated with packaging of compositions as described herein. Instructions also can include any oral or electronic instructions provided in any manner. The "kit" typically, and preferably, defines a package including both any one or a combination of the compositions as described herein and the instructions, but can also include the composition as described herein and instructions of any form that are provided in connection with the composition in a manner such that a clinical professional will clearly recognize that the instructions are to be associated with the specific composition.

Subjects for whom certain treatment methods as described herein(with specific compositions directed toward cell proliferation, cancer, or tumor) are not intended are those who are diagnosed with a condition which may already call for treatment with the specific composition. Accordingly, one case described herein involves treatment of cell proliferation, cancer, or tumor with a specific composition disclosed herein for that purpose, not in combination with another agent where the other agent has been taught previously for use in treatment of cell proliferation, cancer, or tumor itself. Another instance involves treatment of cell proliferation, cancer, or tumor with this specific composition alone, not in combination with any other active agent. Another instance involves treatment of cell proliferation, cancer, or tumor with this specific composition where the use of the composition in the treatment is specifically instructed (through, e.g. written instructions that can accompany the composition) for the treatment of cell proliferation, cancer, or tumor. A preferred instance of this disclosure involves treatment of cell proliferation, cancer, or tumor with the specific composition where the use of the composition in the treatment is specifically instructed to affect a mechanism associated with cell proliferation, cancer, or tumor as disclosed herein. In yet another set of cases, the drugs and agents of described herein can be used for the purpose of disease prevention. In this context, the treatment is particularly directed to a patient population never before treated with drugs useful according to certain methods described herein, including patients who are not suffering from cell proliferation, cancer, or tumor and who may or may not be presently indicating susceptibility to cell proliferation, cancer, or tumor. In other words, the preventative treatment preferably is directed to patient populations that otherwise are free of disease symptoms that call for active treatment with any of the drugs described herein as useful according to the present disclosure.

The present disclosure involves the discovery that certain antibodies and antigen-binding fragments thereof, particularly, monovalent antibodies or monovalent antigen-binding fragments of antibodies, having specific affinity for MGFR (e.g. those raised against a PSMGFR, such as nat-PSMGFR (SEQ ID NO: 36) or var-PSMGFR (SEQ ID NO: 7) can interrupt the interaction of MGFR with its ligand(s) that otherwise would bind to MGFR and promote tumorigensis. This involves treatment of subjects associated with tumor or cancer associated with aberrant expression of MUC1 with these agents or a combination.

The method comprises administering to the subject any of the above-described antibody derived or based drugs (e.g. a monovalent anti-MGFR antibody or a monovalent MGFR-binding portion of an anti-MGFR antibody), in an amount effective to provide a medically desirable result. In one instance, the method comprises administering to the subject any one of the above-described antibody derived or based drugs (e.g. a monovalent anti-MGFR antibody or a monovalent MGFR-binding portion of an anti-MGFR antibody), in an amount effective to lower the risk/prevent/reduce/inhibit tumors or cancer associated with aberrant expression of MUC1.

The effective amount will vary with the particular condition being treated, the age and physical condition of the subject being treated, the severity of the condition, the duration of the treatment, the nature of the concurrent therapy (if any), the specific route of administration and like factors within the knowledge and expertise of the health practitioner. For example, in connection with tumor or cancer associated with abherrant expression of MUC1, an effective amount is that amount which prevents interaction of MGFR with its ligand that otherwise would promote cell proliferation (for agents that act according to that mechanism, including certain of the above-described antibody derived or based drugs (e.g. a monovalent anti-MGFR antibody or a monovalent MGFR-binding portion of an anti-MGFR antibody).

According to alternate mechanisms of drug activity, an effective amount is that amount which maintains self-aggregation of MUC1 receptors (for agents such as polymers or dendrimers that act according to that mechanism). Alternatively, an effective amount is one which reduces levels of cleaved MUC1 IBRs, or maintains low levels of cleaved MUC1 IBRs (for agents that act according to that mechanism). Likewise, an effective amount for treatment would be an amount sufficient to lessen or inhibit altogether the levels of cleaved MUC1 IBR (for agents that act according to that mechanism) so as to slow or halt the development of or the progression of tumor or cancer associated with aberrant expression of MUC1. It is preferred generally that a maximum dose be used, that is, the highest safe dose according to sound medical judgment

When used therapeutically, the agents described herein are administered in therapeutically effective amounts. In general, a therapeutically effective amount means that amount necessary to delay the onset of, inhibit the progression of, or halt altogether the particular condition being treated. Generally, a therapeutically effective amount will vary with the subject's age, condition, and sex, as well as the nature and extent of the disease in the subject, all of which can be determined by one of ordinary skill in the art. The dosage may be adjusted by the individual physician or veterinarian, particularly in the event of any complication. A therapeutically effective amount typically varies from 0.01 mg/kg to about 1000 mg/kg. It is expected that does ranging from 1-500 mg/kg, and preferably doses ranging from 1-50 mg/kg will be suitable. In other cases, the agents will be administered in doses ranging from 1 µg/kg/day to 10 mg/kg/day, with even more preferred doses ranging from 1-200 µg/kg/day, 1-100 µg/kg/day, 1-50 µg/kg/day or from 1-25 µg/kg/day. In other cases, dosages may range from about 0.1 mg/kg to about 200 mg/kg, and most preferably from about 0.2 mg/kg to about 20 mg/kg. These dosages can be applied in one or more dose administrations daily, for one or more days.

The agent described herein should be administered for a length of time sufficient to provide either or both therapeutic and prophylactic benefit to the subject. Generally, the agent is administered for at least one day. In some instances, the agent may be administered for the remainder of the subject's life. The rate at which the agent is administered may vary depending upon the needs of the subject and the mode of administration. For example, it may be necessary in some instances to administer higher and more frequent doses of the agent to a subject for example during or immediately following a event associated with tumor or cancer, provided still that such doses achieve the medically desirable result. On the other hand, it may be desirable to administer lower doses in order to maintain the medically desirable result once it is achieved. In still other cases, the same dose of agent may be administered throughout the treatment period which as described herein may extend throughout the lifetime of the subject. The frequency of administration may vary depending upon the characteristics of the subject. The agent may be administered daily, every 2 days, every 3 days, every 4 days, every 5 days, every week, every 10 days, every 2 weeks, every month, or more, or any time there between as if such time was explicitly recited herein.

In one case, daily doses of active compounds will be from about 0.01 milligrams/kg per day to 1000 milligrams/kg per day. It is expected that oral doses in the range of 50 to 500 milligrams/kg, in one or several administrations per day, will yield the desired results. Dosage may be adjusted appropriately to achieve desired drug levels, local or systemic, depending upon the mode of administration. In the event that the response in a subject is insufficient at such doses, even higher doses (or effective higher doses by a different, more localized delivery route) may be employed to the extent that patient tolerance permits. Multiple doses per day are contemplated to achieve appropriate systemic levels of compounds.

Preferably, such agents are used in a dose, formulation and administration schedule which favor the activity of the agent and do not impact significantly, if at all, on normal cellular functions.

In one case, the degree of activity of the drug is at least 10%. In other cases, the degree of activity of the drug is as least 20%, at least 30%, at least 40%, at least 50%, at least 60%, at least 70%, at least 80%, at least 90%, or at least 95%.

When administered to subjects for therapeutic purposes, the formulations described herein are applied in pharmaceutically acceptable amounts and in pharmaceutically acceptable compositions. Such a pharmaceutical composition may include the agents described herein in combination with any standard physiologically and/or pharmaceutically acceptable carriers which are known in the art. The compositions should be sterile and contain a therapeutically effective amount of the agent in a unit of weight or volume suitable for administration to a patient. The term "pharmaceutically-acceptable carrier" as used herein means one or more compatible solid or liquid filler, diluents or encapsulating substances which are suitable for administration into a human or other animal. The term "carrier" denotes an organic or inorganic ingredient, natural or synthetic, with which the active ingredient is combined to facilitate the application. The components of the pharmaceutical compositions also are capable of being co-mingled with the molecules described herein, and with each other, in a manner such that there is no interaction which would substantially impair the desired pharmaceutical efficacy. Pharmaceutically acceptable further means a non-toxic material that is compatible with a biological system such as a cell, cell culture, tissue, or organism. The characteristics of the carrier will depend on the route of administration. Physiologically and pharmaceutically acceptable carriers include diluents, fillers, salts, buffers, stabilizers, solubilizers, and other materials which are well known in the art.

Such preparations may routinely contain salts, buffering agents, preservatives, compatible carriers, and optionally other therapeutic ingredients. When used in medicine the salts should be pharmaceutically acceptable, but non-pharmaceutically acceptable salts may conveniently be used to prepare pharmaceutically acceptable salts thereof and are not excluded from the scope of the disclosed prepartions. Such pharmacologically and pharmaceutically acceptable salts include, but are not limited to, those prepared from the following acids: hydrochloric, hydrobromic, sulphuric, nitric, phosphoric, maleic, acetic, salicylic, p-toluene
sulfonic, tartaric, citric, methane sulfonic, formic, malonic, succinic, naphthalene-2-sulfonic, and benzene sulfonic. Also, pharmaceutically acceptable salts can be prepared as alkaline metal or alkaline earth salts, such as sodium, potassium or calcium salts of the carboxylic acid group.

Suitable buffering agents include: acetic acid and a salt (1-2% W/V); citric acid and a salt (1-3% W/V); boric acid and a salt (0.5-2.5% W/V); and phosphoric acid and a salt (0.8-2% W/V).

Suitable preservatives include benzalkonium chloride (0.003-0.03% W/V); chlorobutanol (0.3-0.9% W/V); parabens (0.01-0.25% W/V) and thimerosal (0.004-0.02% W/V).

A variety of administration routes are available. The particular mode selected will depend, of course, upon the particular combination of drugs selected, the severity of the cancer condition being treated, the condition of the patient, and the dosage required for therapeutic efficacy. The methods disclosed herein, generally speaking, may be practiced using any mode of administration that is medically acceptable, meaning any mode that produces effective levels of the active compounds without causing clinically unacceptable adverse effects. Such modes of administration include oral, rectal, topical, nasal, other mucosal forms, direct injection, transdermal, sublingual or other routes. "Parenteral" routes include subcutaneous, intravenous, intramuscular, or infusion. Direct injection may be preferred for local delivery to the site of the cancer. Oral administration may be preferred for prophylactic treatment e.g., in a subject at risk of developing a cancer, because of the convenience to the patient as well as the dosing schedule.

Chemical/physical vectors may be used to deliver the agents disclosed herein to a target (e.g. cell) and facilitate uptake thereby. As used herein, a "chemical/physical vector" refers to a natural or synthetic molecule, other than those derived from bacteriological or viral sources, capable of delivering the agent disclosed herein to a target (e.g. cell).

A preferred chemical/physical vector is a colloidal dispersion system. Colloidal dispersion systems include lipid-based systems including oil-in-water emulsions, micelles, mixed micelles, and liposomes. A preferred colloidal system is a liposome. Liposomes are artificial membrane vessels which are useful as a delivery vector in vivo or in vitro. It has been shown that large unilamellar vessels (LUV), which range in size from 0.2-4.0.mu. can encapsulate large macromolecules. RNA, DNA,
and intact virions can be encapsulated within the aqueous interior and be delivered to cells in a biologically active form (Fraley, et al., Trends Biochem. Sci., v. 6, p. 77 (1981)). In order for a liposome to be an efficient gene transfer vector, one or more of the following characteristics should be present: (1) encapsulation of the gene of interest at high efficiency with retention of biological activity; (2) preferential and substantial binding to a target cell in comparison to non-target cells; (3) delivery of the aqueous contents of the vesicle to the target cell cytoplasm at high efficiency; and (4) accurate and effective expression of genetic information.

Liposomes may be targeted to a particular (e.g. tissue), such as (e.g. the vascular cell wall), by coupling the liposome to a specific ligand such as a monoclonal antibody, sugar, glycolipid, or protein.

Liposomes are commercially available from Gibco BRL, for example, as LIPOFECTIN™. and LIPOFECTACE™., which are formed of cationic lipids such as N-[1-(2,3 dioleyloxy)-propyl]-N, N, N-trimethylammonium chloride (DOTMA) and dimethyl dioctadecylammonium bromide (DDAB). Methods for making liposomes are well known in the art and have been described in many publications. Liposomes also have been reviewed by Gregoriadis, G. in Trends in Biotechnology, V. 3, p. 235-241 (1985).

In one particular instance, the preferred vehicle is a biocompatible micro particle or implant that is suitable for implantation into the mammalian recipient. Exemplary bioerodible implants that are useful in accordance with this method are described in PCT International application no. PCT/US/03307 (Publication No. WO 95/24929, entitled "Polymeric Gene Delivery System", claiming priority to U.S. patent application Ser. No. 213,668, filed Mar. 15, 1994). PCT/US/0307 describes a biocompatible, preferably biodegradable polymeric matrix for containing an exogenous gene under the control of an appropriate promoter. The polymeric matrix is used to achieve sustained release of the exogenous gene in the patient. In accordance with the instant disclosure, the agent described herein is encapsulated or dispersed within the biocompatible, preferably biodegradable polymeric matrix disclosed in PCT/US/03307. The polymeric matrix preferably is in the form of a micro particle such as a micro sphere (wherein the agent is dispersed throughout a solid polymeric matrix) or a microcapsule (wherein the agent is stored in the core of a polymeric shell). Other forms of the polymeric matrix for containing the agents described herein include films, coatings, gels, implants, and stents.
The size and composition of the polymeric matrix device is selected to result in favorable release kinetics in the tissue into which the matrix device is implanted. The size of the polymeric matrix devise further is selected according to the method of delivery which is to be used, typically injection into a tissue or administration of a suspension by aerosol into the nasal and/or pulmonary areas. The polymeric matrix composition can be selected to have both favorable degradation rates and also to be formed of a material which is bioadhesive, to further increase the effectiveness of transfer when the devise is administered to a vascular surface. The matrix composition also can be selected not to degrade, but rather, to release by diffusion over an extended period of time.

Both non-biodegradable and biodegradable polymeric matrices can be used to deliver agents described herein to the subject. Biodegradable matrices are preferred. Such polymers may be natural or synthetic polymers. Synthetic polymers arc preferred. The polymer is selected based on the period of time over which release is desired, generally in the order of a few hours to a year or longer. Typically, release over a period ranging from between a few hours and three to twelve months is most desirable. The polymer optionally is in the form of a hydrogel that can absorb up to about 90% of its weight in water and further, optionally is cross-linked with multi-valent ions or other polymers.

In general, the agents described herein are delivered using the bioerodible implant by way of diffusion, or more preferably, by degradation of the polymeric matrix. Exemplary synthetic polymers which can be used to form the biodegradable delivery system include: polyamides, polycarbonates, polyalkylenes, polyalkylene glycols, polyalkylene oxides, polyalkylene terepthalates, polyvinyl alcohols, polyvinyl ethers, polyvinyl esters, polyvinyl halides, polyvinylpyrrolidone, polyglycolides, polysiloxanes, polyurethanes and co-polymers thereof, alkyl cellulose, hydroxyalkyl celluloses, cellulose ethers, cellulose esters, nitro celluloses, polymers of acrylic and methacrylic esters, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), polyvinyl acetate, poly vinyl chloride, polystyrene and polyvinylpyrrolidone.

Examples of non-biodegradable polymers include ethylene vinyl acetate, poly(meth)acrylic acid, polyamides, copolymers and mixtures thereof.

Examples of biodegradable polymers include synthetic polymers such as polymers of lactic acid and glycolic acid, polyanhydrides, poly(ortho)esters, polyurethanes, poly(butic acid), poly(valeric acid), and poly(lactide-cocaprolactone), and natural polymers such as alginate and other polysaccharides including dextran and cellulose, collagen, chemical derivatives thereof (substitutions, additions of chemical groups, for example, alkyl, alkylene, hydroxylations, oxidations, and other modifications routinely made by those skilled in the art), albumin and other hydrophilic proteins, zein and other prolamines and hydrophobic proteins, copolymers and mixtures thereof. In general, these materials degrade either by enzymatic hydrolysis or exposure to water in vivo, by surface or bulk erosion.

Bioadhesive polymers of particular interest include bioerodible hydrogels described by H. S. Sawhney, C. P. Pathak and J. A. Hubell in Macromolecules, 1993, 26, 581-587, polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate). Thus, provided is a composition of the above-described agents for use as a medicament, methods for preparing the medicament and methods for the sustained release of the medicament in vivo.

The compositions may conveniently be presented in unit dosage form and may be prepared by any of the methods well known in the art of pharmacy. All methods include the step of bringing the therapeutic agents into association with a carrier which constitutes one or more accessory ingredients. In general, the compositions are prepared by uniformly and intimately bringing the therapeutic agent into association with a liquid carrier, a finely divided solid carrier, or both, and then, if necessary, shaping the product.

Compositions suitable for parenteral administration conveniently comprise a sterile aqueous preparation of the therapeutic agent, which is preferably isotonic with the blood of the recipient. This aqueous preparation may be formulated according to known methods using those suitable dispersing or wetting agents and suspending agents. The sterile injectable preparation may also be a sterile injectable solution or suspension in a non-toxic parenterally-acceptable diluent or solvent, for example as a solution in 1, 3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono or di-glycerides. In addition, fatty acids such as oleic acid find use in the preparation of injectables. Carrier formulations suitable for oral, subcutaneous, intravenous, intramuscular, etc. can be found in Remington's Pharmaceutical Sciences, Mack Publishing Company, Easton, PA.

Compositions suitable for oral administration may be presented as discrete units such as capsules, cachets, tablets, or lozenges, each containing a predetermined amount of the therapeutic agent. Other compositions include suspensions in aqueous liquors or non-aqueous liquids such as a syrup, an elixir, or an emulsion.

Other delivery systems can include time-release, delayed release or sustained release delivery systems. Such systems can avoid repeated administrations of the therapeutic agent described herein, increasing convenience to the subject and the physician. Many types of release delivery systems are available and known to those of ordinary skill in the art. They include polymer based systems such as polylactic and polyglycolic acid, poly(lactide-glycolide), copolyoxalates, polyanhydrides, polyesteramides, polyorthoesters, polyhydroxybutyric acid, and polycaprolactone. Microcapsules of the foregoing polymers containing drugs are described in, for example, U.S. Pat. No. 5,075,109. Nonpolymer systems that are lipids including sterols such as cholesterol, cholesterol esters and fatty acids or neutral fats such as mono-, di- and tri-glycerides; liposomes; phospholipids; hydrogel release systems; silastic systems; peptide based systems; wax coatings, compressed tablets using conventional binders and excipients, partially fused implants and the like. Specific examples include, but are not limited to: (a) erosional systems in which the polysaccharide is contained in a form within a matrix, found in U.S. Patent Nos. 4,452,775, 4,675,189, and 5,736,152, and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

Use of a long-term sustained release implant may be particularly suitable for treatment of established cancer conditions as well as subjects at risk of developing a cancer. "Long-term" release, as used herein, means that the implant is constructed and arranged to deliver therapeutic levels of the active ingredient for at least 7 days, and preferably 30-60 days. The implant may be positioned at the site of the tumor. Long-term sustained release implants are well known to those of ordinary skill in the art and include some of the release systems described above.

The therapeutic agent may be administered in alone or in combination with an anti-cancer drug. If the therapeutic agent is administered in combination the compounds may be administered by the same method, e.g. intravenous, oral, etc. or may be administered separately by different modes, e.g. therapeutic agent administered orally, anti-cancer drug administered intravenously, etc. In one case the therapeutic agent and the anti-cancer drug are co-administered intravenously. In another case the therapeutic agent and the anti-cancer drug are administered separately.

Anti-cancer drugs that can be co-administered with the compounds described herein include, but are not limited to Acivicin; Aclarubicin; Acodazole Hydrochloride; Acronine; Adriamycin; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Etoposide; Etoposide Phosphate; Etoprine; Fadrozole Hydrochloride; Fazarabine; Fenretinide; Floxuridine; Fludarabine Phosphate; Fluorouracil; Flurocitabine; Fosquidone; Fostriecin Sodium; Gemcitabine; Gemcitabine Hydrochloride; Hydroxyurea; Idarubicin Hydrochloride; Ifosfamide; Ilmofosine; Interferon Alfa-2a; Interferon Alfa-2b; Interferon Alfa-n1; Interferon Alfa-n3; Interferon Beta- I a; Interferon Gamma-I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Sulofenur; Talisomycin; Taxol; Tecogalan Sodium; Tegafur; Teloxantrone Hydrochloride; Temoporfin; Teniposide; Teroxirone; Testolactone; Thiamiprine; Thioguanine; Thiotepa; Tiazofurin; Tirapazamine; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride. Additional antineoplastic agents include those disclosed in Chapter 52, Antineoplastic Agents (Paul Calabresi and Bruce A. Chabner), and the introduction thereto, 1202-1263, of Goodman and Gilman's "The Pharmacological Basis of Therapeutics", Eighth Edition, 1990, McGraw-Hill, Inc. (Health Professions Division).

**Table 1: Peptide sequences (listed from N-terminus to C-terminus):**

| |
|---|
| Histidine-Tagged Truncated receptor (His-TR) (having "SPY" sequence of var-PSMGFR): |
| GTINVHDVETQFNQYKTEAA**SPY**NLTISDVSVSHHHHHH (SEQ ID NO: 1) |
| |
| An example of a Histidine-Tagged Primary Sequence of the MUC1 Growth Factor Receptor (His-var-PSMGFR) (having "SPY" sequence of var-PSMGFR): |
| GTINVHDVETQFNQYKTEAASPYNLTISDVSVSDVPFPFSAQSGAHHHHHH (SEQ ID NO: 2) |
| |
| An example of a Histidine-Tagged Primary Sequence of the MUC 1 Growth Factor Receptor (His-var-PSMGFR) (having "SPY" sequence of var-PSMGFR) having a single amino acid deletion at the C-terminus of SEQ ID NO: 2): |
| TINVHDVETQFNQYKTEAA**SPY**NLTISDVSVSDVPFPFSAQSGAHHHHHH (SEQ ID NO: 60) |
| |
| Histidine-Tagged Extended Sequence of MUC1 Growth Factor Receptor (ESMGFR) (having "SPY" sequence of var-PSMGFR): |
| |
| |
| Histidine-Tagged Tumor-Specific Extended Sequence of MUC1 Growth Factor Receptor (TSESMGFR) (having "SPY" sequence of var-PSMGFR): |
| |
| |
| Histidine-Tagged Primary Sequence of the Interchain binding Region (His-PSIBR): HHHHHHGFLGLSNIKFRPGSVVVQLTLAFRE (SEQ ID NO: 4) |
| |
| Histidine-Tagged Truncated Interchain binding Region (His-TPSIBR): HHHHHHSVWQLTLAFREG (SEQ ID NO: 62) |
| |
| Histidine-Tagged Repeat Motif 2 (His-RM2): |
| PDTRPAPGSTAPPAHGVTSAPDTRPAPGSTAPPAHGVTSAHHHHHH (SEQ ID NO: 5) |
| Truncated PSMGFR receptor (TR) (having "SPY" sequence of var-PSMGFR): |
| GTINVHDVETQFNQYKTEAA**SPY**NLTISDVSVS (SEQ ID NO: 6) |
| |
| Native Primary Sequence of the MUC1 Growth Factor Receptor (nat-PSMGFR - An example of "PSMGFR"): |
| GTINVHDVETQFNQYKTEAA**SRY**NLTISDVSVSDVPFPFSAQSGA (SEQ ID NO: 36) |
| |
| Native Primary Sequence of the MUC1 Growth Factor Receptor (nat-PSMGFR-An example of "PSMGFR"), having a single amino acid deletion at the C-terminus of SEQ ID NO: 36): |
| TINVHDVETQFNQYKTEAA**SRY**NLTISDVSVSDVPFPFSAQSGA (SEQ ID NO: 63) |
| |
| "SPY" functional variant of the native Primary Sequence of the MUC1 Growth Factor Receptor having enhanced stability (var-PSMGFR - An example of "PSMGFR"): |
| GTINVHDVETQFNQFNQYKTEAA**SPY**NLTISDVSVSDVPFPFSAQSGA (SEQ ID NO: 7) |
| |
| "SPY" functional variant of the native Primary Sequence of the MUC1 Growth Factor Receptor having enhanced stability (var-PSMGFR -An example of "PSMGFR"), having a single amino acid deletion at the C-terminus of SEQ ID NO: 7): |
| TINVHDVETQFNQYKTEAA**SPY**NLTISDVSVSDVPFPFSAQSGA (SEQ ID NO: 64) |
| |
| Primary Sequence of the Interchain Binding Region) (TPSIBR): GFLGLSNIKFRPGSVVVQLTLAFRE (SEQ ID NO: 8) |
| |
| Truncated Interchain Binding Region) (PSIBR): SVWQLTLAFREG (SEQ ID NO: 65) |
| |
| Repeat Motif 2 (RM2): |
| PDTRPAPGSTAPPAHGVTSAPDTRPAPGSTAPPAHGVTSA (SEQ ID NO: 9) |
| |
| Tumor-Specific Extended Sequence of MUC1 Growth Factor Receptor (TSESMGFR) (having "SPY" sequence of var-PSMGFR): |
| |
| |
| Full-length MUC1 Receptor (Mucin 1 precursor, Genbank Accession number: P15941 |
| |
| A truncated MUC1 receptor isoform having nat-PSMGFR at its N-terminus and including the transmembrane and cytoplasmic sequences of a full-length MUC1 receptor ("nat-PSMGFRTC isoform" - An example of "PSMGFRTC" - shown excluding optional N-terminus signal sequence - SEQ ID NOS: 47, 58, or 59 which may be cleaved after translation and prior to expression of the receptor on the cell surface): |
| |
| |
| A truncated MUC1 receptor isoform having nat-PSMGFR and PSIBR at its N-terminus and including the transmembrane and cytoplasmic sequences of a full-length MUC 1 receptor ("CM isoform"- shown excluding optional N-terminus signal sequence - S SEQ ID NOS: |
| 47, 58, or 59 which may be cleaved after translation and prior to expression of the receptor on the cell surface): |
| |
| |
| A truncated MUC1 receptor isoform having nat-PSMGFR + PSIBR + Unique Region at its N-terminus and including the transmembrane and cytoplasmic sequences of a full-length MUC1 receptor ("UR isoform"- shown excluding optional N-terminus signal sequences SEQ ID NO: 47, 58, or 59): |
| |
| A truncated MUC1 receptor isoform including the transmembrane and cytoplasmic sequences of a full-length MUC1 receptor ("Y isoform"- shown excluding optional N-terminus signal sequence - SEQ ID NOS: 47, 58, or 59 which may be cleaved after translation and prior to expression of the receptor on the cell surface): |
| |
| |
| A truncated MUC1 receptor isoform having nat-PSMGFR + PSIBR + Unique Region + Repeats at its N-terminus and including the transmembrane and cytoplasmic sequences of a full-length MUC1 receptor ("Rep isoform"- shown excluding optional N-terminus signal sequence - SEQ ID NOS: 47, 58, or 59 which may be cleaved after translation and prior to expression of the receptor on the cell surface): |
| |
| |
| N-terminal MUC-1 signaling sequence for directing MUC1 receptor and truncated isoforms to cell membrane surface (optionally present, in whole or part - e.g. up to 3 a.a. may be absent at C-terminal end as indicated by variants in SEQ ID NOS: 47, 58, and 59, at N-terminus of above-listed MUC1 truncated receptor isoforms): |
| MTPGTQSPFFLLLLLTVLT (SEQ ID NO: 47). |
| MTPGTQSPFFLLLLLTVLT VVTA (SEQ ID NO: 58) |
| MTPGTQSPFFLLLLLTVLT WTG (SEQ ID NO: 59) |
| Proopiomelanocortin (adrenocorticotropin/ beta-lipotropin/ alpha-melanocyte stimulating hormone/ beta-melanocyte stimulating hormone/ beta-endorphin) [Homo sapiens]. |
| Accession number: XP_002485 |
| |
| |
| |
| RGD |
| HHHHHHSSSSGSSSSGSSSSGGRGDSGRGDS (SEQ ID NO: 12) |

**Table 2: Nucleic acid sequences encoding for truncated isoforms of MUC1 receptor (listed from 5'-terminus to 3'-terminus):**

| |
|---|
| An example of a nucleic acid molecule encoding the nat-PSMGFRTC of SEQ ID NO: 37: |
| |
| |
| |
| An example of a nucleic acid molecule encoding the CM isoform of SEQ ID NO: 38: |
| |
| |
| An example of a nucleic acid molecule encoding the UR isoform of SEQ ID NO: 39: |
| |
| |
| |
| An example of a nucleic acid molecule encoding the Y isoform of SEQ ID NO: 40: |
| |
| An example of a nucleic acid molecule encoding the Rep isoform of SEQ ID NO: 41: |
| |
| |
| |
| An example of a nucleic acid molecule encoding the full-length MUC1 receptor of SEQ ID NO: 10: |
| |
| |
| |

The following examples are intended to illustrate the benefits of the present invention, but do not exemplify the full scope of the invention.

### EXAMPLES

### Colloid Preparation/Drug Screening Methods Employed in the Examples

In certain examples and cases discussed herein, use is made of self-assembled monolayers (SAMs) on surfaces of colloid particles. Colloids were derivatized with SAMs and prepared for drug screening in a manner similar to that described in International Patent Publication No. WO 00/43791, published July 27, 2000, entitled "Rapid and Sensitive Detection of Aberrant Protein Aggregation in Neurodegenerative Diseases".

In a typical example, 1.5 ml of commercially available gold colloid (Auro Dye by Amersham) were pelleted by centrifugation in a microfuge on high for 10 minutes. The pellet was resuspended in 100 µL of the storage buffer (sodium citrate and tween-20). 100 µL of a dimethyl formamide (DMF) solution containing thiols. Following a 3-hour incubation in the thiol solution, the colloids were pelleted and the supernatant discarded. They were then heat cycled in 100 µL of 400 µM tri-ethylene glycol-terminated thiol in DMF for 2 minutes at 55°C, 2 minutes at 37°C, 1 minute at 55°C, 2 minutes at 37°C, then room temperature for 10 minutes. Heat cycling results in the elimination of any species that are not in the lowest energy confirmation, resulting in a stable, close-packed, self-assembled monolayer. Heat cycling can be carried out with any of a wide variety of self-assembled monolayer-forming species. The colloids were then pelleted and 100 µL 100mM NaCl phosphate buffer were added. The colloids were then diluted 1:1 with 180 µM NiSO₄ in the colloid storage buffer.

Thiols used in coating colloids typically were derived from solutions containing about 40 µM nitrilo tri-acetic acid (NTA)-thiol, and other thiols such as methyl-terminated thiol (HS-(CH2)15 CH3), 40% tri-ethylene glycol-terminated thiol, HS(CH₂)₁₁(CH₂CH2)₃OH, (formula) and 50% poly (ethynylphenyl) thiol (C₁₆H₁₀S). Different thiols were used to selectively inhibit non-specific binding optimally.

Colloid aggregation can be sensitively detected by monitoring color change of colloid particles which are initially disperse in suspension. Aggregation results in a color change to blue. No auxiliary signaling entity is necessary. In drug screening, aggregation (or lack thereof) is observed in the presence of candidate drugs.

### Example 1: Dimerization of the MGFR portion of the MUC1 receptor triggers enhanced Cell Proliferation Consistent with the Mechanism Presented for MUC1 Tumor Cells

This example demonstrates the effect of dimerization on the MUC1 receptor. In this example it is shown that exposure of cells to a bivalent antibody grown against the MGFR region of the MUC1 receptor, at varying concentration, results in enhanced cell proliferation (or lack thereof) consistent with the mechanism presented for MUC1 tumor cells. A bivalent antibody was raised against either var-PSMGFR or nat-PSMGFR sequences shown in Table 1 (i.e., a single antibody having the ability to bind simultaneously to two MGFRs was produced). MUC1 tumor cells (T47Ds) were exposed to this antibody, and cell proliferation was studied as a function of concentration of the antibody. A growth/response curve typical of a growth factor/receptor - antibody response was observed. Specifically, at concentration low enough that only a small portion of the cells were exposed to the antibody, cell proliferation was low. At a concentration of antibody high enough that one antibody could bind adjacent MGFRs, cell proliferation was maximized. At a high excess of antibody, each antibody bound only a single MGFR, rather than dimerizing adjacent MGFRs, and proliferation was reduced.

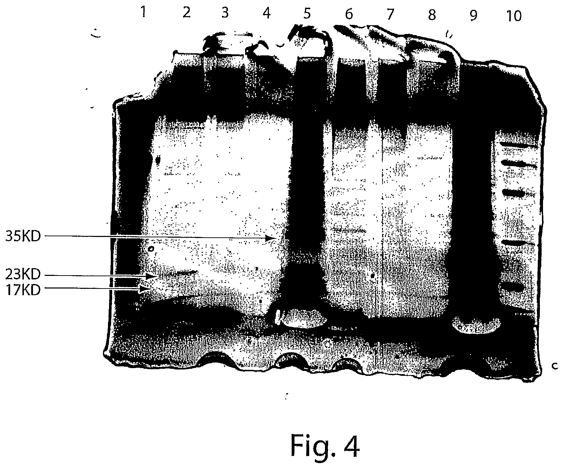

T47D (HTB-133) cells, a human breast cancer cell line that overexpresses MUC1, were cultured to 30% confluency. An antibody raised against the PSMGFR portion of the MUC1 receptor, i.e. an antibody to the MFGR (produced under contract with the inventors from nat-PSMGFR or var-PSMGFR peptide sequences supplied by the inventors by Zymed, San Francisco, California, USA), was added to cells at varying concentrations in a multi-well cell culture plate. As a negative control, a second set of T47D cells was treated with an irrelevant antibody (anti-streptavidin). Prior to adding antibody, cells were counted (at time zero). All experiments were performed in triplicate. Cells were allowed to grow in a CO₂ incubator under normal conditions. Cells were counted using a hemacytometer (3 counts per well) at 24 hours and again at 48 hours. Results, see Fig. 4, show that in a concentration-dependent manner, addition of antibody caused enhanced cell proliferation compared to the proliferation of the same cells treated with a control antibody. Figure 4 is a graph in which measured cell growth at 24 and 48
hours is plotted as a function of anti-MGFR concentration. At the optimal antibody concentration, when presumably one antibody binds bivalently to two MGFR portions of the MUC1 receptor, i.e. dimerizes the receptor, cell proliferation is at a maximum.

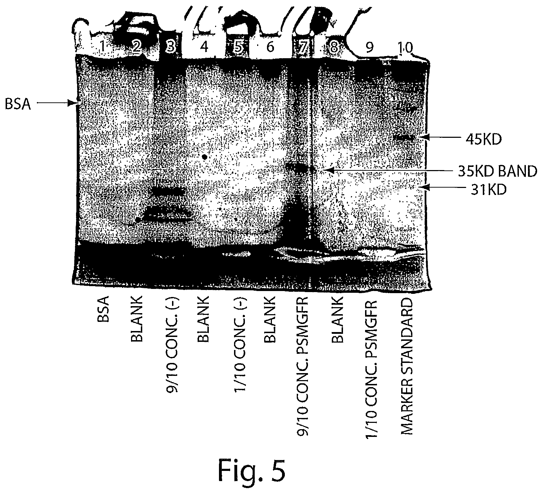

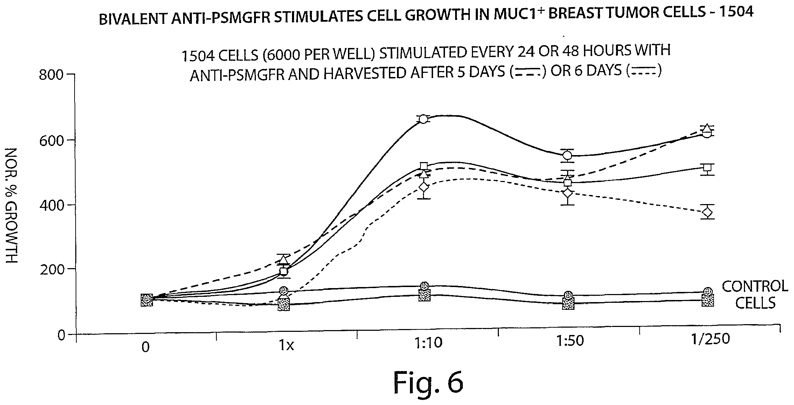

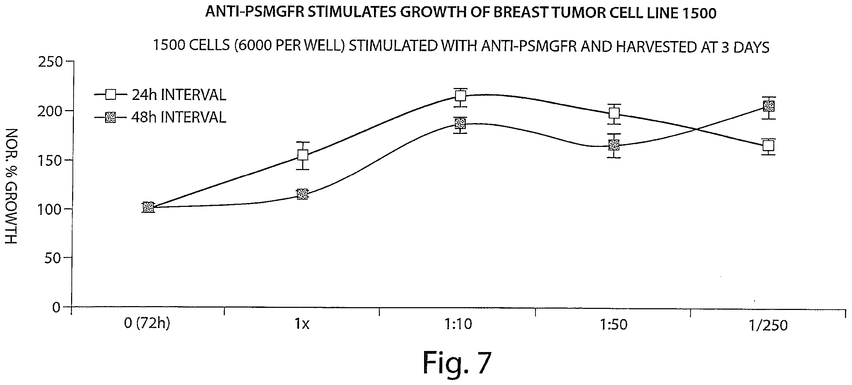

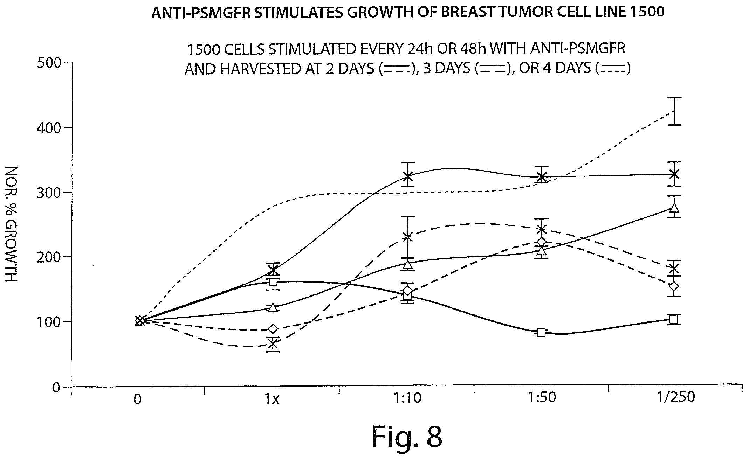

In a similar experiment, a concentration of the anti-MGFR antibody, identified to maximize cell proliferation, was added to a first group of T47D tumor cells, grown as described above. The same amount of the anti-MGFR antibody was added to a set of control cells, K293 cells. Figure 5 shows that the addition of the anti-MGFR antibody to MUC1 tumor cells (T47D) enhanced proliferation by 180% 24 hours, but had no effect on the control cells. The growth of the T47D cells plateaued to saturation, for cells with added antibody, at 48 hours. Control cells never reached saturation within the time frame of the experiment and were at 70% confluency at 48 hours.

### Example 2: Identification of Ligands that bind to the MGFR portion of the MUC1 receptor

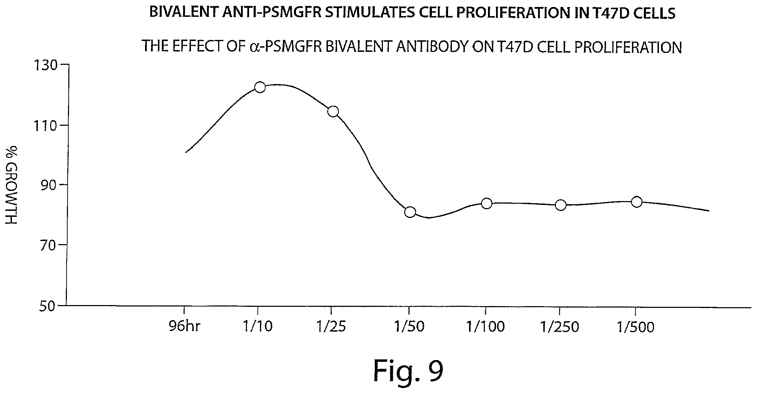

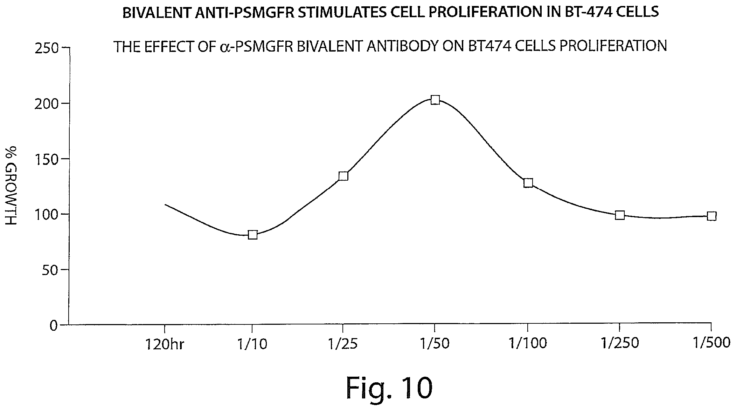

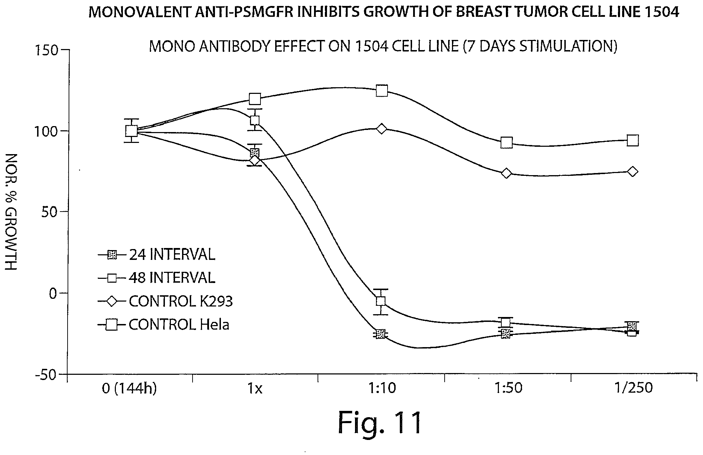

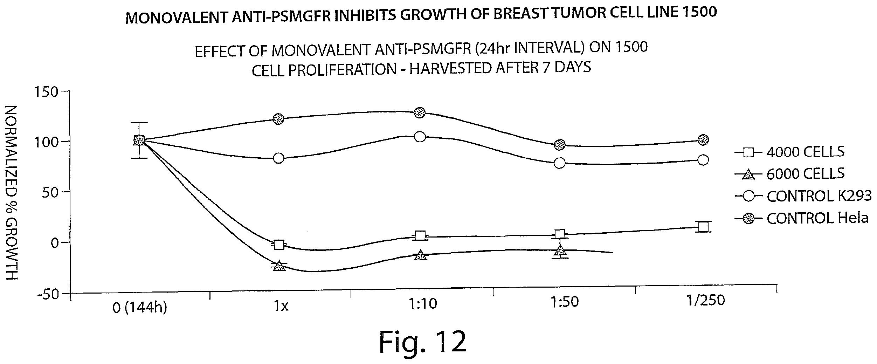

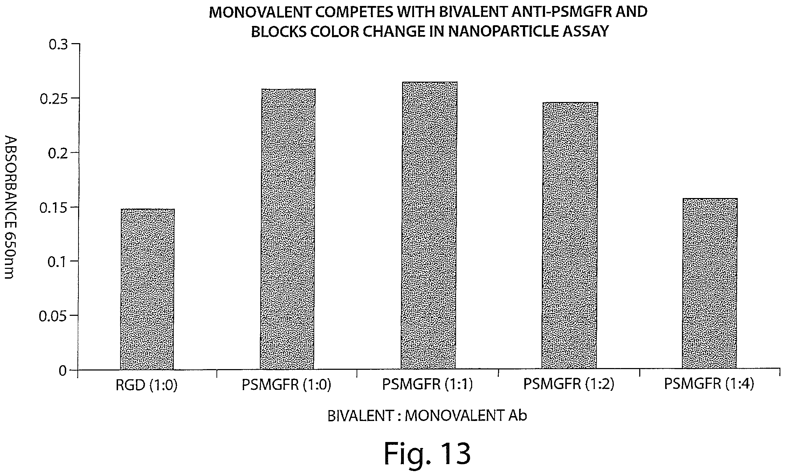

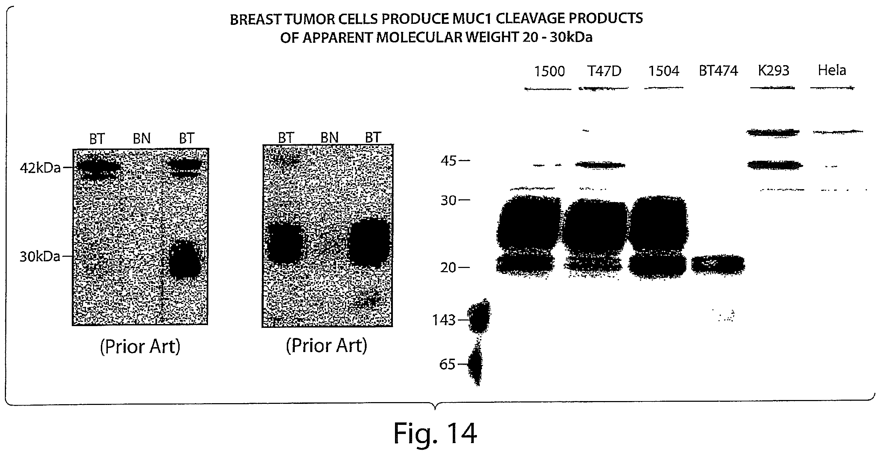

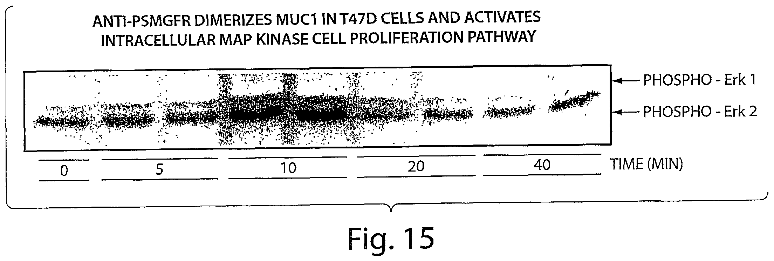

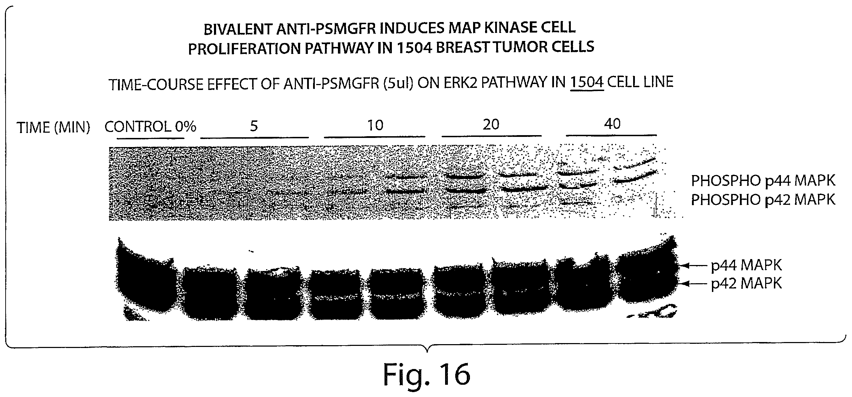

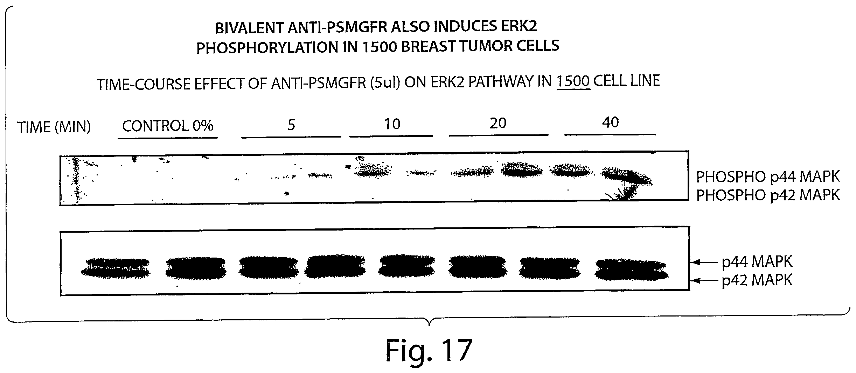

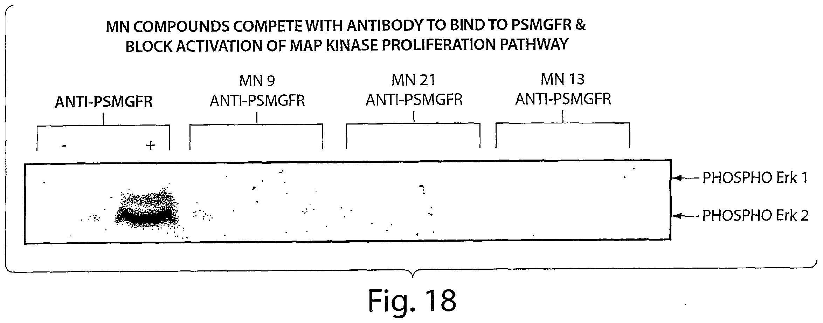

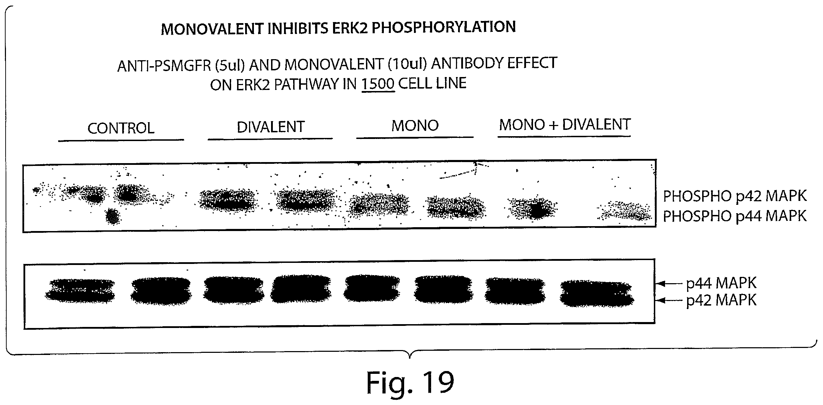

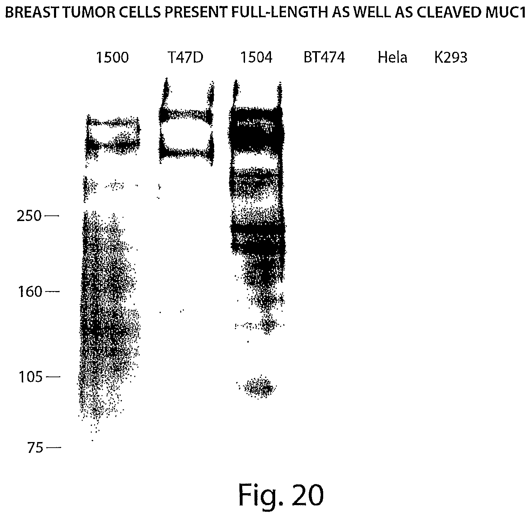

In an effort to identify ligands to the MUC1 receptor, synthetic, His-var-PSMGFR peptides, GTINVIIDVETQFNQYKTEAASPYNLTISDVSVSDVPFPFSAQSGAHHHHHH (SEQ ID NO: 2), which is representative of the portion of the MUC1 receptor, that remains attached to the cell surface after cleavage of the interchain binding region, were loaded onto NTA-Ni beads (cat. #1000630; available from Qiagen GmbH, Germany) and incubated with cell lysates in the presence (Fig. 9) or absence (Fig. 10) of the protease inhibitor PMSF (phenyl methyl sulfonyl fluoride). Lysates from T47D cells were used because this breast tumor cell line is known to overexpress MUC1 and MUC1 ligand(s). T47D cells were cultured then sonicated for 1 minute to lyse the cells. Lysates were mixed with the PSMGFR peptide-presenting beads and incubated on ice with intermittent mixing for 1hr. As a negative control, an irrelevant peptide, HHHHHHRGEFTGTYITAVT (SEQ ID NO: 13), was attached to NTA-Ni beads and treated identically. Both sets of beads were washed 2X with phosphate buffer pH 7.4. Bound protein species were eluted by 3 additions of 100uL of phosphate buffer that also contained 250mM imidazole. For both the peptides, a portion of the first elution was removed and reserved to run as a separate sample, while the remainder was combined with the other 2 elutions and concentrated by TCA (tri-chloro acetic acid)-precipitation (Chen, L. et al., Anal. Biochem. Vol 269; pgs 179-188; 1999). Eluates were run on a 12% SDS gel, see Figure 9. The gel was then silver stained (Schevchenko, A et al; Anal. Chem., Vol. 68; pg 850-858; 1996). Lanes were loaded as follows: (from left to right) (1) Benchmark pre-stained protein ladder (Gibco); (2) first eluate from the MUC1 peptide; (3) 1/10^{th} of TCA-concentrated sample; (4) blank; (5) 9/10^{th} TCA- concentrated sample; (6) first eluate negative control peptide; (7) 1/10^{th} of TCA-concentrated sample from the negative control peptide; (8) 0.5 picomoles BSA (as a standard); (9) 9/10^{th} TCA- concentrated sample from the negative control peptide; (10) silver stain SDS page standard (BioRad cat. #1610314). Referring now to Fig. 9, comparing lanes 2 and 6 (control), it can be seen that the MUC1 PSMGFR peptide bound distinguishably to three peptides: a first unique peptide that runs at an apparent molecular weight of 17kD; and a second peptide (more intense band) that runs at an apparent molecular weight of 23kD. Note that in lane 5, where the sample is the most concentrated, a third unique band is seen at about 35kD.

Figure 10 shows the results of an experiment, which was identical to that shown in Fig. 9, with the exception that the protease inhibitor PMSF was not added. PMSF binds to and blocks the action of several enzymes, such as proteases. This experiment was performed, in the absence of PMSF, to determine whether an enzyme present in the lysate was a ligand of the MUC1 receptor. Referring now to Fig. 10, comparing lanes 3 (control) and 7, it can be seen that the MUC1, PSMGFR peptide bound distinguishably to one peptide, with an apparent molecular weight of 35kD. Note that this band was visible in Fig. 9 (with PMSF), but was much fainter and only co-eluted from the most concentrated sample. These results are consistent with the idea that the PFMGFR portion of the MUC1 receptor is a substrate for a ligand of apparent molecular weight of about 35kD and which may bean enzyme. As mentioned elsewhere herein, drug screens based on inhibition of binding between the PSMGFR and this ligand or the ligand in a crude cell lysate can identify compounds that inhibit the action of this enzyme.

**Table 3. Cell lines were purchased from the ATCC (American Type Culture Collection, Manasses, VA) and are all breast carcinoma cell lines. Some lines have been shown to express or over express the tumor marker receptor MUC1, Her2/neu or the oncogenic enzyme cathepsin K.**

| Cell line | Gel Result Co-elutes with PSMGFR peptide | Color change assay - yes, turned blue | Expression of species in cell line | Common name | ATCC name |
|---|---|---|---|---|---|
| 1. | +++ | ++++ | Expresses MUC1 | T-47D | HTB-133 |
| 2. | + | - | ND on MUC1 over expresses HER2/neu | UACC-893 | CRL-1902 |
| 3. | +++ | ++++ | Overexpresses MUC1 | ZR-75-1 | CRL-1500 |
| 4. | ++ | + | Express MUC1 over express cathepsin K | ZR-75-30 | CRL-1504 |

**Table 4**

| Cell line | Growth Media |
|---|---|
| HTB-133 | RPMI 1640 media, purchased from Mediatech supplemented with 1 mM sodium pyruvate, 10% FBS, 4.5 g/L glucose and 1.5 g/L sodium bicarbonate, with 2 IU bovine insulin per mL. |
| CRL-1902 | Liebovitz L-15 media (Sigma), supplemented with 10% FBS |
| CRL-1500 | RPMI 1640 media from Mediatech supplemented with 1 mM sodium pyruvate, 10% FBS, 4.5 g/L glucose and 1.5 g/L sodium bicarbonate |
| CRL-1504 | RPMI 1640 media from Mediatech supplemented with 1 mM sodium pyruvate, 10% FBS |

### Example 3: Demonstration that the Ligand That Interacts with MUC1 Cancer Cells is a Multimer

In this example, it is demonstrated that a ligand produced by MUC1 cancer cells that triggers cell proliferation in these cells is a multimer.

Protein bands at 17 kD, 23 kD, and 35 kD were excised from the gels described above in Example 2 of and submitted for peptide analysis. These gel bands purportedly contained ligands to the MGFR region of the MUC1 receptor. Recall that the 17 kD and 23 kD species bound to the MGFR peptide in the presence of the protease inhibitor, PMSF, while the 35 kD species bound when PMSF was not added to the cell lysate mixture.

The following peptide analysis was performed. Samples derived from the gel slices were proteolytically digested. Fragments were then separated by microcapillary HPLC which was directly coupled to a nano-electrospray ionization source of an ion trap mass spectrometer. MS/MS spectra was obtained on-line. These fragmentation spectra were then correlated to known sequences using the SEQUEST® algorithm in conjunction with other algorithms. Results were then manually reviewed to confirm consensus with sequences of known proteins.

Peptide sequences contained within both the 17 kD and the 23 kD bands (PMSF added to lysate) corresponded to a protein known as Metastasis Inhibition Factor NM23, which has been implicated in both the promotion and inhibition of metastasis of human cancers. Whether the role of NM23 is a tumor supressor or promoter may depend on the type of cancer. In ovarian, colon and neuroblastoma tumors, NM23 overexpression has been linked to a more malignant phenotype (Schneider J, Romero H, Ruiz R, Centeno MM, Rodriguez-Escudero FJ, "NM23 expression in advanced and borderline ovarian carcinoma", Anticancer Res, 1996; 16(3A): 1197-202). However, breast cancer studies indicate that reduced expression of NM23 correlates with poor prognosis (Mao H, Liu H, Fu X, Fang Z, Abrams J, Worsham MJ, "Loss of nm23 expression predicts distal metastases and poorer survival for breast cancer", Int J Oncol 2001 Mar;18(3):587-91).

The sequences that were identified from the protein gel band described in Figures 9 and 10 and that are derived from a protein implicated in many cancers called Metastasis Inhibition Factor NM23 are shown below in Table 5. NM23 exists as a hexamer and may recognize an unmodified form of the MGFR portion of the MUC1 receptor.

Peptide sequences that were identified from the 35 kD gel band (PMSF NOT added to lysate) corresponded to more than one protein species, including 14-3-3, which is a signaling protein implicated in many cancers, and cathepsin D, which is a protease and is also implicated in tumor progression. 14-3-3 exists as a dimer and can simultaneously bind to two, identical phospho-serine peptides. This would dimerize the MGFR portion of the MUC1 receptor to trigger cell proliferation, which is consistent with the mechanism presented herein. Cathepsin D is a protease and may be involved in the cleavage of the MUC1 receptor.

The identity of these ligands is consistent with the MUC1-dependent cell proliferation mechanism that is disclosed herein, i.e., a ligand that dimerizes the MGFR portion of the MUC1 receptor triggers cell proliferation and cleavage of only a portion of the MUC1 extracellular domain exposes the functional part of the receptor which is defined by most or all of the nat-PSMGFR sequence (SEQ ID NO: 36) given in Table 1.

Consistent with methods disclosed herein, a therapeutic strategy is to identify compounds that either interrupt the interaction of one of the ligands with the MGFR portion of the MUC1 receptor, or to identify compounds that bind to and block the action of the ligand(s).

**Table 5**

| 17 kD species identified herein from gel band | |
|---|---|
| 1) Metastasis Inhibition Factor NM23 | gi: 127982 |
| TFIAIKPDGVQR | (SEQ ID NO: 14) |
| VM*LGETNPADSKPGTIR | (SEQ ID NO: 15) |
| VMLGETNPADSKPGTIR | (SEQ ID NO: 16) |
| NIIHGSDSVK | (SEQ ID NO: 17) |
| GLVGEIIKR | (SEQ ID NO: 18) |
| GLVGEIIK | (SEQ ID NO: 19) |
| | |

| 23 kD species identified herein from gel band | |
|---|---|
| 1) Metastasis Inhibition Factor NM23 | gi: 127982 |
| TFIAIKPDGVQR | (SEQ ID NO: 14) |
| YM*HSGPVVAM*VWEGLNVVK | (SEQ ID NO: 20) |
| | |

| 35 kD identified herein from gel band | |
|---|---|
| 1) 14-3-3 epsilon | gi: 5803225 |
| AAFDDAIAELDTLSEESYK | (SEQ ID NO: 21) |
| AASDIAM*TELPPTHPIR | (SEQ ID NO: 22) |
| YLAEFATGNDR | (SEQ ID NO: 23) |
| DSTLIMQLLR | (SEQ ID NO: 24) |
| YDEMVESMK | (SEQ ID NO: 25) |
| VAGM*DVELTVEER | (SEQ ID NO: 26) |
| HLIPAANTGESK | (SEQ ID NO: 27) |
| | |

| 2) cathepsin D | gi:4503143 |
|---|---|
| DPDAQPGGELM*LGGTDSK | (SEQ ID NO: 28) |
| DPDAQPGGELMLGGTDSK | (SEQ ID NO: 29) |
| ISVNNVLPVFDNLM*QQK | (SEQ ID NO: 30) |
| ISVNNVLPVFDNLMQQK | (SEQ ID NO: 31) |
| QPGITFIAAK | (SEQ ID NO: 32) |
| | |

| 3) human annexin V with Proline substitution by Thrionine | gi: 3212603 |
|---|---|
| GLGTDEESILTLLTSR | (SEQ ID NO: 33) |
| DLLDDLKSELTGK | (SEQ ID NO: 34) |
| SEIDLFNIR | (SEQ ID NO: 35) |

### Prophetic Example 4 Involving Screening for Drugs That Affect MUC 1 Cleavage State

The release of the MUC 1 IBR can be correlated to the progression of cancer. The following is a description of a whole cell assay that identifies drug candidates that affect cleavage state of these receptors. The screen also identifies drug candidates that directly or indirectly modulate any step, including but not limited to enzyme cleavage, receptor production, expression, stability, transport or secretion, that ultimately results in a reduction of the self-aggregating portion of the receptor being shed and released from the cell.

Tumor derived cells expressing a cell surface receptor of the type described above, are cultured and treated with a drug candidate. Following some incubation period, a peptide aggregation assay is performed on the solution surrounding the cell. Colloids bearing a binding peptide e.g. an antibody against a constant region of the receptor, remote from the enzyme cleavage site (amino acid 425-479 for MUC1; numbers refer to Andrew Spicer et al., J. Biol. Chem Vol 266 No. 23, 1991 pgs. 15099-15109; these amino acid numbers correspond to numbers 985-1039 of Genbank accession number P15941; PID G547937), are added to the solution. If the shed portion of the receptor contains the self-aggregating portion, the receptors in solution will aggregate and cause the attached colloids to aggregate, causing a visibly detectable change in the solution, for example: color change or the formation of visible aggregates. An inhibition of this visible change indicates an agent that is effective for treating the disease state.

The list of sequences in Table 1 is representative of sequence fragments that are, in certain instances, found within the overall sequence of the full-length peptide, or are fragments or variants thereof. Any set of, for example, at least 10 contiguous amino acids within any of the sequence fragments of Table 1 may be sufficient to identify the cognate binding motif. The list of sequences of Table 1 is meant to embrace each single sequence and when mentioning fragment size, it is intended that a range embrace the smallest fragment mentioned to the full-length of the sequence (less one amino acid so that it is a fragment), each and every fragment length intended as if specifically enumerated. Thus, if a fragment could be between 10 and 15 in length, it is explicitly meant to mean 10, 11, 12, 13, 14, or 15 in length.

With reference to Table 1, the receptor can be cleaved at a number of different sites to generate peptide fragments with alternative beginnings and endings. For these fragments of Table 1 any stretch of, for example, 8 to 10 contiguous amino acids, either upstream or downstream, may be enough to identify the particular fragment that is the binding entity referred to herein.

### Example 5. Protocols for western blot analysis

### Cell Culture:

All cell lines were obtained from ATCC (American Type Culture Collection) and were cultured according to ATCC recommendations accompanying cell lines. Cells used include [Applicant designation (ATCC No.)]: T-47D (HTB-133), 1500 (CRL-1500), 1504 (CRL-1504), HeLa (CCL-2), HEK-293 (CRL-1705), BT-474 (HTB-20), MDA-MB-453 (HTB-131).

### Lysate Preparation:

For cell lysate preparation, healthy cells were plated on 100mm culture treated dishes and incubated until cells were approximately 80-90% confluent. Media was then removed and monolayers were washed twice with 5 ml cold PBS. Any remaining PBS was removed thoroughly. Cells were lysed by applying 1 ml of cold High Salt RIPA lysis buffer [400mM NaCl, 50mM Tris pH 8.0, 1% NP-40, 0.1% SDS, 0.5% Sodium Deoxycholate, 1x Protease Inhibitor Cocktail (Roche Applied Sciences; Indianapolis, IN)] to the monolayer and incubating on ice for 5 minutes with occasional agitation. Cells were scraped off and lysate collected in 1.5 ml eppendorf tubes. Lysates were centrifuged at 10,000 rpm and clear supernatants were removed, placed in new tubes, and stored at -20°C until use.

### Protein Concentrations:

Protein concentrations were obtained using the Pierce BCA Protein Assay (Rockford, IL). The manufacturer's protocol was followed. Absorbances were read using a U-2010 UV/Vis-Spectrophotometer from Hitachi (Randolph, MA). Data was plotted and curve-fitted with Microsoft Excel.

### Deglycosylations:

Samples were deglycosylated using the Enzymatic Protein Deglycosylation Kit from Sigma (St. Louis, MO), which contains O-glycosidase, PNGase-F, and α-Neuraminidase enzymes, along with reaction and denaturing buffers. Each deglycosylation was performed using 100ug total protein from lysates, and enzymes and buffers were added per manufacturer's protocol accompanying the kit.

### SDS-PAGE:

SDS-PAGE electrophoresis was employed to separate lysate proteins. Cell lysates were thawed and samples were prepared with 50µg of total protein diluted in appropriate SDS-loading buffer to a final volume of 60µl. 15% polyacrylamide gels were used in order to separate the lower molecular weight bands for cleaved MUC1 portions. Gels were electrophoresed in the BioRad Mini-Protean 3 (Hercules, CA).

### PVDF Transfer:

After electrophoresis was completed, gels were prepared for semi-dry transfer to Immobilon-P PVDF membranes from Millipore (Bedford, MA) using the Trans-Blot SD Semi-Dry Transfer Cell from BioRad (Hercules, CA). Gels, PVDF membranes, and blotting paper (BioRad; Hercules, CA) were equilibrated in Tris/Glycine transfer buffer (25mM Tris, 192mM Glycine, 20% Methanol) for 15 minutes. Sandwich was prepared on apparatus as described by manufacturer. Electrophoretic transfer was performed at 25V for 45 minutes.

### Western Blotting:

Membranes were removed and placed immediately in 25 ml Blotto (PBS, 0.05% Tween-20, 5% Milk) and incubated with gentle agitation for 2 hours. Blotto was removed and replaced with 25 ml primary antibody solution [1:1000 dilution of an α-PSMGFR antibody or 1:200 dilution of VU-4H5 antibody (Santa Cruz Biotechnologies; Santa Cruz, CA), in Blotto] and incubated overnight at 4°C. The solution was then discarded and the membrane washed 5 times for 10 minutes each in PBS-T (PBS, 0.05% Tween-20). Membranes were then incubated in secondary antibody solution [1:20000 dilution of HRP(horseradish peroxidase)-conjugated Goat-α-Rabbit IgG antibody or HRP-conjugated Rabbit-α-Mouse IgG antibody (Jackson Immunoresearch; West Grove, PA) in Blotto] for 1 hour at room temperature. Solution was discarded and the membrane was washed 5 times for 10 minutes each in PBS-T. The membrane was then placed in a 1:1 mixture of Immun-Star HRP Luminol/Enhancer and Peroxide Buffer from BioRad Laboratories (Hercules, CA) for 5 minutes. Substrate was removed and membrane placed in saran wrap and exposed to film and developed in Kodak X-OMAT.

### Example 6 - Transfectants: The construction of six mammalian expression plasmids encoding six different lengths of the Muc1 receptor

### pMuc1-Full, Encoding Full-length MUC1 Receptor

The pMuc1-Full construct contains the complete cDNA for MUC1 and encodes the full length Muc1 protein (Figure 38 and SEQ ID NO: 10). The pMuc1-Full plasmid was put together from two separate plasmids containing different parts of the MUC1 cDNA. The amino-terminus of MUC1 was acquired from EST0039670 obtained from the Genome Research Center and the Center for Functional Analysis of Human Genome (GRC/CFAHG) Korea Research Institute of Bioscience and Biotechnology (Taejeon, Korea). The EST0039670 plasmid contained a cDNA starting at the amino-terminus of the MUC1 open reading frame to about 800 base pairs into the tandem repeats segment of MUC1. The carboxy-terminus of the MUC1 cDNA was obtained from Integrated Molecular Analysis of Genomes and their Expression (IMAGE) clone number 2428103 obtained from American Type Culture Collection (ATCC) (Manassas, VA). This plasmid contains the remaining 1300 base pairs of the tandem repeats through the carboxy-terminus of MUC1. The full length Muc1 construct (Muc1-Full) was generated by restriction digesting the plasmid containing IMAGE 2428103 with SalI and subcloning into the XhoI digested EST0039670. The resulting sublcone, pESTMuc1, was confirmed by restriction digest and agarose gel electrophoresis and was found to have the expected size bands for the correctly constructed plasmid. The pESTMuc1 plasmid was used for subcloning into the mammalian expression vector pIRES2-GFP.

The expression vector used for all of the MUC1 constructs was pIRES2-EGFP from Becton Dickinson Clontech (Palo Alto, CA). This vector will express MUC1 from a cytomegalo virus (CMV) early promoter. This vector also contains an internal ribosome entry site (IRES) for expression of the green fluorescent protein (GFP) from the same message as MUC1.

The full length Muc1 containing plasmid, pESTMuc1, was digested with EcoRI and XhoI and the DNA fragment containing MUC1 was gel purified. The expression vector, pIRES2-GFP, was digested with EcoRI and SalI and gel purified. The two pieces of DNA were ligated to yield pMuc1-Full, containing the full length MUC1 protein encoded in the mammalian expression vector pIRES2-EGFP. Subclones of the mammalian expression vector with the correct insert were selected for sequencing. The sequence confirmed the proper construction of the desired plasmid.

All sequencing was carried out by GeneWiz Inc. (North Brunswick, NJ) Sequencing was done by PCR sequencing. All results were confirmed by comparison of sequences to the National Center for Biotechnology (NCBI) database.

### pMuc1-Rep, Encoding the Rep isoform

The pMuc1-Rep (Table 2: SEQ ID NO: 46) construct encodes a Muc1 protein which has been amino terminally deleted (Figure 1- Table 1: SEQ ID NO: 41). The Rep isoform is missing the amino terminus and only contains about half of the terminal repeats of MUC1. First pSP-Rep was generated by subcloning the PCR amplified signal peptide for MUC1 into the EST plasmid (IMAGE 2428103). The signal peptide of MUC1 was subcloned at the amino-terminus to ensure expression of the protein in the plasma membrane. PCR amplification of the normal MUC1 signal peptide from IMAGE clone number 4695020 was carried out using the primers in Table 6. The PCR product was digested with EcoRI and XhoI and subcloned into the EcoRI and SalI digested EST plasmid (IMAGE 2428103). The proper construction of the resulting subclone, pSP-Rep, was confirmed by digestion and agarose gel electrophoresis. Then the pSP-Rep plasmid and pIRES2-GFP were digested with EcoRI and BamHI and ligated together. The correct construction of pMucl- Rep was confirmed by restriction digestion followed by gel electrophoresis. Sequencing also showed that the pMuc1-Rep plasmid was made correctly.

### pMuc1-UR, Encoding the UR isoform; pMuc1-CM, Encoding the CM isoform; and pMuc1-PSMGFRTC, Encodind the nat-PSMGFRTC isoform:

These three constructs encode various amino terminal deletion isoforms of MUC1, carboxy terminal to the tandem repeats (see Figure 1. pMuc1-UR (Table 2; SEQ ID NO: 44) encodes Table 1: SEQ ID NO: 39; pMuc1-CM (Table 2: SEQ ID NO: 43) encodes Table 1: SEQ ID NO: 38; and pMuc1-PMMGFRTC (Table 2: SEQ ID NO: 42) encodes Table 1 SEQ NO: 37)). pMuc1-UR encodes an isoform that contains from amino acid 981 to 1255 of MUC1. pMucl-CM encodes an isoform that contains from amino acids 1085 to 1255 of MUC1 and encodes a peptide of 168 amino acids. pMuc1-PSMGFRTC encodes a peptide from amino acid 1110 to amino acid 1255 of Muc1 and will be 143 amino acids long. All three of these plasmids were constructed following the same procedure. First the signal peptide of MUC1 was amplified by PCR from IMAGE clone number 4695020 using primers that each contained either a restriction site for EcoRI or EagI. Then the carboxy-terminal portions of the constructs were amplified by PCR with primers that contained a EagI or BamHI site. The primers used for PCR are listed in Table 6. The PCR products were digested with EagI and then ligated. The ligation was amplified again by PCR using the EcoRI and BamHI containing primers used previously. This amplified the joined PCR products. The resulting DNA fragment was digested with EcoRI and BamHI and subcloned into pIRES2-EGFP that had been similarly digested. The size of the desired PCR products and plasmids were confirmed by agarose gel electrophoresis. Sequencing established that the correct plasmids had been created.

### pMuc1-Y, Encoding the Y isoform:

The pMuc1-Y plasmid encodes an alternately spliced form of MUC1 (see Figure 1. pMuc1-UR (Table 2; SEQ ID NO: 45) encodes Table 1: SEQ ID NO: 40). The cDNA of the Muc1-Y was obtained from IMAGE clone number 4695020. When this clone was sequenced it was found to contain an extra 9 amino acids. These were deleted by PCR. The front part of the Muc1-Y cDNA was amplified with a primer containing the restriction site EcoRI and a primer containing the AlwNI restriction site. The terminal part of Muc1-Y was amplified with a primer containing the AlwNI restriction site and a primer containing the restriction site BamHI. After AlwNI digestion of both PCR products the two fragments were ligated. The ligation was amplified again by PCR using the EcoRI and BamHI containing primers used previously. This amplified the joined PCR products. The resulting DNA fragment was digested with EcoRI and BamHI and subcloned into pIRES2-EGFP that had been similarly digested. The size of the desired PCR products and plasmids were confirmed by agarose gel electrophoresis. Sequencing established that the correct plasmid with the 9 amino acid deletion had been created.

**Table 6. Primers for PCR**

| | |
|---|---|
| nTerMuc1EcoRI | 5'-GGGAATTCATGACACCGGGCACCCAGTC-3' (SEQ ID NO: 49) |
| Muc1-CtermSP-XhoI | 5'-GGTCTCGAGAACAACTGTAAGCACTGT-3' (SEQ ID NO: 50) |
| Muc1-CtermSP-EagI | 5'-GGTCGGCCGTAACAACTGTAAGGACTGT-3' (SEQ ID NO: 51) |
| Muc1-UR-EagI | 5'-GCACGGCCGCTACCACAACCCCAGCCAG-3' (SEQ ID NO: 52) |
| Muc1-CM-EagI | 5'-GCACGGCCGGTTTTCTGGGCCTCTCCAA-3' (SEQ ID NO: 53) |
| Muc1-PSMGFRTC-EagI | 5'-GCACGGCCGGTACCATCAATGTCCACGAC-3' (SEQ ID NO: 54) |
| cTerMuc1BamHI | 5'-GGGGGATCCTACAAGTTGGCAGAAGCGG-3' (SEQ ID NO: 55) |
| Muc1-YMid-For | 5'-TGCTCCTCACAGTGCTTACAGGTTCTGGTCATGCAAGCT-3' (SEQ ID NO: 56) |
| Muc1-YRev3 | 5'-GAGCTTGCATGACCAGAACCTGTAACAACTGT-3' (SEQ ID NO: 57) |

### Methods-DNA Manipulations:

Polymerase chain reaction (PCR) was preformed using a MiniCylcer from MJ Research (Watertown, MA). The following steps were used for PCR. First step 94°C for 2 minutes, Second denaturation step 94°C for 30 seconds, Third step annealing at 55°C for 30 seconds, Fourth step of extension at 68°C for one minute, fifth step 34 cycles of steps 2 through 4, sixth step a 68°C for 5 minutes and hold at 4°C. The Platinum *Pfx* DNA Polymerase from Invitrogen (Carlsbad,CA) was used for amplification. The PCR reaction contained 2ng plasmid DNA, 1X *Pfx* amplification buffer, 1mM MgSO₄, 0.3µl of each primer, 1.25 Units of *Pfx* polymerase, and 0.3µM of each dNTP in a 50µl reaction volume. PCR products were purified away from primers and buffers using Qiaquick PCR clean up kit form Qiagen. PCR products were treated this way before restriction digestion.

All DNA restriction enzymes were purchased from New England BioLabs (Beverly, MA). Restriction digests were carried out as recommended by the manufacuter at 37°C in supplied reaction buffers.To purify DNA fragments, bands on agarose gels were visualized with ethidium bromide and cut out of the gel. The Qiaquick gel purifaction columns from Qiagen (Valencia, CA) were then used to purify the fragment following the recommended protocol of the manufacturer.

Small quantities of plasmid DNA were prepared by alkaline lysis as outlined in Ausubel (ref). Large quantities of DNA were prepared using Qiagen Maxi Prep columns. Other than adding a addition step of phenol chloroform extraction after the DNA was isolated the protocol was carried out as per the instructions recommended by the company. DNA concentrations were determined by measuring the optical density of diluted sample at 260nm in a Hitachi 3000 spectrophotometer. DNA fragments were ligated using the Rapid DNA Ligation Kit supplied by Roche (Indianapolis, IN). Ligated DNA was transformed into the chemically compotent *E. coli* strain JM101 by heat shock.
DNA sequencing was done by PCR sequencing and was carried out by GeneWiz Inc. (North Brunswick, NJ).

### Example 7: Transfection of HEK293 cells enabled expression of MUC1 isoforms on the surface of cells:

The goal was to transfect HEK293 cells with various isoforms of the MUC1 protein and have the protein expressed at the cell surface. HBK293 cells were transfected with MUC1 isoforms and demonstrated the expression of MUC1 isoforms on the surface of the transfected cells.

### Transfection Procedure:

Human embryonic kidney (HEK) 293 cells were plated to yield 90% confluency. Lipofectamine 2000 from Invitrogen (Carlsbad, CA) was use to transfect the cell. The protocol from the manufacturer was followed. Both the DNA and Lipofectamine were diluted in serum and antibiotic free media. After 5 minutes the DNA and Lipofectamine were mixed and incubated for 30 minutes at room temperature. Cells were washed once with sterile 1x PBS and then the transfection mixture was added to the cells. Cells were incubated with the DNA:Lipofectamine complexes for 4-6 hours before the media was changed to media containing 10% serum. These cells were then assayed 24 or 48 hours later for expression of MUC1 isoforms. Stable cell lines were selected 48 hrs after transfection with 600µg/ml Genetimicin (G418) from Invitrogen for 10 days.

### Example 8: Polyclonal anti-PSMGFR Antibody Production:

The sequence of the peptide used for immunication was GTINVHDVETQFNQYKTEAASPYNLTISDVSVSDVPFPFSAQSGA (var-PSMGFR SEQ ID NO: 7). Two rabbits were immunized using the Polyquick^{™} method, a proprietary commercially available technology. After four weeks bleeds were evaluated for PSMGFR specific antibodies. The rabbits received an additional boost of antigen two weeks before harvesting the antibody. The antibody was affinity purified using the peptide conjugated to a column support.

### Example 9: Production of monovalent antibody fragments:

Antibody fragmentation of the polyclonal antibody as produced in Example 8 was performed using papain digestion by Maine Biotechnology Services Inc (Portand, ME). Fragmented antibody was purified from uncleaved antobody and Fc fragments. The cleavage reaction and purification was evaluated on SDS PAGE.

### Example 10: Evaluation of transfectants for MUC1 isoform expression:

To establish that the MUC1 isoform transfectants expressed the protein on the surface of the cell, flow cytometry was carried out on the transfected cells. The indirect staining protocol of the cells follows breifly below. One million cells were stained with the 1mg of primary anti-PSMGFR antibody. The cells were then washed with 1x PBS. The cells were then stained with a Phycoerythrin (PE)-labeled-Fab-fragment anti-rabbit secondary antibody from Jackson Laboratories. The cells were washed and stained with PI. Flow cytometery was performed on the Becton Dickenson (Palo Alto, CA) FACS Calibur machine. Cell population were gated for live cells by forward and side scatter and propidium iodide (PI) negative staining. Cells were evaluated for levels of GFP, indicating a transfectant, and for PE, indicating MUC1 staining. Transfectants stained for PSMGFR over the level of the vector control (data not shown). It was observed that the MUC1-PSMGFRTC and MUC1-Y constructs stained to higher levels compared to the other transfectants. A large portion of the other transfectants are GFP-low, possibly indicating low transfection levels.

We used fluorescent microscopy to look at the localization of MUC1 isoforms on the surface of the transfected cells. The procedure for staining the cells was as follows. Cells were grown on sterile cover slips. The cells were washed with 1x PBS. The cells were fixed with 4% paraformaldehyde in 1x PBS. The cover slips were then washed with 1x PBS. anti-PSMGFR antibodies were added to the cover slips at 2ug/ml. The cover slips were washed again in 1x PBS. Anti-rabbit tetramethyl rhodamine isothiocyanate (TRITC) labeled secondary antibody from Zymed was added to the cover slip after which they were washed in lxPBS. Nuclei were stained with 4',6-diamidino-2-phenylindole dihydrochloride hydrate (DAPI) from Sigma (St.Louis, MO). The cover slips were placed on slides using Mounting Media. The cover slips were sealed with nail polish. The slides were viewed using the 60X objective of a Olympus IX70 microscope equipped with Delta Vision® microscope system Applied Precision (Issaquah, WA). Pictures were taken using SoftwoRx® software from Applied Precision. The resulting pattern of staining seen with the anti-PSMGFR antibody was characteristic of that for a protein localized to the surface of the cell.

### Example 11- The stimulatory/inhibitory effects of bi- versus mono-valent anti-PSMGFR antibodies on cell proliferation:

### Reagents:

* Serial dilutions of the ANTI-PSMGFR antibody produced as described in Examples 8 and 9 in serum free RPMI media (1X, 1/10 of 1X, 1/50,1/250, 1/1000)
* 60-70% confluent flask with the desired cells
* 10% and 0.1% cell-specific media
* Trypsin
* 96-well plate

### Method:

1. Place 100ul of media in the peripheral wells of a 96 well plate. Plate 6000 cells per well (in 100 ul volume of growth media containing 10 % serum) into the inside wells.
2. Next day, change media to 0.1% serum growth media and incubate over night (12-24 hours), except for BT474 cells where the media is changed to that containing 2.5% serum.
3. Next Add antibody (lul per well) to the desired well, gently mix and put back in incubator. Antibodies were added every 24 hrs. For the tumor cell lines 1500, 1504 and BT474, antibody was added 5 times and the cells counted 24 hr after the last antibody addition. For the tumor cell line T47D antibody was added two or three times. For the K293 cell stable transfectants, produced as described above in Example 7 antibody was added two times. For the competition experiments between the monovalent antibody fragments and bivalent antibodies, the monovalent antibody fragment was added between 10-15 minutes prior to the addition of the bivalent antibody.
4. Performed the desired assay (BrdU, individual cell counting) to count the cells.
5. Percentage growth was calculated using the following equation: $% growth = 100 \left\{Final cell count-Starting cell count\right\} / Starting cell count$
6. Normalized growth was calculated as follows: $Normalized % growth = 100 \left\{Final cell count with/without antibody\right\} / Final cell count without antibody .$

Human breast adenocarcinoma CRL-1500 cells were obtained form the American Type Culture Collection (ATCC). Cells were plated in T75 vented flask in RPMI 1640 containing 10% fetal calf serum, Pen/Sterp, 1mM sodium pyruvate, 0.5% glucose, and 0.15% sodium bicarbonate. In addition, cells were passaged 10 times prior to experiments. When ready, cells were plated on a 96-well plate (6000 cells per well in 100 ul media) in in RPMI 1640 containing 10% fetal calf serum, Pen/Sterp, 1mM sodium pyruvate, 0.5% glucose, and 0.15% sodium bicarbonate and incubated for 24 h at 5% CO2 and 37C. The following day, growth media was replaced with RPMI media containing 0.1% fetal calf serum, Pen/Sterp, 1mM sodium pyruvate, 0.5% glucose, and 0.15% sodium bicarbonate over night at 5% CO2 and 37 degree C. To test the effect MUC1 receptor dimerization on 1500 cell growth, cells were incubated with either different concentrations of bivalent affinity purified anti-PSMFGR polyclonal antibody, different concentrations of monovalent affinity purified anti-PSMFGR polyclonal antibody fragment or both together. Antibodies/fragments were added to the cell five times every 24 hours. To demonstrate the specificity of the anti-PSMFGR antibody, a control experiment was performed using an irrelevant antibody (anti-sterptavidin polyclonal Antibody). Cells were harvested 24 hours after the last antibody addition with 50 ul trypsin, and the number of cells per well was determined using a hemacytometer (3 wells counted per condition).

The same protocol as described above for CRL-1500 cells was used for CRL-1504 cells. For BT-474 (HTB-20) cells, the protocol used was the same as that for the CRL-1500 cells with two exceptions: (1) The cells were cultured in ATCC Hybricare (Cat # 46-X) media supplemented with 10% fetal bovine serum and penicillin/streptomycin, and (2) during the experiment, the cells were cultured in the presence of 2.5 % serum or 5.0 % serum containing media as specified in the individual experiment.

For the T47-D (HTB-133) cells, the media used was RPMI 1640 supplemented with 10% fetal bovine serum, 1mM sodium pyruvate, penicillin/streptomycin, 1.25 g glucose/500 ml media, 0.2 units/ml bovine insulin and 1.5 g/liter sodium bicarbonate. For the antibody stimulatory/inhibitory experiments, no insulin was added, and the experiments were carried out in media containing 0.1 % fetal bovine serum.

HeLa (CCL-2) cells were cultured in MEM Earle's BSS media (ATCC #30-2003) supplemented with penicillin/streptomycin and 10 % fetal bovine serum. The antibody experiments were done as described for the CRL-1500 cells.
HEK293 (CRL-1705) cells were cultured in DMEM (BioWhittaker #BW12-640F) media supplemented with penicillin/streptomycin and 10% fetal bovine serum. The antibody experiments were carried out as described for the CRL-1500 cells.

HEK293 (CRL-1705) cell transfectants were cultured in DMEM (BioWhittaker #BW12-640F media supplemented with penicillin/streptomycin, 10% fetal bovine serum and 600ug/ml geneticin (GibcoBRL #11101-011). The selecting drug geniticin was not added to the media during the experiment. Antibody stimulatory/inhibitory experiments were carried out in the presence of 0.1% serum containing media and antibody/antibodies were added two times at 24 hour intervals.

Percent cell growth was calculated using the following equation: $% growth = 100 \times \left\{Final cell count-Starting cell count\right\} / Starting cell count$ Normalized percent cell growth was calculated as follows: $Normalized % growth = 100 \times \left\{Final cell count with or without antibody\right\} / Final cell count without antibody .$

### Example 12 - Measurement of ERK2 phosphorylation state in breast tumor cells as a determinant of MGFR dimerization

Human breast adenocarcinoma CRL-1500 cells were obtained form the American Type Culture Collection (ATCC). Cells were plated in T75 vented flask in RPMI 1640 containing 10% fetal calf serum, Pen/Sterp, 1mM sodium pyruvate, 0.5% glucose, and 0.15% sodium bicarbonate. In addition, cells were passage 10 times prior to experiments. When ready, cells were seeded at 1 x 10⁶ per 60mm plate in RPMI 1640 containing 10% fetal calf serum, Pen/Sterp, 1mM sodium pyruvate, 0.5% glucose, 0.15% sodium bicarbonate and insulin (10 ug/ml). The following day, the cells at 70% confluence were washed carefully in order not to disturb them (1x) with serum-free RPMI media, and incubated in 2 ml serum-free RPMI media at 5% CO₂ and 37C over night. The following day, cells were stimulated with either 5 µl of affinity purified divalent anti-PSMGFR polyclonal antibody, Monovalent anti-PSMGFR alone (10 µl) or both together. Untreated cells were used as control. After activation, cells were immediately washed (2x) with ice-cold PBS and lysed using buffer (1% Triton X-100, 10% glycerol, 20 mM HEPES, pH 72, 100 mM NaCl, 1 mM phenylmethylsulfonyl fluoride, 10 µg/ml aprotinin, 10 µg/ml leupeptin, and 1 mM Na₃VO₄. The cell lysate was incubated on a rotating plate for 15 minute and then centrifuged for 10 minutes (10g) to remove the detergent-insoluble material. Equal amounts of protein (100 µg per lane) were mixed with SDS-PAGE sample buffer, boiled for 5 minutes, separated on 10% SDS-polyacrylamide gels, and then transferred to polyvinylidene difluoride membrane. The membrane was then blocked for 1 h at room temperature in Phosphate-buffered saline (PBS) containing 5% nonfat dry milk. The membrane was the incubated over night at 4 degrees with either anti-ERK2 or anti-ppERK1/2 antibody (Cell Signaling Technology Inc., Beverly, MA, USA) (diluted 1:1,000 in PBS with 0.5% milk). Immunoblots were washed with PBS once for 20 minutes and twice for 5 minutes, incubated with the secondary antibody conjugated with horseradish peroxidase (diluted 1:20,000) for 1 hour, washed with PBS once for 20 minutes and twice for 5 minutes and visualized by chemiluminescence using enhanced chemiluminescence reagents (BioRad).

### Example 13 - Colloid Assay for testing the Monovalent - Bivalent antibody competition

Colloids were prepared as described above with 25 uM NTA thiol. Next, Histidine tagged PSMGFR peptide (e.g. His-var-PSMGFR SEQ ID NO:2) or control RGD peptide were bound to the colloid.

In the wells of the microtiter plate, 55 ul phosphate buffer was added followed by 5 ul of 100 uM BSA (bovine serum albumin).

Antibody/antibodies (minimum of 10ul of undiluted stock, roughly 1 ug/ul) were then added to the wells and the contents mixed by pipetting.

30 ul of either the control RGD colloid or His-PSMGFR colloid was added next followed by mixing. The antibody-peptide interaction was allowed to proceed for 3 - 4 hrs.

Wells were scored by measuring absorbance at 650 nm.

In addition to the diagnostics and screening assays disclosed herein, also provided are therapeutic methods for the treatment and prevention of cancer and related products. For instance, one case relates to a method for treating a subject having a cancer or at risk of developing cancer by administering to the subject an agent that reduces cleavage of a cell surface receptor IBR from a cell surface receptor.

Those skilled in the art would readily appreciate that all parameters listed herein are meant to be exemplary and that actual parameters will depend upon the specific application for which the methods and apparatus are used. It is, therefore, to be understood that the foregoing embodiments are presented by way of example only and that, within the scope of the appended claims, the invention may be practiced otherwise than as specifically described. Specifically, those of ordinary skill in the art will recognize, or be able to ascertain using no more than routine experimentation, many equivalents to the specific embodiments of the invention described herein. Such equivalents are intended to be encompassed by the following claims.

Several methods are disclosed herein of administering a subject with a compound for prevention or treatment of a particular condition. It is to be understood that in each such instance as disclosed, the disclosure specifically includes, also, the compound for use in the treatment or prevention of that particular condition, as well as use of the compound for the manufacture of a medicament for the treatment or prevention of that particular condition.

In the claims, all transitional phrases such as "comprising", "including", "carrying", "having", "containing", "involving", and the like are to be understood to be open-ended, i.e. to mean including but not limited to. Only the transitional phrases "consisting of" and "consisting essentially of", respectively, shall be closed or semi-closed transitional phrases.

## Claims

1. A method comprising:
transfecting or transforming a host cell *in vitro* with an expression vector encoding an amino acid sequence of SEQ ID NO.37, or a variant of amino acid sequence of SEQ ID NO. 37 having amino acid substitutions up to 15 or any amino acid additions or deletions up to 15 at its N-terminus or C-terminus, being free of an interchain binding region of the cell surface receptor to the extent necessary to prevent spontaneous binding between them; and
facilitating expression of the peptide by the cell so that the cell expresses the peptide as a transmembrane peptide on the cell surface.

2. A method as in claim 1, wherein the host cell is a cell line.

3. A method as in claim 1, wherein the host cell is a primary cell.

4. A transgenic non-human animal comprising an expression vector encoding an amino acid sequence of SEQ ID NO.37, or a variant of amino acid sequence of SEQ ID NO. 37 having amino acid substitutions up to 15 or any amino acid additions or deletions up to 15 at its N-terminus or C-terminus, being free of an interchain binding region of the cell surface receptor to the extent necessary to prevent spontaneous binding between them.

5. A method of carrying out experiments to test biochemical or physiological effects of diagnostics or therapeutics, using a transgenic non-human animal according to claim 4.

6. A method as in any of claims 1 to 3, wherein the amino acid further comprises a signalling sequence.

7. A method as in any of claims 1 to 3 or 6, wherein the nucleic acid is as set forth in SEQ ID NO.42.

8. A method as in any of claims 1 to 3, 6 or 7, wherein the nucleic acid is operably linked to a promoter.

9. A method as in any of claims 1 to 3 or 6 to 8, wherein the expression vector encodes an amino acid sequence of SEQ ID NO.37 or a variant of amino acid SEQ ID NO.37 having amino acid additions or deletions up to 10 at its N-terminus, being free of an interchain binding region of the cell surface receptor to the extent necessary to prevent spontaneous binding between them.

10. The method according to claim 5, wherein the diagnostics or therapeutics is on cancer cells **characterized by** aberrant expression of MUC1.

11. The method or use according to claim 6, where the signalling sequence is MUC1 specific N-terminal signalling sequence.

## Patentansprüche

1. Verfahren, umfassend:
Transfizieren oder Transformieren einer Wirtszelle *in vitro* mit einem Expressionsvektor, der eine Aminosäuresequenz von SEQ ID NO: 37 oder eine Variante der Aminosäuresequenz von SEQ ID NO: 37 codiert, die bis zu 15 Aminosäuresubstitutionen oder bis zu 15 beliebige Aminosäureadditionen oder -deletionen an ihrem N-Terminus oder C-Terminus umfasst und keine Zwischenketten-Bindungsregion des Zelloberflächenrezeptors in dem Maße aufweist, das nötig ist, um eine spontane Bindung zwischen ihnen zu verhindern; und
Erleichtern der Expression des Peptids durch die Zelle, so dass die Zelle das Peptid als Transmembranpeptid auf der Zelloberfläche exprimiert.

2. Verfahren nach Anspruch 1, wobei die Wirtszelle eine Zelllinie ist.

3. Verfahren nach Anspruch 1, wobei die Wirtszelle eine primäre Zelle ist.

4. Transgenes nicht-menschliches Tier, umfassend einen Expressionsvektor, der eine Aminosäuresequenz von SEQ ID NO: 37 oder eine Variante der Aminosäuresequenz von SEQ ID NO: 37 codiert, die bis zu 15 Aminosäuresubstitutionen oder bis zu 15 beliebige Aminosäureadditionen oder -deletionen an ihrem N-Terminus oder C-Terminus umfasst und keine Zwischenketten-Bindungsregion des Zelloberflächenrezeptors in dem Maße aufweist, das nötig ist, um eine spontane Bindung zwischen ihnen zu verhindern.

5. Verfahren zur Durchführung von Experimenten, um biochemische oder physiologische Auswirkungen von Diagnostika oder Therapeutika zu testen, unter Verwendung eines transgenen nicht-menschlichen Tiers nach Anspruch 4.

6. Verfahren nach einem der Ansprüche 1 bis 3, wobei die Aminosäure zudem eine Signalsequenz umfasst.

7. Verfahren nach einem der Ansprüche 1 bis 3 oder 6, wobei die Nukleinsäure wie in SEQ ID NO: 42 offenbart ist.

8. Verfahren nach einem der Ansprüche 1 bis 3, 6 oder 7, wobei die Nukleinsäure funktionsfähig mit einem Promotor verknüpft ist.

9. Verfahren nach einem der Ansprüche 1 bis 3 oder 6 bis 8, wobei der Expressionsvektor eine Aminosäuresequenz von SEQ ID NO: 37 oder eine Variante der Aminosäure SEQ ID NO: 37 codiert, die bis zu 10 Aminosäureadditionen oder -deletionen an ihrem N-Terminus umfasst und keine Zwischenketten-Bindungsregion des Zelloberflächenrezeptors in dem Maße aufweist, das nötig ist, um eine spontane Bindung zwischen ihnen zu verhindern.

10. Verfahren nach Anspruch 5, wobei die Diagnostika oder Therapeutika an Krebszellen angewendet werden, die durch aberrante Expression von MUC₁ gekennzeichnet sind.

11. Verfahren oder Verwendung nach Anspruch 6, wobei die Signalsequenz MUC₁-spezifische N-terminale Signalsequenz ist.

## Revendications

1. Procédé comprenant les étapes consistant à :
transfecter ou transformer une cellule hôte *in vitro* avec un vecteur d'expression codant pour une séquence d'acides aminés de SEQ ID n° : 37 ou un variant de la séquence d'acides aminés de SEQ ID n° : 37 ayant jusqu'à 15 substitutions d'acides aminés, ou n'importe quel(s) ajout(s) ou quelle(s) délétion(s) allant jusqu'à 15 acides aminés, à son extrémité N-terminale ou C-terminale, étant dépourvue d'une région de liaison inter-chaînes du récepteur de surface cellulaire dans la mesure où cela est nécessaire à la prévention d'une liaison spontanée entre elles ; et
faciliter une expression du peptide par la cellule, de sorte que la cellule exprime le peptide sous forme de peptide transmembranaire à la surface de la cellule.

2. Procédé selon la revendication 1, dans lequel la cellule hôte est une lignée de cellules.

3. Procédé selon la revendication 1, dans lequel la cellule hôte est une cellule primaire.

4. Animal non humain transgénique comprenant un vecteur d'expression qui code pour une séquence d'acides aminés de SEQ ID n° : 37 ou un variant de la séquence d'acides aminés de SEQ ID n° : 37 ayant jusqu'à 15 substitutions d'acides aminés, ou n'importe quel(s) ajout(s) ou quelle(s) délétion(s) allant jusqu'à 15 acides aminés, à son extrémité N-terminale ou C-terminale, étant dépourvue d'une région de liaison inter-chaînes du récepteur de surface cellulaire dans la mesure où cela est nécessaire à la prévention d'une liaison spontanée entre elles.

5. Procédé de réalisation d'expériences destinées à tester des effets biochimiques ou physiologiques de substances diagnostiques ou thérapeutiques en utilisant un animal non humain transgénique selon la revendication 4.

6. Procédé selon l'une quelconque des revendications 1 à 3, dans lequel l'acide aminé comprend en outre une séquence de signalisation.

7. Procédé selon l'une quelconque des revendications 1 à 3 ou 6, dans lequel l'acide nucléique est tel qu'indiqué dans la SEQ ID n° : 42.

8. Procédé selon l'une quelconque des revendications 1 à 3, 6 ou 7, dans lequel l'acide nucléique est lié, de manière opérationnelle, à un promoteur.

9. Procédé selon l'une quelconque des revendications 1 à 3 ou 6 à 8, dans lequel le vecteur d'expression code pour une séquence d'acides aminés de SEQ ID n° : 37 ou un variant de la SEQ ID n° : 37 d'acides aminés ayant jusqu'à 10 ajouts ou délétions d'acides aminés à son extrémité N-terminale, étant dépourvue d'une région de liaison inter-chaînes du récepteur de surface cellulaire dans la mesure où cela est nécessaire à la prévention d'une liaison spontanée entre elles.

10. Procédé selon la revendication 5, dans lequel la substance diagnostique ou thérapeutique est, sur les cellules cancéreuses, **caractérisée par** une expression aberrante de MUC₁.

11. Procédé selon la revendication 6, où la séquence de signalisation est une séquence de signalisation N-terminale spécifique de MUC₁.


### SEQUENCE LISTING

<110> Minerva Biotechnologies Corporation
<120> Techniques and Compositions for the Diagnosis and Treatment of Cancer (MUC1)
<130> M1015.70089WO00
<140> not yet assigned
   <141> 2004-08-26
<150> US 60/498,260
   <151> 2003-08-26
<160> 66
<170> Patent In version 3.3
<210> 1
   <211> 39
   <212> PRT
   <213> Artificial Sequence
<220>
   <223> Synthetic Peptide
<400> 1
   Gly Thr Ile Asn Val His Asp Val Glu Thr Gln Phe Asn Gln Tyr Lys
   1               5                   10                  15
   Thr Glu Ala Ala Ser Pro Tyr Asn Leu Thr Ile Ser Asp Val Ser Val
               20                  25                  30
   Ser His His His His His His
           35
<210> 2
   <211> 51
   <212> PRT
   <213> Artificial Sequence
<220>
   <223> Synthetic Peptide
<400> 2
   Gly Thr Ile Asn Val His Asp Val Glu Thr Gln Phe Asn Gln Tyr Lys
   1               5                   10                  15
   Thr Glu Ala Ala Ser Pro Tyr Asn Leu Thr Ile Ser Asp Val Ser Val
               20                  25                  30
   Ser Asp Val Pro Phe Pro Phe Ser Ala Gln Ser Gly Ala His His His
           35                  40                  45
   His His His
       50
<210> 3
   <211> 54
   <212> PRT
   <213> Artificial Sequence
<220>
   <223> Synthetic Peptide
<400> 3
   Val Gln Leu Thr Leu Ala Phe Arg Glu Gly Thr Ile Asn Val His Asp
   1               5                   10                  15
   Val Glu Thr Gln Phe Asn Gln Tyr Lys Thr Glu Ala Ala Ser Pro Tyr
               20                  25                  30
   Asn Leu Thr Ile Ser Asp Val Ser Val Ser Asp Val Pro Phe Pro Phe
           35                  40                  45
   His His His His His His
       50
<210> 4
   <211> 31
   <212> PRT
   <213> Artificial Sequence
<220>
   <223> Synthetic Peptide
<400> 4
   His His His His His His Gly Phe Leu Gly Leu Ser Asn Ile Lys Phe
   1               5                   10                  15
   Arg Pro Gly Ser Val Val Val Gln Leu Thr Leu Ala Phe Arg Glu
               20                  25                  30
<210> 5
   <211> 46
   <212> PRT
   <213> Artificial Sequence
<220>
   <223> Synthetic Peptide
<400> 5
   Pro Asp Thr Arg Pro Ala Pro Gly Ser Thr Ala Pro Pro Ala His Gly
   1               5                   10                  15
   Val Thr Ser Ala Pro Asp Thr Arg Pro Ala Pro Gly Ser Thr Ala Pro
               20                  25                  30
   Pro Ala His Gly Val Thr Ser Ala His His His His His His
           35                  40                  45
<210> 6
   <211> 33
   <212> PRT
   <213> Artificial Sequence
<220>
   <223> Synthetic Peptide
<400> 6
   Gly Thr Ile Asn Val His Asp Val Glu Thr Gln Phe Asn Gln Tyr Lys
   1               5                   10                  15
   Thr Glu Ala Ala Ser Pro Tyr Asn Leu Thr Ile Ser Asp Val Ser Val
               20                  25                  30
   Ser
<210> 7
   <211> 45
   <212> PRT
   <213> Artificial Sequence
<220>
   <223> Synthetic Peptide
<400> 7
   Gly Thr Ile Asn Val His Asp Val Glu Thr Gln Phe Asn Gln Tyr Lys
   1               5                   10                  15
   Thr Glu Ala Ala Ser Pro Tyr Asn Leu Thr Ile Ser Asp Val Ser Val
               20                  25                  30
   Ser Asp Val Pro Phe Pro Phe Ser Ala Gln Ser Gly Ala
           35                  40                  45
<210> 8
   <211> 25
   <212> PRT
   <213> Homo sapiens
<400> 8
   Gly Phe Leu Gly Leu Ser Asn Ile Lys Phe Arg Pro Gly Ser Val Val
   1               5                   10                  15
   Val Gln Leu Thr Leu Ala Phe Arg Glu
               20                  25
<210> 9
   <211> 40
   <212> PRT
   <213> Homo sapiens
<400> 9
   Pro Asp Thr Arg Pro Ala Pro Gly Ser Thr Ala Pro Pro Ala His Gly
   1               5                   10                  15
   Val Thr Ser Ala Pro Asp Thr Arg Pro Ala Pro Gly Ser Thr Ala Pro
               20                  25                  30
   Pro Ala His Gly Val Thr Ser Ala
           35                  40
<210> 10
   <211> 1255
   <212> PRT
   <213> Homo sapiens
<400> 10
   Met Thr Pro Gly Thr Gln Ser Pro Phe Phe Leu Leu Leu Leu Leu Thr
   1               5                   10                  15
   Val Leu Thr Val Val Thr Gly Ser Gly His Ala Ser Ser Thr Pro Gly
               20                  25                  30
   Gly Glu Lys Glu Thr Ser Ala Thr Gln Arg Ser Ser Val Pro Ser Ser
           35                  40                  45
   Thr Glu Lys Asn Ala Val Ser Met Thr Ser Ser Val Leu Ser Ser His
       50                  55                  60
   Ser Pro Gly Ser Gly Ser Ser Thr Thr Gln Gly Gln Asp Val Thr Leu
   65                  70                  75                  80
   Ala Pro Ala Thr Glu Pro Ala Ser Gly Ser Ala Ala Thr Trp Gly Gln
                   85                  90                  95
   Asp Val Thr Ser Val Pro Val Thr Arg Pro Ala Leu Gly Ser Thr Thr
               100                 105                 110
   Pro Pro Ala His Asp Val Thr Ser Ala Pro Asp Asn Lys Pro Ala Pro
           115                 120                 125
   Gly Ser Thr Ala Pro Pro Ala His Gly Val Thr Ser Ala Pro Asp Thr
       130                 135                 140
   Arg Pro Ala Pro Gly Ser Thr Ala Pro Pro Ala His Gly Val Thr Ser
   145                 150                 155                 160
   Ala Pro Asp Thr Arg Pro Ala Pro Gly Ser Thr Ala Pro Pro Ala His
                   165                 170                 175
   Gly Val Thr Ser Ala Pro Asp Thr Arg Pro Ala Pro Gly Ser Thr Ala
               180                 185                 190
   Pro Pro Ala His Gly Val Thr Ser Ala Pro Asp Thr Arg Pro Ala Pro
   195 200 205
   Gly Ser Thr Ala Pro Pro Ala His Gly Val Thr Ser Ala Pro Asp Thr
   210 215 220
   Arg Pro Ala Pro Gly Ser Thr Ala Pro Pro Ala His Gly Val Thr Ser
   225 230 235 240
   Ala Pro Asp Thr Arg Pro Ala Pro Gly Ser Thr Ala Pro Pro Ala His
   245 250 255
   Gly Val Thr Ser Ala Pro Asp Thr Arg Pro Ala Pro Gly Ser Thr Ala
   260 265 270
   Pro Pro Ala His Gly Val Thr Ser Ala Pro Asp Thr Arg Pro Ala Pro
   275 280 285
   Gly Ser Thr Ala Pro Pro Ala His Gly Val Thr Ser Ala Pro Asp Thr
   290 295 300
   Arg Pro Ala Pro Gly Ser Thr Ala Pro Pro Ala His Gly Val Thr Ser
   305 310 315 320
   Ala Pro Asp Thr Arg Pro Ala Pro Gly Ser Thr Ala Pro Pro Ala His
   325 330 335
   Gly Val Thr Ser Ala Pro Asp Thr Arg Pro Ala Pro Gly Ser Thr Ala
   340 345 350
   Pro Pro Ala His Gly Val Thr Ser Ala Pro Asp Thr Arg Pro Ala Pro
   355 360 365
   Gly Ser Thr Ala Pro Pro Ala His Gly Val Thr Ser Ala Pro Asp Thr
   370 375 380
   Arg Pro Ala Pro Gly Ser Thr Ala Pro Pro Ala His Gly Val Thr Ser
   385 390 395 400
   Ala Pro Asp Thr Arg Pro Ala Pro Gly Ser Thr Ala Pro Pro Ala His
   405 410 415
   Gly Val Thr Ser Ala Pro Asp Thr Arg Pro Ala Pro Gly Ser Thr Ala
   420 425 430
   Pro Pro Ala His Gly Val Thr Ser Ala Pro Asp Thr Arg Pro Ala Pro
   435 440 445
   Gly Ser Thr Ala Pro Pro Ala His Gly Val Thr Ser Ala Pro Asp Thr
   450 455 460
   Arg Pro Ala Pro Gly Ser Thr Ala Pro Pro Ala His Gly Val Thr Ser
   465 470 475 480
   Ala Pro Asp Thr Arg Pro Ala Pro Gly Ser Thr Ala Pro Pro Ala His
   485 490 495
   Gly Val Thr Ser Ala Pro Asp Thr Arg Pro Ala Pro Gly Ser Thr Ala
   500 505 510
   Pro Pro Ala His Gly Val Thr Ser Ala Pro Asp Thr Arg Pro Ala Pro
           515                 520                 525
   Gly Ser Thr Ala Pro Pro Ala His Gly Val Thr Ser Ala Pro Asp Thr
       530                 535                 540
   Arg Pro Ala Pro Gly Ser Thr Ala Pro Pro Ala His Gly Val Thr Ser
   545                 550                 555                 560
   Ala Pro Asp Thr Arg Pro Ala Pro Gly Ser Thr Ala Pro Pro Ala His
                   565                 570                 575
   Gly Val Thr Ser Ala Pro Asp Thr Arg Pro Ala Pro Gly Ser Thr Ala
               580                 585                 590
   Pro Pro Ala His Gly Val Thr Ser Ala Pro Asp Thr Arg Pro Ala Pro
           595                 600                 605
   Gly Ser Thr Ala Pro Pro Ala His Gly Val Thr Ser Ala Pro Asp Thr
       610                 615                 620
   Arg Pro Ala Pro Gly Ser Thr Ala Pro Pro Ala His Gly Val Thr Ser
   625                 630                 635                 640
   Ala Pro Asp Thr Arg Pro Ala Pro Gly Ser Thr Ala Pro Pro Ala His
                   645                 650                 655
   Gly Val Thr Ser Ala Pro Asp Thr Arg Pro Ala Pro Gly Ser Thr Ala
               660                 665                 670
   Pro Pro Ala His Gly Val Thr Ser Ala Pro Asp Thr Arg Pro Ala Pro
           675                 680                 685
   Gly Ser Thr Ala Pro Pro Ala His Gly Val Thr Ser Ala Pro Asp Thr
       690                 695                 700
   Arg Pro Ala Pro Gly Ser Thr Ala Pro Pro Ala His Gly Val Thr Ser
   705                 710                 715                 720
   Ala Pro Asp Thr Arg Pro Ala Pro Gly Ser Thr Ala Pro Pro Ala His
                   725                 730                 735
   Gly Val Thr Ser Ala Pro Asp Thr Arg Pro Ala Pro Gly Ser Thr Ala
               740                 745                 750
   Pro Pro Ala His Gly Val Thr Ser Ala Pro Asp Thr Arg Pro Ala Pro
           755                 760                 765
   Gly Ser Thr Ala Pro Pro Ala His Gly Val Thr Ser Ala Pro Asp Thr
       770                 775                 780
   Arg Pro Ala Pro Gly Ser Thr Ala Pro Pro Ala His Gly Val Thr Ser
   785                 790                 795                 800
   Ala Pro Asp Thr Arg Pro Ala Pro Gly Ser Thr Ala Pro Pro Ala His
                   805                 810                 815
   Gly Val Thr Ser Ala Pro Asp Thr Arg Pro Ala Pro Gly Ser Thr Ala
               820                 825                 830
   Pro Pro Ala His Gly Val Thr Ser Ala Pro Asp Thr Arg Pro Ala Pro
           835                 840                 845
   Gly Ser Thr Ala Pro Pro Ala His Gly Val Thr Ser Ala Pro Asp Thr
       850                 855                 860
   Arg Pro Ala Pro Gly Ser Thr Ala Pro Pro Ala His Gly Val Thr Ser
   865                 870                 875                 880
   Ala Pro Asp Thr Arg Pro Ala Pro Gly Ser Thr Ala Pro Pro Ala His
                   885                 890                 895
   Gly Val Thr Ser Ala Pro Asp Thr Arg Pro Ala Pro Gly Ser Thr Ala
               900                 905                 910
   Pro Pro Ala His Gly Val Thr Ser Ala Pro Asp Thr Arg Pro Ala Pro
           915                 920                 925
   Gly Ser Thr Ala Pro Pro Ala His Gly Val Thr Ser Ala Pro Asp Asn
       930                 935                 940
   Arg Pro Ala Leu Gly Ser Thr Ala Pro Pro Val His Asn Val Thr Ser
   945                 950                 955                 960
   Ala Ser Gly Ser Ala Ser Gly Ser Ala Ser Thr Leu Val His Asn Gly
                   965                 970                 975
   Thr Ser Ala Arg Ala Thr Thr Thr Pro Ala Ser Lys Ser Thr Pro Phe
               980                 985                 990
   Ser Ile Pro Ser His His Ser Asp  Thr Pro Thr Thr Leu  Ala Ser His
           995                 1000                1005
   Ser Thr  Lys Thr Asp Ala Ser  Ser Thr His His Ser  Ser Val Pro
       1010                 1015                 1020
   Pro Leu  Thr Ser Ser Asn His  Ser Thr Ser Pro Gln  Leu Ser Thr
       1025                 1030                 1035
   Gly Val  Ser Phe Phe Phe Leu  Ser Phe His Ile Ser  Asn Leu Gln
       1040                 1045                 1050
   Phe Asn  Ser Ser Leu Glu Asp  Pro Ser Thr Asp Tyr  Tyr Gln Glu
       1055                 1060                 1065
   Leu Gln  Arg Asp Ile Ser Glu  Met Phe Leu Gln Ile  Tyr Lys Gln
       1070                 1075                 1080
   Gly Gly  Phe Leu Gly Leu Ser  Asn Ile Lys Phe Arg  Pro Gly Ser
       1085                 1090                 1095
   Val Val  Val Gln Leu Thr Leu  Ala Phe Arg Glu Gly  Thr Ile Asn
       1100                 1105                 1110
   Val His  Asp Val Glu Thr Gln  Phe Asn Gln Tyr Lys  Thr Glu Ala
       1115                 1120                 1125
   Ala Ser  Arg Tyr Asn Leu Thr  Ile Ser Asp Val Ser  Val Ser Asp
       1130                 1135                 1140
   Val Pro Phe Pro Phe Ser Ala Gln Ser Gly Ala Gly Val Pro Gly
   1145 1150 1155
   Trp Gly Ile Ala Leu Leu Val Leu Val Cys Val Leu Val Ala Leu
   1160 1165 1170
   Ala Ile Val Tyr Leu Ile Ala Leu Ala Val Cys Gln Cys Arg Arg
   1175 1180 1185
   Lys Asn Tyr Gly Gln Leu Asp Ile Phe Pro Ala Arg Asp Thr Tyr
   1190 1195 1200
   His Pro Met Ser Glu Tyr Pro Thr Tyr His Thr His Gly Arg Tyr
   1205 1210 1215
   Val Pro Pro Ser Ser Thr Asp Arg Ser Pro Tyr Glu Lys Val Ser
   1220 1225 1230
   Ala Gly Asn Gly Gly Ser Ser Leu Ser Tyr Thr Asn Pro Ala Val
   1235 1240 1245
   Ala Ala Ala Ser Ala Asn Leu
   1250 1255
<210> 11
   <211> 302
   <212> PRT
   <213> Homo sapiens
<400> 11
   Ala Ala Ala Lys Glu Gly Lys Lys Ser Arg Asp Arg Glu Arg Pro Pro
   1 5 10 15
   Ser Val Pro Ala Leu Arg Glu Gln Pro Pro Glu Thr Glu Pro Gln Pro
   20 25 30
   Ala Trp Lys Met Pro Arg Ser Cys Cys Ser Arg Ser Gly Ala Leu Leu
   35 40 45
   Leu Ala Leu Leu Leu Gln Ala Ser Met Glu Val Arg Gly Trp Cys Leu
   50 55 60
   Glu Ser Ser Gln Cys Gln Asp Leu Thr Thr Glu Ser Asn Leu Leu Glu
   65 70 75 80
   Cys Ile Arg Ala Cys Lys Pro Asp Leu Ser Ala Glu Thr Pro Met Phe
   85 90 95
   Pro Gly Asn Gly Asp Glu Gln Pro Leu Thr Glu Asn Pro Arg Lys Tyr
   100 105 110
   Val Met Gly His Phe Arg Trp Asp Arg Phe Gly Arg Arg Asn Ser Ser
   115 120 125
   Ser Ser Gly Ser Ser Gly Ala Gly Gln Lys Arg Glu Asp Val Ser Ala
   130 135 140
   Gly Glu Asp Cys Gly Pro Leu Pro Glu Gly Gly Pro Glu Pro Arg Ser
   145 150 155 160
   Asp Gly Ala Lys Pro Gly Pro Arg Glu Gly Lys Arg Ser Tyr Ser Met
   165 170 175
   Glu His Phe Arg Trp Gly Lys Pro Val Gly Lys Lys Arg Arg Pro Val
   180 185 190
   Lys Val Tyr Pro Asn Gly Ala Glu Asp Glu Ser Ala Glu Ala Phe Pro
   195 200 205
   Leu Glu Phe Lys Arg Glu Leu Thr Gly Gln Arg Leu Arg Glu Gly Asp
   210 215 220
   Gly Pro Asp Gly Pro Ala Asp Asp Gly Ala Gly Ala Gln Ala Asp Leu
   225 230 235 240
   Glu His Ser Leu Leu Val Ala Ala Glu Lys Lys Asp Glu Gly Pro Tyr
   245 250 255
   Arg Met Glu His Phe Arg Trp Gly Ser Pro Pro Lys Asp Lys Arg Tyr
   260 265 270
   Gly Gly Phe Met Thr Ser Glu Lys Ser Gln Thr Pro Leu Val Thr Leu
   275 280 285
   Phe Lys Asn Ala Ile Ile Lys Asn Ala Tyr Lys Lys Gly Glu
   290 295 300
<210> 12
   <211> 31
   <212> PRT
   <213> Artificial Sequence
<220>
   <223> Synthetic Peptide
<400> 12
   His His His His His His Ser Ser Ser Ser Gly Ser Ser Ser Ser Gly
   1 5 10 15
   Ser Ser Ser Ser Gly Gly Arg Gly Asp Ser Gly Arg Gly Asp Ser
   20 25 30
<210> 13
   <211> 19
   <212> PRT
   <213> Artificial Sequence
<220>
   <223> Synthetic Peptide
<400> 13
   His His His His His His Arg Gly Glu Phe Thr Gly Thr Tyr Ile Thr
   1 5 10 15
   Ala Val Thr
<210> 14
   <211> 12
   <212> PRT
   <213> Homo sapiens
<400> 14
   Thr Phe Ile Ala Ile Lys Pro Asp Gly Val Gln Arg
   1 5 10
<210> 15
   <211> 18
   <212> PRT
   <213> Homo sapiens
<220>
   <221> misc_feature
   <222> (3)..(3)
   <223> Xaa can be any naturally occurring amino acid
<400> 15
   Val Met Xaa Leu Gly Glu Thr Asn Pro Ala Asp Ser Lys Pro Gly Thr
   1 5 10 15
   Ile Arg
<210> 16
   <211> 17
   <212> PRT
   <213> Homo sapiens
<400> 16
   Val Met Leu Gly Glu Thr Asn Pro Ala Asp Ser Lys Pro Gly Thr Ile
   1 5 10 15
   Arg
<210> 17
   <211> 10
   <212> PRT
   <213> Homo sapiens
<400> 17
   Asn Ile Ile His Gly Ser Asp Ser Val Lys
   1 5 10
<210> 18
   <211> 9
   <212> PRT
   <213> Homo sapiens
<400> 18
   Gly Leu Val Gly Glu Ile Ile Lys Arg
   1 5
<210> 19
   <211> 8
   <212> PRT
   <213> Homo sapiens
<400> 19
   Gly Leu Val Gly Glu Ile Ile Lys
   1               5
<210> 20
   <211> 21
   <212> PRT
   <213> Homo sapiens
<220>
   <221> mise_feature
   <222> (3)..(3)
   <223> Xaa can be any naturally occurring amino acid
<220>
   <221> misc_feature
   <222> (12)..(12)
   <223> Xaa can be any naturally occurring amino acid
<400> 20
   Tyr Met Xaa His Ser Gly Pro Val Val Ala Met Xaa Val Trp Glu Gly
   1               5                   10                  15
   Leu Asn Val Val Lys
               20
<210> 21
   <211> 19
   <212> PRT
   <213> Homo sapiens
<400> 21
   Ala Ala Phe Asp Asp Ala Ile Ala Glu Leu Asp Thr Leu Ser Glu Glu
   1               5                   10                  15
   Ser Tyr Lys
<210> 22
   <211> 18
   <212> PRT
   <213> Homo sapiens
<220>
   <221> misc_feature
   <222> (8)..(8)
   <223> Xaa can be any naturally occurring amino acid
<400> 22
   Ala Ala Ser Asp Ile Ala Met Xaa Thr Glu Leu Pro Pro Thr His Pro
   1 5 10 15
   Ile Arg
<210> 23
   <211> 11
   <212> PRT
   <213> Homo sapiens
<400> 23
   Tyr Leu Ala Glu Phe Ala Thr Gly Asn Asp Arg
   1 5 10
<210> 24
   <211> 10
   <212> PRT
   <213> Homo sapiens
<400> 24
   Asp Ser Thr Leu Ile Met Gln Leu Leu Arg
   1 5 10
<210> 25
   <211> 9
   <212> PRT
   <213> Homo sapiens
<400> 25
   Tyr Asp Glu Met Val Glu Ser Met Lys
   1 5
<210> 26
   <211> 14
   <212> PRT
   <213> Homo sapiens
<220>
   <221> misc_feature
   <222> (5)..(5)
   <223> Xaa can be any naturally occurring amino acid
<400> 26
   Val Ala Gly Met Xaa Asp Val Glu Leu Thr Val Glu Glu Arg
   1 5 10
<210> 27
   <211> 12
   <212> PRT
   <213> Homo sapiens
<400> 27
   His Leu Ile Pro Ala Ala Asn Thr Gly Glu Ser Lys
   1 5 10
<210> 28
   <211> 19
   <212> PRT
   <213> Homo sapiens
<220>
   <221> misc_feature
   <222> (12)..(12)
   <223> Xaa can be any naturally occurring amino acid
<400> 28
   Asp Pro Asp Ala Gln Pro Gly Gly Glu Leu Met Xaa Leu Gly Gly Thr
   1 5 10 15
   Asp Ser Lys
<210> 29
   <211> 18
   <212> PRT
   <213> Homo sapiens
<400> 29
   Asp Pro Asp Ala Gln Pro Gly Gly Glu Leu Met Leu Gly Gly Thr Asp
   1 5 10 15
   Ser Lys
<210> 30
   <211> 18
   <212> PRT
   <213> Homo sapiens
<220>
   <221> misc_feature
   <222> (15)..(15)
   <223> Xaa can be any naturally occurring amino acid
<400> 30
   Ile Ser Val Asn Asn Val Leu Pro Val Phe Asp Asn Leu Met Xaa Gln
   1 5 10 15
   Gln Lys
<210> 31
   <211> 17
   <212> PRT
   <213> Homo sapiens
<400> 31
   Ile Ser Val Asn Asn Val Leu Pro Val Phe Asp Asn Leu Met Gln Gln
   1               5                   10                  15
   Lys
<210> 32
   <211> 10
   <212> PRT
   <213> Homo sapiens
<400> 32
   Gln Pro Gly Ile Thr Phe Ile Ala Ala Lys
   1               5                   10
<210> 33
   <211> 16
   <212> PRT
   <213> Homo sapiens
<400> 33
   Gly Leu Gly Thr Asp Glu Glu Ser Ile Leu Thr Leu Leu Thr Ser Arg
   1               5                   10                  15
<210> 34
   <211> 13
   <212> PRT
   <213> Homo sapiens
<400> 34
   Asp Leu Leu Asp Asp Leu Lys Ser Glu Leu Thr Gly Lys
   1               5                   10
<210> 35
   <211> 9
   <212> PRT
   <213> Homo sapiens
<400> 35
   Ser Glu Ile Asp Leu Phe Asn Ile Arg
   1               5
<210> 36
   <211> 45
   <212> PRT
   <213> Homo sapiens
<400> 36
   Gly Thr Ile Asn Val His Asp Val Glu Thr Gln Phe Asn Gln Tyr Lys
   1               5                   10                  15
   Thr Glu Ala Ala Ser Arg Tyr Asn Leu Thr Ile Ser Asp Val Ser Val
               20                  25                  30
   Ser Asp Val Pro Phe Pro Phe Ser Ala Gln Ser Gly Ala
           35                  40                  45
<210> 37
   <211> 146
   <212> PRT
   <213> Homo sapiens
<400> 37
   Gly Thr Ile Asn Val His Asp Val Glu Thr Gln Phe Asn Gln Tyr Lys
   1               5                   10                  15
   Thr Glu Ala Ala Ser Arg Tyr Asn Leu Thr Ile Ser Asp Val Ser Val
               20                  25                  30
   Ser Asp Val Pro Phe Pro Phe Ser Ala Gln Ser Gly Ala Gly Val Pro
           35                  40                  45
   Gly Trp Gly Ile Ala Leu Leu Val Leu Val Cys Val Leu Val Ala Leu
       50                  55                  60
   Ala Ile Val Tyr Leu Ile Ala Leu Ala Val Cys Gln Cys Arg Arg Lys
   65                  70                  75                  80
   Asn Tyr Gly Gln Leu Asp Ile Phe Pro Ala Arg Asp Thr Tyr His Pro
                   85                  90                  95
   Met Ser Glu Tyr Pro Thr Tyr His Thr His Gly Arg Tyr Val Pro Pro
               100                 105                 110
   Ser Ser Thr Asp Arg Ser Pro Tyr Glu Lys Val Ser Ala Gly Asn Gly
           115                 120                 125
   Gly Ser Ser Leu Ser Tyr Thr Asn Pro Ala Val Ala Ala Ala Ser Ala
       130                 135                 140
   Asn Leu
   145
<210> 38
   <211> 171
   <212> PRT
   <213> Homo sapiens
<400> 38
   Gly Phe Leu Gly Leu Ser Asn Ile Lys Phe Arg Pro Gly Ser Val Val
   1               5                   10                  15
   Val Gln Leu Thr Leu Ala Phe Arg Glu Gly Thr Ile Asn Val His Asp
               20                  25                  30
   Val Glu Thr Gln Phe Asn Gln Tyr Lys Thr Glu Ala Ala Ser Arg Tyr
           35                  40                  45
   Asn Leu Thr Ile Ser Asp Val Ser Val Ser Asp Val Pro Phe Pro Phe
       50                  55                  60
   Ser Ala Gln Ser Gly Ala Gly Val Pro Gly Trp Gly Ile Ala Leu Leu
   65                  70                  75                  80
   Val Leu Val Cys Val Leu Val Ala Leu Ala Ile Val Tyr Leu Ile Ala
                   85                  90                  95
   Leu Ala Val Cys Gln Cys Arg Arg Lys Asn Tyr Gly Gln Leu Asp Ile
               100                 105                 110
   Phe Pro Ala Arg Asp Thr Tyr His Pro Met Ser Glu Tyr Pro Thr Tyr
           115                 120                 125
   His Thr His Gly Arg Tyr Val Pro Pro Ser Ser Thr Asp Arg Ser Pro
       130                 135                 140
   Tyr Glu Lys Val Ser Ala Gly Asn Gly Gly Ser Ser Leu Ser Tyr Thr
   145                 150                 155                 160
   Asn Pro Ala Val Ala Ala Ala Ser Ala Asn Leu
                   165                 170
<210> 39
   <211> 275
   <212> PRT
   <213> Homo sapiens
<400> 39
   Ala Thr Thr Thr Pro Ala Ser Lys Ser Thr Pro Phe Ser Ile Pro Ser
   1               5                   10                  15
   His His Ser Asp Thr Pro Thr Thr Leu Ala Ser His Ser Thr Lys Thr
               20                  25                  30
   Asp Ala Ser Ser Thr His His Ser Thr Val Pro Pro Leu Thr Ser Ser
           35                  40                  45
   Asn His Ser Thr Ser Pro Gln Leu Ser Thr Gly Val Ser Phe Phe Phe
       50                  55                  60
   Leu Ser Phe His Ile Ser Asn Leu Gln Phe Asn Ser Ser Leu Glu Asp
   65 70 75 80
   Pro Ser Thr Asp Tyr Tyr Gln Glu Leu Gln Arg Asp Ile Ser Glu Met
   85 90 95
   Phe Leu Gln Ile Tyr Lys Gln Gly Gly Phe Leu Gly Leu Ser Asn Ile
   100 105 110
   Lys Phe Arg Pro Gly Ser Val Val Val Gln Leu Thr Leu Ala Phe Arg
   115 120 125
   Glu Gly Thr Ile Asn Val His Asp Val Glu Thr Gln Phe Asn Gln Tyr
   130 135 140
   Lys Thr Glu Ala Ala Ser Arg Tyr Asn Leu Thr Ile Ser Asp Val Ser
   145 150 155 160
   Val Ser Asp Val Pro Phe Pro Phe Ser Ala Gln Ser Gly Ala Gly Val
   165 170 175
   Pro Gly Trp Gly Ile Ala Leu Leu Val Leu Val Cys Val Leu Val Ala
   180 185 190
   Leu Ala Ile Val Tyr Leu Ile Ala Leu Ala Val Cys Gln Cys Arg Arg
   195 200 205
   Lys Asn Tyr Gly Gln Leu Asp Ile Phe Pro Ala Arg Asp Thr Tyr His
   210 215 220
   Pro Met Ser Glu Tyr Pro Thr Tyr His Thr His Gly Arg Tyr Val Pro
   225 230 235 240
   Pro Ser Ser Thr Asp Arg Ser Pro Tyr Glu Lys Val Ser Ala Gly Asn
   245 250 255
   Gly Gly Ser Ser Leu Ser Tyr Thr Asn Pro Ala Val Ala Ala Ala Ser
   260 265 270
   Ala Asn Leu
   275
<210> 40
   <211> 233
   <212> PRT
   <213> Homo sapiens
<400> 40
   Gly Ser Gly His Ala Ser Ser Thr Pro Gly Gly Glu Lys Glu Thr Ser
   1 5 10 15
   Ala Thr Gln Arg Ser Ser Val Pro Ser Ser Thr Glu Lys Asn Ala Phe
   20 25 30
   Asn Ser Ser Leu Glu Asp Pro Ser Thr Asp Tyr Tyr Gln Glu Leu Gln
   35 40 45
   Arg Asp Ile Ser Glu Met Phe Leu Gln Ile Tyr Lys Gln Gly Gly Phe
       50                  55                  60
   Leu Gly Leu Ser Asn Ile Lys Phe Arg Pro Gly Ser Val Val Val Gln
   65                  70                  75                  80
   Leu Thr Leu Ala Phe Arg Glu Gly Thr Ile Asn Val His Asp Met Glu
                   85                  90                  95
   Thr Gln Phe Asn Gln Tyr Lys Thr Glu Ala Ala Ser Arg Tyr Asn Leu
               100                 105                 110
   Thr Ile Ser Asp Val Ser Val Ser Asp Val Pro Phe Pro Phe Ser Ala
           115                 120                 125
   Gln Ser Gly Ala Gly Val Pro Gly Trp Gly Ile Ala Leu Leu Val Leu
       130                 135                 140
   Val Cys Val Leu Val Ala Leu Ala Ile Val Tyr Leu Ile Ala Leu Ala
   145                 150                 155                 160
   Val Cys Gln Cys Arg Arg Lys Asn Tyr Gly Gln Leu Asp Ile Phe Pro
                   165                 170                 175
   Ala Arg Asp Thr Tyr His Pro Met Ser Glu Tyr Pro Thr Tyr His Thr
               180                 185                 190
   His Gly Arg Tyr Val Pro Pro Ser Ser Thr Asp Arg Ser Pro Tyr Glu
           195                 200                 205
   Lys Val Ser Ala Gly Asn Gly Gly Ser Ser Leu Ser Tyr Thr Asn Pro
       210                 215                 220
   Ala Val Ala Ala Thr Ser Ala Asn Leu
   225                 230
<210> 41
   <211> 863
   <212> PRT
   <213> Homo sapiens
<400> 41
   Leu Asp Pro Arg Val Arg Thr Ser Ala Pro Asp Thr Arg Pro Ala Pro
   1               5                   10                  15
   Gly Ser Thr Ala Pro Gln Ala His Gly Val Thr Ser Ala Pro Asp Thr
               20                  25                  30
   Arg Pro Ala Pro Gly Ser Thr Ala Pro Pro Ala His Gly Val Thr Ser
           35                  40                  45
   Ala Pro Asp Thr Arg Pro Ala Pro Gly Ser Thr Ala Pro Pro Ala His
       50                  55                  60
   Gly Val Thr Ser Ala Pro Asp Thr Arg Pro Ala Pro Gly Ser Thr Ala
   65                  70                  75                  80
   Pro Pro Ala His Gly Val Thr Ser Ala Pro Asp Thr Arg Pro Ala Pro
                   85                  90                  95
   Gly Ser Thr Ala Pro Pro Ala His Gly Val Thr Ser Ala Pro Asp Thr
               100                 105                 110
   Arg Pro Ala Pro Gly Ser Thr Ala Pro Pro Ala His Gly Val Thr Ser
           115                 120                 125
   Ala Pro Asp Thr Arg Pro Ala Pro Gly Ser Thr Ala Pro Pro Ala His
       130                 135                 140
   Gly Val Thr Ser Ala Pro Asp Thr Arg Pro Ala Pro Gly Ser Thr Ala
   145                 150                 155                 160
   Pro Pro Ala His Gly Val Thr Ser Ala Pro Asp Thr Arg Pro Ala Pro
                   165                 170                 175
   Gly Ser Thr Ala Pro Pro Ala His Gly Val Thr Ser Ala Pro Asp Thr
               180                 185                 190
   Arg Pro Ala Pro Gly Ser Thr Ala Pro Pro Ala His Gly Val Thr Ser
           195                 200                 205
   Ala Pro Asp Thr Arg Pro Ala Pro Gly Ser Thr Ala Pro Pro Ala His
       210                 215                 220
   Gly Val Thr Ser Ala Pro Asp Thr Arg Pro Ala Pro Gly Ser Thr Ala
   225                 230                 235                 240
   Pro Pro Ala His Gly Val Thr Ser Ala Pro Asp Thr Arg Pro Ala Pro
                   245                 250                 255
   Gly Ser Thr Ala Pro Pro Ala His Gly Val Thr Ser Ala Pro Asp Thr
               260                 265                 270
   Arg Pro Ala Pro Gly Ser Thr Ala Pro Pro Ala His Gly Val Thr Ser
           275                 280                 285
   Ala Pro Asp Thr Arg Pro Ala Pro Gly Ser Thr Ala Pro Pro Ala His
       290                 295                 300
   Gly Val Thr Ser Ala Pro Asp Thr Arg Pro Ala Pro Gly Ser Thr Ala
   305                 310                 315                 320
   Pro Pro Ala His Gly Val Thr Ser Ala Pro Asp Thr Arg Pro Ala Pro
                   325                 330                 335
   Gly Ser Thr Ala Pro Pro Ala His Gly Val Thr Ser Ala Pro Asp Thr
               340                 345                 350
   Arg Pro Ala Pro Gly Ser Thr Ala Pro Pro Ala His Gly Val Thr Ser
           355                 360                 365
   Ala Pro Asp Thr Arg Pro Ala Pro Gly Ser Thr Ala Pro Pro Ala His
       370                 375                 380
   Gly Val Thr Ser Ala Pro Asp Thr Arg Pro Ala Pro Gly Ser Thr Ala
   385                 390                 395                 400
   Pro Pro Ala His Gly Val Thr Ser Ala Pro Asp Thr Arg Pro Ala Pro
                   405                 410                 415
   Gly Ser Thr Ala Pro Pro Ala His Gly Val Thr Ser Ala Pro Asp Thr
   420 425 430
   Arg Pro Ala Pro Gly Ser Thr Ala Pro Pro Ala His Gly Val Thr Ser
   435 440 445
   Ala Pro Asp Thr Arg Pro Ala Pro Gly Ser Thr Ala Pro Pro Ala His
   450 455 460
   Gly Val Thr Ser Ala Pro Asp Thr Arg Pro Ala Pro Gly Ser Thr Ala
   465 470 475 480
   Pro Pro Ala His Gly Val Thr Ser Ala Pro Asp Thr Arg Pro Ala Pro
   485 490 495
   Gly Ser Thr Ala Pro Pro Ala His Gly Val Thr Ser Ala Pro Asp Thr
   500 505 510
   Arg Pro Ala Pro Gly Ser Thr Ala Pro Pro Ala His Gly Val Thr Ser
   515 520 525
   Ala Pro Asp Thr Arg Pro Ala Pro Gly Ser Thr Ala Pro Pro Ala His
   530 535 540
   Gly Val Thr Ser Ala Pro Asp Asn Arg Pro Ala Leu Gly Ser Thr Ala
   545 550 555 560
   Pro Pro Val His Asn Val Thr Ser Ala Ser Gly Ser Ala Ser Gly Ser
   565 570 575
   Ala Ser Thr Leu Val His Asn Gly Thr Ser Ala Arg Ala Thr Thr Thr
   580 585 590
   Pro Ala Ser Lys Ser Thr Pro Phe Ser Ile Pro Ser His His Ser Asp
   595 600 605
   Thr Pro Thr Thr Leu Ala Ser His Ser Thr Lys Thr Asp Ala Ser Ser
   610 615 620
   Thr His His Ser Ser Val Pro Pro Leu Thr Ser Ser Asn His Ser Thr
   625 630 635 640
   Ser Pro Gln Leu Ser Thr Gly Val Ser Phe Phe Phe Leu Ser Phe His
   645 650 655
   Ile Ser Asn Leu Gln Phe Asn Ser Ser Leu Glu Asp Pro Ser Thr Asp
   660 665 670
   Tyr Tyr Gln Glu Leu Gln Arg Asp Ile Ser Glu Met Phe Leu Gln Ile
   675 680 685
   Tyr Lys Gln Gly Gly Phe Leu Gly Leu Ser Asn Ile Lys Phe Arg Pro
   690 695 700
   Gly Ser Val Val Val Gln Leu Thr Leu Ala Phe Arg Glu Gly Thr Ile
   705 710 715 720
   Asn Val His Asp Val Glu Thr Gln Phe Asn Gln Tyr Lys Thr Glu Ala
   725 730 735
   Ala Ser Arg Tyr Asn Leu Thr Ile Ser Asp Val Ser Val Ser Asp Val
               740                 745                 750
   Pro Phe Pro Phe Ser Ala Gln Ser Gly Ala Gly Val Pro Gly Trp Gly
           755                 760                 765
   Ile Ala Leu Leu Val Leu Val Cys Val Leu Val Ala Leu Ala Ile Val
       770                 775                 780
   Tyr Leu Ile Ala Leu Ala Val Cys Gln Cys Arg Arg Lys Asn Tyr Gly
   785                 790                 795                 800
   Gln Leu Asp Ile Phe Pro Ala Arg Asp Thr Tyr His Pro Met Ser Glu
                   805                 810                 815
   Tyr Pro Thr Tyr His Thr His Gly Arg Tyr Val Pro Pro Ser Ser Thr
               820                 825                 830
   Asp Arg Ser Pro Tyr Glu Lys Val Ser Ala Gly Asn Gly Gly Ser Ser
           835                 840                 845
   Leu Ser Tyr Thr Asn Pro Ala Val Ala Ala Ala Ser Ala Asn Leu
       850                 855                 860
<210> 42
   <211> 751
   <212> DNA
   <213> Homo sapiens
<400> 42
   acgggcacgg ccggtaccat caatgtccac gacgtggaga cacagttcaa tcagtataaa    60
   acggaagcag cctctcgata taacctgacg atctcagacg tcagcgtgag tgatgtgcca   120
   tttcctttct ctgcccagtc tggggctggg gtgccaggct ggggcatcgc gctgctggtg   180
   ctggtctgtg ttctggttgc gctggccatt gtctatctca ttgccttggc tgtctgtcag   240
   tgccgccgaa agaactacgg gcagctggac atctttccag cccgggatac ctaccatcct   300
   atgagcgagt accccaccta ccacacccat gggcgctatg tgccccctag cagtaccgat   360
   cgtagcccct atgagaaggt ttctgcaggt aacggtggca gcagcctctc ttacacaaac   420
   ccagcagtgg cagccgcttc tgccaacttg tagggcacgt cgccgctgag ctgagtggcc   480
   agccagtgcc attccactcc actcaggttc ttcaggccag agcccctgca ccctgtttgg   540
   gctggtgagc tgggagttca ggtgggctgc tcacagcctc cttcagaggc cccaccaatt   600
   tctcggacac ttctcagtgt gtggaagctc atgtgggccc ctgaggctca tgcctgggaa   660
   gtgttgtggg ggctcccagg aggactggcc cagagagccc tgagatagcg gggatcctga   720
   actggactga ataaaacgtg gtctcccact g                                  751
<210> 43
   <211> 820
   <212> DNA
   <213> Homo sapiens
<400> 43
   acggccggtt ttctgggcct ctccaatatt aagttcaggc caggatctgt ggtggtacaa     60
   ttgactctgg ccttccgaga aggtaccatc aatgtccacg acgtggagac acagttcaat    120
   cagtataaaa cggaagcagc ctctcgatat aacctgacga tctcagacgt cagcgtgagt    180
   gatgtgccat ttcctttctc tgcccagtct ggggctgggg tgccaggctg gggcatcgcg    240
   ctgctggtgc tggtctgtgt tctggttgcg ctggccattg tctatctcat tgccttggct    300
   gtctgtcagt gccgccgaaa gaactacggg cagctggaca tctttccagc ccgggatacc    360
   taccatccta tgagcgagta ccccacctac cacacccatg ggcgctatgt gccccctagc    420
   agtaccgatc gtagccccta tgagaaggtt tctgcaggta acggtggcag cagcctctct    480
   tacacaaacc cagcagtggc agccgcttct gccaacttgt agggcacgtc gccgctgagc    540
   tgagtggcca gccagtgcca ttccactcca ctcaggttct tcaggccaga gcccctgcac    600
   cctgtttggg ctggtgagct gggagttcag gtgggctgct cacagcctcc ttcagaggcc    660
   ccaccaattt ctcggacact tctcagtgtg tggaagctca tgtgggcccc tgaggctcat    720
   gcctgggaag tgttgtgggg gctcccagga ggactggccc agagagccct gagatagcgg    780
   ggatcctgaa ctggactgaa taaaacgtgg tctcccactg                          820
<210> 44
   <211> 1132
   <212> DNA
   <213> Homo sapiens
<400> 44
   acggccgcta ccacaacccc agccagcaag agcactccat tctcaattcc cagccaccac     60
   tctgatactc ctaccaccct tgccagccat agcaccaaga ctgatgccag tagcactcac    120
   catagctcgg tacctcctct cacctcctcc aatcacagca cttctcccca gttgtctact    180
   ggggtctctt tctttttcct gtcttttcac atttcaaacc tccagtttaa ttcctctctg    240
   gaagatccca gcaccgacta ctaccaagag ctgcagagag acatttctga aatgtttttg    300
   cagatttata aacaaggggg ttttctgggc ctctccaata ttaagttcag gccaggatct    360
   gtggtggtac aattgactct ggccttccga gaaggtacca tcaatgtcca cgacgtggag    420
   acacagttca atcagtataa aacggaagca gcctctcgat ataacctgac gatctcagac    480
   gtcagcgtga gtgatgtgcc atttcctttc tctgcccagt ctggggctgg ggtgccaggc    540
   tggggcatcg cgctgctggt gctggtctgt gttctggttg cgctggccat tgtctatctc    600
   attgccttgg ctgtctgtca gtgccgccga aagaactacg ggcagctgga catctttcca    660
   gcccgggata cctaccatcc tatgagcgag taccccacct accacaccca tgggcgctat 720
   gtgcccccta gcagtaccga tcgtagcccc tatgagaagg tttctgcagg taacggtggc 780
   agcagcctct cttacacaaa cccagcagtg gcagccgctt ctgccaactt gtagggcacg 840
   tcgccgctga gctgagtggc cagccagtgc cattccactc cactcaggtt cttcaggcca 900
   gagcccctgc accctgtttg ggctggtgag ctgggagttc aggtgggctg ctcacagcct 960
   ccttcagagg ccccaccaat ttctcggaca cttctcagtg tgtggaagct catgtgggcc 1020
   cctgaggctc atgcctggga agtgttgtgg gggctcccag gaggactggc ccagagagcc 1080
   ctgagatagc ggggatcctg aactggactg aataaaacgt ggtctcccac tg 1132
<210> 45
   <211> 717
   <212> DNA
   <213> Homo sapiens
<400> 45
   acaggttctg gtcatgcaag ctctacccca ggtggagaaa aggagacttc ggctacccag 60
   agaagttcag tgcccagctc tactgagaag aatgctttta attcctctct ggaagatccc 120
   agcaccgact actaccaaga gctgcagaga gacatttctg aaatgttttt gcagatttat 180
   aaacaagggg gttttctggg cctctccaat attaagttca ggccaggatc tgtggtggta 240
   caattgactc tggccttccg agaaggtacc atcaatgtcc acgacgtgga gacacagttc 300
   aatcagtata aaacggaagc agcctctcga tataacctga cgatctcaga cgtcagcgtg 360
   agtgatgtgc catttccttt ctctgcccag tctggggctg ggtgccaggc ctggggcatc 420
   gcgctgctgg tgctggtctg tgttctggtt gcgctggcca ttgtctatct cattgccttg 480
   gctgtctgtc agtgccgccg aaagaactac gggcagctgg acatctttcc agcccgggat 540
   acctaccatc ctatgagcga gtaccccacc taccacaccc atgggcgcta tgtgccccct 600
   agcagtaccg atcgtagccc ctatgagaag gtttctgcag gtaatggtgg cagcagcctc 660
   tcttacacaa acccagcagt ggcagccact tctgccaact tgtaggggca cgtcgcc 717
<210> 46
   <211> 2487
   <212> DNA
   <213> Homo sapiens
<400> 46
   ctcgacccac gcgtccgctc gacccacgcg tccgcacctc ggccccggac accaggccgg 60
   ccccgggctc caccgccccc ccagcccacg gtgtcacctc ggccccggac accaggccgg 120
   ccccgggctc caccgccccc ccagcccacg gtgtcacctc ggccccggac accaggccgg 180
   ccccgggctc caccgccccc ccagcccacg gtgtcacctc ggccccggac accaggccgg 240
   ccccgggctc caccgccccc ccagcccacg gtgtcacctc ggccccggac accaggccgg 300
   ccccgggctc caccgccccc ccagcccacg gtgtcacctc ggccccggac accaggccgg 360
   ccccgggctc caccgccccc ccagcccacg gtgtcacctc ggccccggac accaggccgg 420
   ccccgggctc caccgccccc ccagcccacg gtgtcacctc ggccccggac accaggccgg 480
   ccccgggctc caccgccccc ccagcccacg gtgtcacctc ggccccggac accaggccgg 540
   ccccgggctc caccgccccc ccagcccacg gtgtcacctc ggccccggac accaggccgg 600
   ccccgggctc caccgccccc ccagcccacg gtgtcacctc ggccccggac accaggccgg 660
   ccccgggctc caccgccccc ccagcccacg gtgtcacctc ggccccggac accaggccgg 720
   ccccgggctc caccgccccc ccagcccacg gtgtcacctc ggccccggac accaggccgg 780
   ccccgggctc caccgccccc ccagcccacg gtgtcacctc ggccccggac accaggccgg 840
   ccccgggctc caccgccccc ccagcccacg gtgtcacctc ggccccggac accaggccgg 900
   ccccgggctc caccgccccc ccagcccacg gtgtcacctc ggccccggac accaggccgg 960
   ccccgggctc caccgccccc ccagcccacg gtgtcacctc ggccccggac accaggccgg 1020
   ccccgggctc caccgccccc ccagcccacg gtgtcacctc ggccccggac accaggccgg 1080
   ccccgggctc caccgccccc ccagcccacg gtgtcacctc ggccccggac accaggccgg 1140
   ccccgggctc caccgccccc ccagcccacg gtgtcacctc ggccccggac accaggccgg 1200
   ccccgggctc caccgccccc ccagcccatg gtgtcacctc ggccccggac aacaggcccg 1260
   ccttgggctc caccgcccct ccagtccaca atgtcacctc ggcctcaggc tctgcatcag 1320
   gctcagcttc tactctggtg cacaacggca cctctgccag ggctaccaca accccagcca 1380
   gcaagagcac tccattctca attcccagcc accactctga tactcctacc acccttgcca 1440
   gccatagcac caagactgat gccagtagca ctcaccatag ctcggtacct cctctcacct 1500
   cctccaatca cagcacttct ccccagttgt ctactggggt ctctttcttt ttcctgtctt 1560
   ttcacatttc aaacctccag tttaattcct ctctggaaga tcccagcacc gactactacc 1620
   aagagctgca gagagacatt tctgaaatgt ttttgcagat ttataaacaa gggggttttc 1680
   tgggcctctc caatattaag ttcaggccag gatctgtggt ggtacaattg actctggcct 1740
   tccgagaagg taccatcaat gtccacgacg tggagacaca gttcaatcag tataaaacgg 1800
   aagcagcctc tcgatataac ctgacgatct cagacgtcag cgtgagtgat gtgccatttc 1860
   ctttctctgc ccagtctggg gctggggtgc caggctgggg catcgcgctg ctggtgctgg 1920
   tctgtgttct ggttgcgctg gccattgtct atctcattgc cttggctgtc tgtcagtgcc 1980
   gccgaaagaa ctacgggcag ctggacatct ttccagcccg ggatacctac catcctatga 2040
   gcgagtaccc cacctaccac acccatgggc gctatgtgcc ccctagcagt accgatcgta   2100
   gcccctatga gaaggtttct gcaggtaacg gtggcagcag cctctcttac acaaacccag   2160
   cagtggcagc cgcttctgcc aacttgtagg gcacgtcgcc gctgagctga gtggccagcc   2220
   agtgccattc cactccactc aggttcttca ggccagagcc cctgcaccct gtttgggctg   2280
   gtgagctggg agttcaggtg ggctgctcac agcctccttc agaggcccca ccaatttctc   2340
   ggacacttct cagtgtgtgg aagctcatgt gggcccctga ggctcatgcc tgggaagtgt   2400
   tgtgggggct cccaggagga ctggcccaga gagccctgag atagcgggga tcctgaactg   2460
   gactgaataa aacgtggtct cccactg                                       2487
<210> 47
   <211> 19
   <212> PRT
   <213> Homo sapiens
<400> 47
   Met Thr Pro Gly Thr Gln Ser Pro Phe Phe Leu Leu Leu Leu Leu Thr
   1               5                   10                  15
   Val Leu Thr
<210> 48
   <211> 4003
   <212> DNA
   <213> Homo sapiens
<400> 48
   acaggttctg gtcatgcaag ctctacccca ggtggagaaa aggagacttc ggctacccag     60
   agaagttcag tgcccagctc tactgagaag aatgctgtga gtatgaccag cagcgtactc    120
   tccagccaca gccccggttc aggctcctcc accactcagg gacaggatgt cactctggcc    180
   ccggccacgg aaccagcttc aggttcagct gccacctggg gacaggatgt cacctcggtc    240
   ccagtcacca ggccagccct gggctccacc accccgccag cccacgatgt cacctcagcc    300
   ccggacaaca agccagcccc gggctccacc gcccccccag cccacggtgt cacctcggcc    360
   ccggacacca ggccggcccc gggctccacc gcccccccag cccacggtgt cacctcggcc    420
   ccggacacca ggccggcccc gggctccacc gcccccccag cccacggtgt cacctcggcc    480
   ccggacacca ggccggcccc gggctccacc gcccccccag cccacggtgt cacctcggcc    540
   ccggacacca ggccggcccc gggctccacc gcccccccag cccacggtgt cacctcggcc    600
   ccggacacca ggccggcccc gggctccacc gcccccccag cccacggtgt cacctcggcc    660
   ccggacacca ggccggcccc gggctccacc gcccccccag cccacggtgt cacctcggcc    720
   ccggacacca ggccggcccc gggctccacc gcccccccag cccacggtgt cacctcggcc 780
   ccggacacca ggccggcccc gggctccacc gcccccccag cccacggtgt cacctcggcc 840
   ccggacacca ggccggcccc gggctccacc gcccccccag cccacggtgt cacctcggcc 900
   ccggacacca ggccggcccc gggctccacc gcccccccag cccacggtgt cacctcggcc 960
   ccggacacca ggccggcccc gggctccacc gcccccccag cccacggtgt cacctcggcc 1020
   ccggacacca ggccggcccc gggctccacc gcccccccag cccacggtgt cacctcggcc 1080
   ccggacacca ggccggcccc gggctccacc gcccccccag cccacggtgt cacctcggcc 1140
   ccggacacca ggccggcccc gggctccacc gcccccccag cccacggtgt cacctcggcc 1200
   ccggacacca ggccggcccc gggctccacc gcccccccag cccacggtgt cacctcggcc 1260
   ccggacacca ggccggcccc gggctccacc gcccccccag cccacggtgt cacctcggcc 1320
   ccggacacca ggccggcccc gggctccacc gcccccccag cccacggtgt cacctcggcc 1380
   ccggacacca ggccggcccc gggctccacc gcccccccag cccacggtgt cacctcggcc 1440
   ccggacacca ggccggcccc gggctccacc gcccccccag cccacggtgt cacctcggcc 1500
   ccggacacca ggccggcccc gggctccacc gcccccccag cccacggtgt cacctcggcc 1560
   ccggacacca ggccggcccc gggctccacc gcccccccag cccacggtgt cacctcggcc 1620
   ccggacacca ggccggcccc gggctccacc gcccccccag cccacggtgt cacctcggcc 1680
   ccggacacca ggccggcccc gggctccacc gcccccccag cccacggtgt cacctcggcc 1740
   ccggacacca ggccggcccc gggctccacc gcccccccag cccacggtgt cacctcggcc 1800
   ccggacacca ggccggcccc gggctccacc gcccccccag cccacggtgt cacctcggcc 1860
   ccggacacca ggccggcccc gggctccacc gcccccccag cccacggtgt cacctcggcc 1920
   ccggacacca ggccggcccc gggctccacc gcccccccag cccacggtgt cacctcggcc 1980
   ccggacacca ggccggcccc gggctccacc gcccccccag cccacggtgt cacctcggcc 2040
   ccggacacca ggccggcccc gggctccacc gcccccccag cccacggtgt cacctcggcc 2100
   ccggacacca ggccggcccc gggctccacc gcccccccag cccacggtgt cacctcggcc 2160
   ccggacacca ggccggcccc gggctccacc gcccccccag cccacggtgt cacctcggcc 2220
   ccggacacca ggccggcccc gggctccacc gcccccccag cccacggtgt cacctcggcc 2280
   ccggacacca ggccggcccc gggctccacc gcccccccag cccacggtgt cacctcggcc 2340
   ccggacacca ggccggcccc gggctccacc gcccccccag cccacggtgt cacctcggcc 2400
   ccggacacca ggccggcccc gggctccacc gcccccccag cccacggtgt cacctcggcc 2460
   ccggacacca ggccggcccc gggctccacc gcccccccag cccacggtgt cacctcggcc 2520
   ccggacacca ggccggcccc gggctccacc gcccccccag cccacggtgt cacctcggcc 2580
   ccggacacca ggccggcccc gggctccacc gcccccccag cccacggtgt cacctcggcc 2640
   ccggacacca ggccggcccc gggctccacc gcccccccag cccacggtgt cacctcggcc 2700
   ccggacacca ggccggcccc gggctccacc gcccccccag cccatggtgt cacctcggcc 2760
   ccggacaaca ggcccgcctt gggctccacc gcccctccag tccacaatgt cacctcggcc 2820
   tcaggctctg catcaggctc agcttctact ctggtgcaca acggcacctc tgccagggct 2880
   accacaaccc cagccagcaa gagcactcca ttctcaattc ccagccacca ctctgatact 2940
   cctaccaccc ttgccagcca tagcaccaag actgatgcca gtagcactca ccatagctcg 3000
   gtacctcctc tcacctcctc caatcacagc acttctcccc agttgtctac tggggtctct 3060
   ttctttttcc tgtcttttca catttcaaac ctccagttta attcctctct ggaagatccc 3120
   agcaccgact actaccaaga gctgcagaga gacatttctg aaatgttttt gcagatttat 3180
   aaacaagggg gttttctggg cctctccaat attaagttca ggccaggatc tgtggtggta 3240
   caattgactc tggccttccg agaaggtacc atcaatgtcc acgacgtgga gacacagttc 3300
   aatcagtata aaacggaagc agcctctcga tataacctga cgatctcaga cgtcagcgtg 3360
   agtgatgtgc catttccttt ctctgcccag tctggggctg ggtgccaggc ctggggcatc 3420
   gcgctgctgg tgctggtctg tgttctggtt gcgctggcca ttgtctatct cattgccttg 3480
   gctgtctgtc agtgccgccg aaagaactac gggcagctgg acatctttcc agcccgggat 3540
   acctaccatc ctatgagcga gtaccccacc taccacaccc atgggcgcta tgtgccccct 3600
   agcagtaccg atcgtagccc ctatgagaag gtttctgcag gtaacggtgg cagcagcctc 3660
   tcttacacaa acccagcagt ggcagccgct tctgccaact tgtagggcac gtcgccgctg 3720
   agctgagtgg ccagccagtg ccattccact ccactcaggt tcttcaggcc agagcccctg 3780
   caccctgttt gggctggtga gctgggagtt caggtgggct gctcacagcc tccttcagag 3840
   gccccaccaa tttctcggac acttctcagt gtgtggaagc tcatgtgggc ccctgaggct 3900
   catgcctggg aagtgttgtg gggctcccca ggaggactgg cccagagagc cctgagatag 3960
   cggggatcct gaactggact gaataaaacg tggtctccca ctg 4003
<210> 49
   <211> 28
   <212> DNA
   <213> Artificial Sequence
<220>
   <223> PCR Primer
<400> 49
   gggaattcat gacaccgggc acccagtc 28
<210> 50
   <211> 27
   <212> DNA
   <213> Artificial Sequence
<220>
   <223> PCR Primer
<400> 50
   ggtctcgaga acaactgtaa gcactgt 27
<210> 51
   <211> 28
   <212> DNA
   <213> Artificial Sequence
<220>
   <223> PCR Primer
<400> 51
   ggtcggccgt aacaactgta agcactgt 28
<210> 52
   <211> 28
   <212> DNA
   <213> Artificial Sequence
<220>
   <223> PCR Primer
<400> 52
   gcacggccgc taccacaacc ccagccag 28
<210> 53
   <211> 28
   <212> DNA
   <213> Artificial Sequence
<220>
   <223> PCR Primer
<400> 53
   gcacggccgg ttttctgggc ctctccaa 28
<210> 54
   <211> 29
   <212> DNA
   <213> Artificial Sequence
<220>
   <223> PCR Primer
<400> 54
   gcacggccgg taccatcaat gtccacgac 29
<210> 55
   <211> 28
   <212> DNA
   <213> Artificial Sequence
<220>
   <223> PCR Primer
<400> 55
   gggggatcct acaagttggc agaagcgg 28
<210> 56
   <211> 39
   <212> DNA
   <213> Artificial Sequence
<220>
   <223> PCR Primer
<400> 56
   tgctcctcac agtgcttaca ggttctggtc atgcaagct 39
<210> 57
   <211> 32
   <212> DNA
   <213> Artificial Sequence
<220>
   <223> PCR Primer
<400> 57
   gagcttgcat gaccagaacc tgtaacaact gt 32
<210> 58
   <211> 23
   <212> PRT
   <213> Homo sapiens
<400> 58
   Met Thr Pro Gly Thr Gln Ser Pro Phe Phe Leu Leu Leu Leu Leu Thr
   1               5                   10                  15
   Val Leu Thr Val Val Thr Ala
               20
<210> 59
   <211> 24
   <212> PRT
   <213> Homo sapiens
<400> 59
   Met Thr Pro Gly Thr Gln Ser Pro Phe Phe Leu Leu Leu Leu Leu Thr
   1               5                   10                  15
   Val Leu Thr Val Val Thr Ala Gly
               20
<210> 60
   <211> 50
   <212> PRT
   <213> Artificial Sequence
<220>
   <223> Synthetic Peptide
<400> 60
   Thr Ile Asn Val His Asp Val Glu Thr Gln Phe Asn Gln Tyr Lys Thr
   1               5                   10                  15
   Glu Ala Ala Ser Pro Tyr Asn Leu Thr Ile Ser Asp Val Ser Val Ser
               20                  25                  30
   Asp Val Pro Phe Pro Phe Ser Ala Gln Ser Gly Ala His His His His
           35                  40                  45
   His His
       50
<210> 61
   <211> 63
   <212> PRT
   <213> Artificial Sequence
<220>
   <223> Synthetic Peptide
<400> 61
   Ser Val Val Val Gln Leu Thr Leu Ala Phe Arg Glu Gly Thr Ile Asn
   1               5                   10                  15
   Val His Asp Val Glu Thr Gln Phe Asn Gln Tyr Lys Thr Glu Ala Ala
               20                  25                  30
   Ser Pro Tyr Asn Leu Thr Ile Ser Asp Val Ser Val Ser Asp Val Pro
           35                  40                  45
   Phe Pro Phe Ser Ala Gln Ser Gly Ala His His His His His His
       50                  55                  60
<210> 62
   <211> 19
   <212> PRT
   <213> Artificial Sequence
<220>
   <223> Synthetic Peptide
<400> 62
   His His His His His His Ser Val Val Val Gln Leu Thr Leu Ala Phe
   1               5                   10                  15
   Arg Glu Gly
<210> 63
   <211> 44
   <212> PRT
   <213> Homo sapiens
<400> 63
   Thr Ile Asn Val His Asp Val Glu Thr Gln Phe Asn Gln Tyr Lys Thr
   1               5                   10                  15
   Glu Ala Ala Ser Arg Tyr Asn Leu Thr Ile Ser Asp Val Ser Val Ser
               20                  25                  30
   Asp Val Pro Phe Pro Phe Ser Ala Gln Ser Gly Ala
           35                  40
<210> 64
   <211> 44
   <212> PRT
   <213> Artificial Sequence
<220>
   <223> Synthetic Peptide
<400> 64
   Thr Ile Asn Val His Asp Val Glu Thr Gln Phe Asn Gln Tyr Lys Thr
   1               5                   10                  15
   Glu Ala Ala Ser Pro Tyr Asn Leu Thr Ile Ser Asp Val Ser Val Ser
               20                  25                  30
   Asp Val Pro Phe Pro Phe Ser Ala Gln Ser Gly Ala
           35                  40
<210> 65
   <211> 13
   <212> PRT
   <213> Homo sapiens
<400> 65
   Ser Val Val Val Gln Leu Thr Leu Ala Phe Arg Glu Gly
   1               5                   10
<210> 66
   <211> 57
   <212> PRT
   <213> Homo sapiens
<400> 66
   Ser Val Val Val Gln Leu Thr Leu Ala Phe Arg Glu Gly Thr Ile Asn
   1               5                   10                  15
   Val His Asp Val Glu Thr Gln Phe Asn Gln Tyr Lys Thr Glu Ala Ala
               20                  25                  30
   Ser Pro Tyr Asn Leu Thr Ile Ser Asp Val Ser Val Ser Asp Val Pro
           35                  40                  45
   Phe Pro Phe Ser Ala Gln Ser Gly Ala
       50                  55